# EUROPEAN PATENT SPECIFICATION

(11) **EP 4 469 463 B1**
(45) Date of publication and mention of the grant of the patent: **29.10.2025**
(21) Application number: 23701975.7
(22) Date of filing: 27.01.2023
(51) Int. Cl.: C07D 498/22, A61P 31/04, A61K 31/4188

(54) **RIFAMYCIN DERIVATIVES FOR THE TREATMENT OF DISEASES**
RIFAMYCINDERIVATE ZUR BEHANDLUNG VON KRANKHEITEN
DÉRIVÉS DE LA RIFAMYCINE POUR LE TRAITEMENT DE MALADIES

(30) Priority: 28.01.2022 EP 22154022
(43) Date of publication of application: 04.12.2024
(73) Proprietor: BioVersys AG, 4057 Basel (CH)
(72) Inventor: ANTRAYGUES, Kevin, 59120 Loos (FR); BOUROTTE, Marilyne, 59840 Perenchies (FR); DALE, Glenn E., 4053 Basel (CH); DEFERT, Olivier, 59000 Lille (FR); GIOIA, Bruna, 59800 Lille (FR); GITZINGER, Marc, 4057 Basel (CH); LOCIURO, Sergio, 4058 Basel (CH); MAINGOT, Mathieu, 59800 Lille (FR); TREBOSC, Vincent, 68680 Kembs-Loechlé (FR); WILLAND, Nicolas, 59800 Lille (FR)
(74) Representative: Latscha Schöllhorn Partner AG
(86) International application number: PCT/EP2023/051991
(87) International publication number: WO 2023/144297

(56) References cited:
- GARCÍA ANA-BELÉN ET AL: "Strong In Vitro Activities of Two New Rifabutin Analogs against Multidrug-Resistant Mycobacterium tuberculosis", vol. 54, no. 12, 1 December 2010 (2010-12-01), US, pages 5363 - 5365, XP055929413, ISSN: 0066-4804, Retrieved from the Internet <URL:https://journals.asm.org/doi/pdf/10.1128/AAC.00149-10> DOI: 10.1128/AAC.00149-10
- FIGUEIREDO R ET AL: "Synthesis and evaluation of rifabutin analogs against Mycobacteriumavium and H"3"7Rv, MDR and NRP Mycobacterium tuberculosis", BIOORGANIC, ELSEVIER, AMSTERDAM, NL, vol. 17, no. 2, 15 January 2009 (2009-01-15), pages 503 - 511, XP025893420, ISSN: 0968-0896, [retrieved on 20090115], DOI: 10.1016/J.BMC.2008.12.006
- BARLUENGA J ET AL: "New rifabutin analogs: Synthesis and biological activity against Mycobacterium tuberculosis", BIOORGANIC & MEDICINAL CHEMISTRY LETTERS, ELSEVIER, AMSTERDAM, NL, vol. 16, no. 22, 15 November 2006 (2006-11-15), pages 5717 - 5722, XP027966071, ISSN: 0960-894X, [retrieved on 20061115]
- ZLOH MIRE ET AL: "Novel C-3-(N-alkyl-aryl)-aminomethyl rifamycin SV derivatives exhibit activity against rifampicin-resistant Mycobacterium tuberculosis RpoBS522L strain and display a different binding mode at the RNAP [beta]-subunit site compared to rifampicin", EUROPEAN JOURNAL OF MEDICINAL CHEMISTRY, vol. 225, 1 December 2021 (2021-12-01), AMSTERDAM, NL, pages 113734, XP055929410, ISSN: 0223-5234, DOI: 10.1016/j.ejmech.2021.113734

## Description

The present disclosure relates to compounds and pharmaceutical compositions comprising the same for the treatment, amelioration and/or prevention of disease. In some embodiments, the disease is a bacterial infection. In some embodiments, the bacteria belong to the genus *Acinetobacter* spp., *Clostridium* spp., *Enterococcus* spp., *Hemophilus* spp., *Legionella* spp., *Mycobacterium* spp., *Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and/or *Toxoplasma gondii.* In some embodiments the infection is caused by a bacterium of the genus *Acinetobacter.*

### RELATED ART

Rifamycins such as rifabutin are known antibiotics with activity against a broad spectrum of pathogens such as *Clostridium* spp., *Enterococcus* spp., *Hemophilus* spp., *Legionella* spp., *Mycobacterium* spp. (tuberculous and non-tuberculous *Mycobacteria*)*, Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and *Toxoplasma gondii* (Kunin, Clin. Infect. Dis., 1996; Farr and Mandell, Med. Clin. North. Am., 1982; Thornsberry et al., Rev. Infect. Dis., 1983; Hoover et al., Diagn. Microbiol. Infect. Dis., 1993; Kerry et al., J. Antimicrob. Chemother., 1975)*.*

Rifabutin has been recently shown to have potent *in vitro* and *in vivo* activity against *Mycobacterium abscessus* (Aziz et al., Antimicrob. Agents Chemother., 2017; Dick et al., Antimicrob. Agents Chemother., 2020) and *Acinetobacter baumannii* (Luna et al., Nat. Microbiol., 2020; Trebosc et al., Drug Discov. Today, 2021; Trebosc et al., J. Antimicrob. Chemother., 2020). However, pathogens such as *A*. *baumannii* can show reduced rifabutin sensitivity through mechanisms such as mutation of the RNA polymerase β-subunit (RpoB) gene (Trebosc et al., J. Antimicrob. Chemother., 2020). Accordingly, there is a need for more effective rifamycins for the treatment of bacterial infections such as those caused by a bacteria of the genus *Acinetobacter.*

### SUMMARY OF THE INVENTION

In a first aspect, the invention provides a compound of Formula I or a pharmaceutically acceptable salt, tautomer, solvate, hydrate, enantiomer or diastereomer thereof: 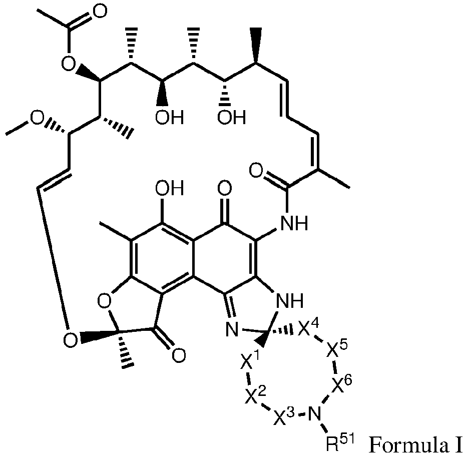 wherein:
X¹ is -CR¹R^{1A}-;
X² is independently, at each occurrence, selected from -CR²R2^{A}-, -NR^{2B}-, -O-, and a chemical bond;
X³ is independently, at each occurrence, selected from -CR³R^{3A}-, -NR^{3B}-, -O-, and a chemical bond;
X⁴ is -CR⁴R^{4A}-;
X⁵ is independently, at each occurrence, selected from -CR⁵R^{5A}-, -NR^{5B}-, -O-, and a chemical bond;
X⁶ is independently, at each occurrence, selected from -CR⁶R^{6A}-, -NR^{6B}-, -O-, and a chemical bond;
R⁵¹ is independently, at each occurrence, selected from -H, -C₁-C₆ alkyl, and -C₃-C₈ cycloalkyl;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R^{2B}, R^{3B}, R^{5B} and R^{6B} are independently, at each occurrence, selected from -H and -C₁-C₆ alkyl; or
any of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹, together with the atom to which they are attached, can independently combine with any other member of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹ to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₆ alkyl or -C₁-C₆ alkoxy;
wherein when any of R¹, R^{1A}, R², R^{2A}, R^{2B}, R³, R^{3A}, R^{3B}, R⁴, R^{4A} R⁵, R^{5A}, R^{5B}, R⁶, R^{6A} and R^{6B} combine to form said first bridging cycloalkyl ring or said first bridging heterocycloalkyl ring, said first bridging cycloalkyl or first bridging heterocycloalkyl can independently combine with R⁵¹ to form a second bridging 5-7 membered heterocycloalkyl, wherein said second bridging heterocycloalkyl is optionally substituted by one or more -C₁-C₆ alkyl, or -C₁-C₆ alkoxy; and
provided that at least two of R¹, R^{1A}, R², R^{2A}, R^{2B}, R³, R^{3A}, R^{3B}, R⁴, R^{4A}, R⁵, R^{5A} R^{5B} R⁶, R^{6A}, R^{6B} and R⁵¹ combine to form a bridging cycloalkyl or heterocycloalkyl; or no more than one of X², X³, X⁵, and X⁶ is a chemical bond.

In one aspect, the invention provides a pharmaceutical composition comprising at least one compound as set forth herein, or a pharmaceutically acceptable salt, tautomer, solvate or hydrate thereof, and a pharmaceutically acceptable excipient.

In one aspect, the invention provides a compound or a pharmaceutical composition as described herein, for use in a method of preventing or treating a disease in a subject, preferably an infection, further preferably a bacterial infection, further preferably a bacterial infection caused by a bacteria belonging to *Acinetobacter* spp., *Clostridium* spp., *Enterococcus* spp., *Hemophilus* spp., *Legionella* spp., *Mycobacterium* spp. (tuberculous and non-tuberculous *Mycobacteria*), *Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and/or *Toxoplasma gondii*; more preferably a bacterial infection caused by *S*. *aureus, A. baumannii, E. faecium, E. faecalis, S. epidermidis, S. pneumoniae, S. pyogenes, H. influenzae, M. abscessus, M. tuberculosis,* and/or *M. smegmatis*; yet more preferably a bacterial infection caused by one or more bacterium belonging to the genus *Acinetobacter*; yet more preferably *A*. *baumannii;* and even more preferably *A*. *baumannii* that carries one or more resistance mechanisms against rifamycins such as rifabutin and/or rifampicin.

The inventive compounds are novel rifamycin derivatives containing novel modified spiro-imidazole moieties. In addition to showing broad antibacterial activity characteristic of the rifamycin class of antibiotics (e.g., against *Clostridium* spp., *Enterococcus* spp., *Hemophilus* spp., *Legionella* spp., *Mycobacterium* spp. (tuberculous and non-tuberculous *Mycobacteria*)*, Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and *Toxoplasma gondii*)*,* the inventive compounds additionally showed enhanced antibacterial activity against strains of the genus *Acinetobacter.* In particular, the inventive compounds exhibited activity against strains of *A*. *baumannii* that carry one or more resistance mechanisms against rifamycins such as rifampicin and/or rifabutin. Additional features and advantages of the inventive compounds are set forth in the Detailed Description, below.

### DESCRIPTION OF FIGURES

FIG. 1: ROESY and HSQC spectra superposition of Compound 13B.
FIG. 2: Attribution of the stereochemistry of the tropane moiety of Compound 13B.

### DETAILED DESCRIPTION OF THE INVENTION

The present disclosure provides novel rifamycin derivatives containing novel modified spiro-imidazole moieties that are effective at treating bacterial infections, preferably bacterial infections caused by a bacteria belonging to *Acinetobacter* spp., *Clostridium* spp., *Enterococcus* spp., *Hemophilus* spp., *Legionella* spp., *Mycobacterium* spp. (tuberculous and non-tuberculous *Mycobacteria*), *Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and/or *Toxoplasma gondii;* more preferably bacterial infections caused by *S*. *aureus, A. baumannii, E. faecium, E. faecalis, S. epidermidis, S. pneumoniae, S. pyogenes, H. influenzae, M. abscessus, M. tuberculosis,* and/or *M. smegmatis*; yet more preferably bacterial infections caused by one or more bacterium belonging to the genus *Acinetobacter*; yet more preferably *A*. *baumannii*; and even more preferably *A*. *baumannii* that carries one or more resistance mechanisms against rifamycins such as rifabutin and/or rifampicin.

The details of the technology are set forth in the accompanying description below. Although methods and materials similar or equivalent to those described herein can be used in the practice or testing of the present technology, illustrative methods and materials are now described. Other features, objects, and advantages of the disclosure will be apparent from the description and from the claims.

### Definitions

Unless defined otherwise, all technical and scientific terms used herein have the same meanings as commonly understood by one of ordinary skill in the art to which this invention belongs.

The articles "a" and "an" are used in this disclosure to refer to one or more than one (i.e., to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety (e.g. an alkyl group) can (but is not required to) be bonded other substituents (e.g. heteroatoms or alkoxy groups). For instance, an alkyl group that is optionally substituted can be a fully saturated alkyl chain (i.e. a pure hydrocarbon). Alternatively, the same optionally substituted alkyl group can have substituents different from hydrogen. For instance, it can, at any point along the chain be bounded to, e.g., an alkoxy group or any other substituent described herein. Thus, the term "optionally substituted" means that a given chemical moiety has the potential to contain other functional groups, but does not necessarily have any further functional groups.

The term "alkyl" refers to a straight or branched chain saturated hydrocarbon. C₁-C₆ alkyl groups contain 1 to 6 carbon atoms. Examples of a C₁-C₆ alkyl group include, but are not limited to, methyl, ethyl, propyl, butyl, pentyl, isopropyl, isobutyl, sec-butyl and tert-butyl, isopentyl and neopentyl.

The terms "alkylene" or "alkylenyl," as used herein, refer to a straight or branched hydrocarbon chain bi-radical derived from alkyl, as defined herein, wherein one hydrogen of said alkyl is cleaved off generating the second radical of said alkylene. Examples of alkylene are, by way of illustration, -CH₂-, -CH₂-CH₂-, -CH(CH₃)-, -CH₂-CH₂-CH₂-, -CH(CH₃)-CH₂-, or -CH(CH₂CH₃)-.

The term "cycloalkyl" means monocyclic saturated carbon rings containing 3-8 carbon atoms. A C₃-C₆ cycloalkyl contains between 3 and 6 carbon atoms. Examples of cycloalkyl groups include, without limitations, cyclopropyl, cyclobutyl, cyclopentyl, cyclohexyl, cycloheptanyl, and cyclooctanyl.

The terms "heterocyclyl" or "heterocycloalkyl" or "heterocycle" refer to 3 to 8-membered rings containing carbon and heteroatoms taken from oxygen, nitrogen, sulfur, and/or phosphorous (preferably oxygen and nitrogen) and wherein the ring does not comprise delocalized π electrons (aromaticity) shared among the ring carbon or heteroatoms. In preferred embodiments, the heterocycloalkyl ring is fully saturated. Heterocyclyl rings include, but are not limited to, oxetanyl, azetadinyl, tetrahydrofuranyl, pyrrolidinyl, oxazolinyl, oxazolidinyl, thiazolinyl, thiazolidinyl, pyranyl, thiopyranyl, tetrahydropyranyl, dioxanyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl. In some embodiments, the heterocyclyl or heterocycloalkyl ring is bridged, e.g., forms a bicyclic or tricyclic ring with the atoms or substituents to which it is attached.

"C₁-C₆-alkoxy", as used herein, refers to a substituted hydroxyl of the formula (-OR'), wherein R' is an optionally substituted C₁-C₆ alkyl, as defined herein, and the oxygen moiety is directly attached to the parent molecule, and thus the term "C₁-C₆ alkoxy", as used herein, refers to straight chain or branched C₁-C₆ alkoxy which may be, for example, methoxy, ethoxy, n-propoxy, isopropoxy, n-butoxy, isobutoxy, *sec*-butoxy, *tert*-butoxy, straight or branched pentoxy, straight or branched hexyloxy. Preferred are C₁-C₄ alkoxy and C₁-C₃ alkoxy.

The term "pharmaceutically acceptable salt" as used herein refers to a salt that possesses the desired pharmacological activity of the parent compound. Such salts include acid addition salts formed with inorganic acids or organic acids known to the skilled person in the art (P. Heinrich Stahl (Editor), Camille G. Wermuth (Editor); Handbook of Pharmaceutical Salts: Properties, Selection, and Use, 2nd Revised Edition, March 2011, Wiley-VCH, ISBN: 978-3-90639-051-2). Representative "pharmaceutically acceptable salts" of the inventive compounds include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, hydroiodide, sethionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "stereoisomers" refers to the set of compounds which have the same number and type of atoms and share the same bond connectivity between those atoms, but differ in three-dimensional structure. The term "stereoisomer" refers to any member of this set of compounds.

The term "diastereomers" refers to the set of stereoisomers which cannot be made superimposable by rotation around single bonds. For example, *cis-* and *trans*-double bonds, *endo-* and *exo*-substitution on bicyclic ring systems, and compounds containing multiple stereogenic centers with different relative configurations are considered to be diastereomers. The term "diastereomer" refers to any member of this set of compounds. In some examples presented, the synthetic route may produce a single diastereomer or a mixture of diastereomers. In such cases all possible diastereomers are contemplated by the present invention.

The term "enantiomers" refers to a pair of stereoisomers which are non-superimposable mirror images of one another. The term "enantiomer" refers to a single member of this pair of stereoisomers. The term "racemic" refers to a 1:1 mixture of a pair of enantiomers.

The term "tautomers" refers to a set of compounds that have the same number and type of atoms, but differ in bond connectivity and are in equilibrium with one another. A "tautomer" is a single member of this set of compounds. Typically a single tautomer is drawn but it is understood that this single structure is meant to represent all possible tautomers that might exist. Examples include enol-ketone tautomerism. When a ketone is drawn it is understood that both the enol and ketone forms are part of the present disclo sure.

The term "solvate" refers to a complex of variable stoichiometry formed by a solute and solvent. Such solvents for the purpose of the disclosure do not interfere with the biological activity of the solute. Examples of suitable solvents include, but are not limited to, water, MeOH, EtOH, and AcOH. Solvates wherein water is the solvent molecule are typically referred to as "hydrates." Hydrates include compositions containing stoichiometric amounts of water, as well as compositions containing variable amounts of water.

An "effective amount" when used in connection with a compound is an amount effective for treating or preventing a disease in a subject as described herein.

The term "carrier", as used in this disclosure, encompasses excipients and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed compound or pharmaceutically acceptable salt of the disclosed compound or a composition comprising the same to a subject.

A "patient" or "subject" is a mammal, e.g., a human, mouse, rat, guinea pig, dog, cat, horse, cow, pig, or non-human primate, such as a monkey, chimpanzee, baboon or rhesus, preferably a human.

### Compounds of the Invention

In a first aspect, the invention provides a compound of Formula I or a pharmaceutically acceptable salt, tautomer, solvate, hydrate, enantiomer or diastereomer thereof: 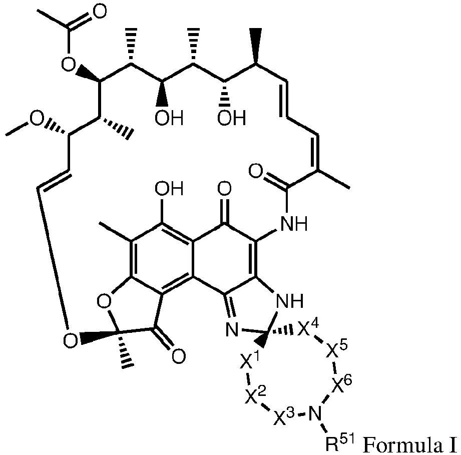 wherein:
X¹ is -CR¹R^{1A}-;
X² is independently, at each occurrence, selected from -CR²R^{2A}-, -NR^{2B}-, -O-, and a chemical bond;
X³ is independently, at each occurrence, selected from -CR³R^{3A}-, -NR^{3B}-, -O-, and a chemical bond;
X⁴ is -CR⁴R^{4A-};
X⁵ is independently, at each occurrence, selected from -CR⁵R^{5A}-, -NR^{5B}-, -O-, and a chemical bond;
X⁶ is independently, at each occurrence, selected from -CR⁶R^{6A}-, -NR^{6B}-, -O-, and a chemical bond;
R⁵¹ is independently, at each occurrence, selected from -H, -C₁-C₆ alkyl, and -C₃-C₈ cycloalkyl;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R^{2B}, R^{3B}, R^{5B} and R^{6B} are independently, at each occurrence, selected from -H and -C₁-C₆ alkyl; or
any of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹, together with the atom to which they are attached, can independently combine with any other member of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹ to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₆ alkyl or -C₁-C₆ alkoxy;
wherein when any of R¹, R^{1A}, R², R^{2A}, R^{2B}, R³, R^{3A}, R^{3B}, R⁴, R^{4A}, R⁵, R^{5A}, R^{5B}, R⁶, R^{6A} and R^{6B} combine to form said first bridging cycloalkyl ring or said first bridging heterocycloalkyl ring, said first bridging cycloalkyl or first bridging heterocycloalkyl can independently combine with R⁵¹ to form a second bridging 5-7 membered heterocycloalkyl, wherein said second bridging heterocycloalkyl is optionally substituted by one or more -C₁-C₆ alkyl, or -C₁-C₆ alkoxy; and
provided that at least two of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹ combine to form a bridging cycloalkyl or heterocycloalkyl; or no more than one of X², X³, X⁵, and X⁶ is a chemical bond.

In some embodiments, X¹, X², X³, X⁴, X⁵, X⁶ and NR⁵¹ combine to form an azepane, wherein said azepane optionally further comprises a bridging C₄-C₈ cycloalkyl or bridging 5 to 8-membered heterocycloalkyl; or
X¹, X², X³, X⁴, X⁵, X⁶ and NR⁵¹ combine to form a piperidine further comprising a bridging C₄-C₈ cycloalkyl or bridging 5 to 8-membered heterocycloalkyl.

In some embodiments, X¹ is -CR¹R^{1A}-;
X² is independently, at each occurrence, selected from -CR²R^{2A}-, -NR^{2B}-, and -O-;
X³ is independently, at each occurrence, selected from -CR³R^{3A}-, -NR^{3B}-, -O-, and a chemical bond;
X⁴ is -CR⁴R^{4A}-;
X⁵ is independently, at each occurrence, selected from -CR⁵R^{5A}-, -NR^{5B}-, and -O-;
X⁶ is independently, at each occurrence, selected from -CR⁶R^{6A}-, -NR^{6B}-, -O-, and a chemical bond;
R⁵¹ is independently, at each occurrence, selected from -H, -C₁-C₆ alkyl, and -C₃-C₈ cycloalkyl;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R^{2B}, R^{3B}, R^{5B} and R^{6B} are independently, at each occurrence, selected from -H and -C₁-C₆ alkyl; or
any of R¹, R², R³, R⁴, R⁵, R⁶, and R⁵¹, together with the atom to which they are attached, can independently combine with any other member of R¹, R², R³, R⁴, R⁵, R⁶, R^{6A}, and R⁵¹ to form a first bridging C₄-C₈ cycloalkyl ring or a bridging 5-8 membered heterocycloalkyl ring, wherein each bridging cycloalkyl or bridging heterocycloalkyl is optionally substituted by one or more -C₁-C₆ alkyl or -C₁-C₆ alkoxy;
wherein when any of R¹, R², R³, R⁴, R⁵, and R⁶ combine to form a first bridging cycloalkyl ring or a first bridging heterocycloalkyl ring, the first bridging cycloalkyl or first bridging heterocycloalkyl can independently combine with R⁵¹ to form a second bridging 5-7 membered heterocycloalkyl, wherein said second bridging heterocycloalkyl is optionally substituted by one or more -C₁-C₆ alkyl or -C₁-C₆ alkoxy; and
provided that at least two of R¹, R², R³, R⁴, R⁵, R⁶, and R⁵¹ combine to form a bridging cycloalkyl or heterocycloalkyl; or no more than one of X¹, X², X³, X⁴, X⁵, and X⁶ is a chemical bond.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy; R⁵¹ is independently, at each occurrence, selected from -H, -C₁-C₆ alkyl, and -C₃-C₈ cycloalkyl; or
any of R¹, R² and R³, together with the carbon atoms to which they are attached, can independently combine with any of R⁴, R⁵, R⁶, and R⁵¹ to form a bridging C₄-C₈ cycloalkyl ring or a bridging 5-8 membered heterocycloalkyl ring; or any of R⁴, R⁵, R⁶, together with the carbon atoms to which they are attached, can independently combine with R⁵¹ to form a bridging 5-8 membered heterocycloalkyl ring, wherein said heterocycloalkyl is optionally substituted by one or more -C₁-C₆ alkyl or -C₁-C₆ alkoxy;
wherein when any of R¹, R², R³, R⁴, R⁵, and R⁶, and combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted by one or more -C₁-C₆ alkyl, or -C₁-C₆ alkoxy; and
provided that at least two of R¹, R², R³, R⁴, R⁵, R⁶ and R⁵¹ combine to form a bridging cycloalkyl or heterocycloalkyl; or no more than one of X³ and X⁶ is a chemical bond.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R⁵¹ is independently, at each occurrence, selected from -H, -C₁-C₆ alkyl, and -C₃-C₈ cycloalkyl; or
any of R¹, R² and R³, together with the carbon atoms to which they are attached, can independently combine with any of R⁴, R⁵, R⁶, and R⁵¹ to form a bridging C₄-C₈ cycloalkyl ring or a bridging 5-8 membered heterocycloalkyl ring; or any of R⁴, R⁵, R⁶, together with the carbon atoms to which they are attached, can independently combine with R⁵¹ to form a bridging C₄-C₈ cycloalkyl ring or a bridging 5-8 membered heterocycloalkyl ring, wherein each bridging cycloalkyl or bridging heterocycloalkyl is optionally substituted by one or more -C₁-C₆ alkyl or -C₁-C₆ alkoxy;
wherein when any of R¹, R², and R³ combine with any of R⁴, R⁵, and R⁶ to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted by one or more -C₁-C₆ alkyl, or -C₁-C₆ alkoxy; and
provided that at least two of R¹, R², R³, R⁴, R⁵, R⁶, and R⁵¹ combine to form a bridging cycloalkyl or heterocycloalkyl; or no more than one of X³ and X⁶ is a chemical bond.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₄ alkyl, and -C₁-C₄ alkoxy, preferably -H, C₁-C₃ alkyl and C₁-C₃ alkoxy, more preferably -H, C₁-C₂ alkyl and -C₂ alkoxy, yet more preferably -H;
R⁵¹ is independently, at each occurrence, selected from -H, -C₁-C₄ alkyl, and -C₃-C₆ cycloalkyl; or
any of R¹, R² and R³, together with the carbon atoms to which they are attached, can independently combine with any of R⁴, R⁵, R⁶, and R⁵¹ to form a bridging C₄-C₈ cycloalkyl ring or a bridging 5-8 membered heterocycloalkyl ring; or any of R⁴, R⁵, R⁶, together with the carbon atoms to which they are attached, can independently combine with R⁵¹ to form a bridging C₄-C₈ cycloalkyl ring or a bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably one or more -C₁-C₃ alkyl or -C₁-C₃alkoxy, more preferably one or more -C₁-C₃ alkyl or -C₁-C₃alkoxy;
wherein when any of R¹, R², and R³ combine any of R⁴, R⁵, and R⁶ to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted by one or more -C₁-C₄ alkyl or -C₁-C₄ alkoxy, preferably -C₁-C₃ alkyl, or -C₁-C₃ alkoxy, more preferably -C₁-C₂ alkyl, or C₁-C₂ alkoxy;
provided that (i) R¹ and R⁴ combine to form a first bridging cycloalkyl or heterocycloalkyl and X³ and X⁶ are chemical bonds; (ii) R² and R⁵ combine to form a first bridging cycloalkyl or heterocycloalkyl and X³ and X⁶ are chemical bonds; (iii) one of R¹ and R⁴ combine with R⁵¹ to form a first bridging cycloalkyl ring or first bridging heterocycloalkyl ring and X³ and X⁶ are chemical bonds; or (iv) exactly one of X³ and X⁶ is a chemical bond.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₄ alkyl, and -C₁-C₄ alkoxy, preferably -H, C₁-C₃ alkyl and C₁-C₃ alkoxy, more preferably -H, C₁-C₂ alkyl and -C₂ alkoxy, yet more preferably -H;
R⁵¹ is independently, at each occurrence, selected from -H, -C₁-C₄ alkyl, and -C₃-C₆ cycloalkyl; or
any of R¹, R² and R³, together with the carbon atoms to which they are attached, can independently combine with any of R⁴, R⁵, R⁶, and R⁵¹ to form a bridging C₄-C₈ cycloalkyl ring or a bridging 5-8 membered heterocycloalkyl ring; or any of R⁴, R⁵, R⁶, together with the carbon atoms to which they are attached, can independently combine with R⁵¹ to form a bridging C₄-C₈ cycloalkyl ring or a bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably one or more -C₁-C₃ alkyl or -C₁-C₃alkoxy, more preferably one or more -C₁-C₃ alkyl or -C₁-C₃alkoxy;
wherein when any of R¹, R², and R³ combine any of R⁴, R⁵, and R⁶ to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted by one or more -C₁-C₄ alkyl or -C₁-C₄ alkoxy, preferably -C₁-C₃ alkyl, or -C₁-C₃ alkoxy, more preferably -C₁-C₂ alkyl, or C₁-C₂ alkoxy;
provided that (i) R¹ and R⁴ combine to form a bridging cycloalkyl or heterocycloalkyl and X³ and X⁶ are chemical bonds; (ii) R² and R⁵ combine to form a bridging cycloalkyl or heterocycloalkyl and X³ and X⁶ are chemical bonds; or (iii) exactly one of X³ and X⁶ is a chemical bond.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond; wherein
(i) R^{1A}, R², R^{2A}, R^{4A}, R⁵ and R^{5A} are -H; R³, R^{3A}, R⁶ and R^{6A} are absent or -H; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(ii) R^{1A}, R¹, R^{2A}, R^{4A}, R⁴ and R^{5A} are -H; R³, R^{3A}, R⁶ and R^{6A} are absent or -H; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iii) R^{1A}, R², R^{2A}, R⁴, R^{4A}, R⁵ and R^{5A} are -H; R³, R^{3A}, R⁶ and R^{6A} are absent or -H; R¹ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iv) R¹, R^{1A}, R², R^{2A}, R^{4A}, R⁵, and R^{5A} are -H; R³, R^{3A}, R⁶ and R^{6A} are absent or -H; R⁴ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted with one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(v) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond; wherein
(i) R^{1A}, R², R^{2A}, R^{4A}, R⁵ and R^{5A} are -H; R³, R^{3A}, R⁶ and R^{6A} are absent or -H; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(ii) R^{1A}, R¹, R^{2A}, R^{4A}, R⁴ and R^{5A} are -H; R³, R^{3A}, R⁶ and R^{6A} are absent or -H; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iii) R^{1A}, R², R^{2A}, R⁴, R^{4A}, R⁵ and R^{5A} are -H; R³, R^{3A}, R⁶ and R^{6A} are absent or -H; R¹ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iv) R¹, R^{1A}, R², R^{2A}, R^{4A} , R⁵, and R^{5A} are -H; R³, R^{3A}, R⁶ and R^{6A} are absent or -H; R⁴ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted with one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(v) exactly one of X³ and X⁶ is a chemical bond.

In some embodiments, X¹ is CR¹R^{1A}; X² is CR²R^{2A}; X³ is independently, at each occurrence, selected from CR³R^{3A} and a chemical bond, preferably X³ is a chemical bond and R³ and R^{3A} are absent; X⁴ is CR⁴R^{4A}; X⁵ is CR⁵R^{5A} X⁶ is independently, at each occurrence, selected from CR⁶R^{6A} and a chemical bond, preferably X⁶ is a chemical bond and R⁶ and R^{6A} are absent; wherein
(i) R^{1A}, R², R^{2A}, R^{4A}, R⁵ and R^{5A} are -H; R³, R^{3A}, R⁶ and R^{6A} are absent or -H, preferably R³, R^{3A}, R⁶ and R^{6A} are absent; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(ii) R^{1A}, R¹, R^{2A}, R^{4A}, R⁴ and R^{5A} are -H; R³, R^{3A}, R⁶ and R^{6A} are absent or -H, preferably R³, R^{3A}, R⁶ and R^{6A} are absent; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iii) R^{1A}, R², R^{2A}, R⁴, R^{4A}, R⁵ and R^{5A} are -H; R³, R^{3A}, R⁶ and R^{6A} are absent or -H, preferably R³, R^{3A}, R⁶ and R^{6A} are absent; R¹ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iv) R¹, R^{1A}, R², R^{2A}, R^{4A}, R⁵, and R^{5A} are -H; R³, R^{3A}, R⁶ and R^{6A} are absent or -H, preferably R³, R^{3A}, R⁶ and R^{6A} are absent; R⁴ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted with one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(v) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X³ is independently, at each occurrence, selected from CR³R^{3A} and a chemical bond, preferably X³ is a chemical bond and R³ and R^{3A} are absent; X⁴ is CR⁴R^{4A}; X⁵ is CR⁵R^{5A}; X⁶ is independently, at each occurrence, selected from CR⁶R^{6A} and a chemical bond, preferably X⁶ is a chemical bond and R⁶ and R^{6A} are absent; wherein
(i) R^{1A}, R², R^{2A}, R^{4A}, R⁵ and R^{5A} are -H; R³, R^{3A}, R⁶ and R^{6A} are absent or -H, preferably R³, R^{3A}, R⁶ and R^{6A} are absent; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(ii) R^{1A}, R¹, R^{2A}, R^{4A}, R⁴ and R^{5A} are -H; R³, R^{3A}, R⁶ and R^{6A} are absent or -H, preferably R³, R^{3A}, R⁶ and R^{6A} are absent; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iii) R^{1A}, R², R^{2A}, R⁴, R^{4A}, R⁵ and R^{5A} are -H; R³, R^{3A}, R⁶ and R^{6A} are absent or -H, preferably R³, R^{3A}, R⁶ and R^{6A} are absent; R¹ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iv) R¹, R^{1A}, R², R^{2A}, R^{4A}, R⁵, and R^{5A} are -H; R³, R^{3A}, R⁶ and R^{6A} are absent or -H, preferably R³, R^{3A}, R⁶ and R^{6A} are absent; R⁴ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted with one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(v) exactly one of X³ and X⁶ is a chemical bond.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging 5-8 membered heterocycloalkyl ring, wherein said bridging heterocycloalkyl is optionally substituted by one or more -C₁-C₃ alkyl or -C₁-C₃ alkoxy, preferably -C₁-C₂ alkyl or -C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl wherein the second bridging heterocycloalkyl is optionally substituted by one or more -C₁-C₃ alkyl or -C₁-C₃ alkoxy, preferably -C₁-C₂ alkyl or -C₁-C₂ alkoxy; or
(iv) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging 5-8 membered heterocycloalkyl ring, wherein said bridging heterocycloalkyl is optionally substituted by one or more -C₁-C₃ alkyl or -C₁-C₃ alkoxy, preferably -C₁-C₂ alkyl or -C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl wherein the second bridging heterocycloalkyl is optionally substituted by one or more -C₁-C₃ alkyl or -C₁-C₃ alkoxy, preferably -C₁-C₂ alkyl or -C₁-C₂ alkoxy; or
(iv) exactly one of X³ and X⁶ is a chemical bond.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging C₄-C₆ cycloalkyl ring or a first bridging 5-7 membered heterocycloalkyl ring, wherein the heterocycloalkyl ring comprises one or more atoms selected form O and N, preferably one or two atoms selected from O and N, more preferably exactly one atom selected from O and N, wherein each bridging cycloalkyl or bridging heterocycloalkyl is optionally substituted by one or more -C₁-C₃ alkyl or -C₁-C₃ alkoxy, more preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H, R² and R⁵ combine to form a first bridging C₅-C₇ cycloalkyl ring or a first bridging 5-7 membered heterocycloalkyl ring, wherein the heterocycloalkyl ring comprises one or more atoms selected form O and N, preferably one or two atoms selected from O and N, more preferably exactly one atom selected from O and N, wherein each bridging cycloalkyl or bridging heterocycloalkyl is optionally substituted by one or more -C₁-C₃ alkyl or -C₁-C₃ alkoxy, more preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging 5-7 membered heterocycloalkyl ring wherein the heterocycloalkyl ring comprises one or more atoms selected form O and N, preferably one or two atoms selected from O and N, wherein the bridging heterocycloalkyl is optionally substituted by one or more -C₁-C₃ alkyl or -C₁-C₃ alkoxy, more preferably -C₁-C₂ alkyl or -C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl wherein the second bridging heterocycloalkyl comprises one or more atoms selected form O and N, preferably one or two atoms selected from O and N and is optionally substituted by one or more -C₁-C₃ alkyl or -C₁-C₃ alkoxy, more preferably -C₁-C₂ alkyl or -C₁-C₂ alkoxy; or
(iv) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging C₄-C₆ cycloalkyl ring or a first bridging 5-7 membered heterocycloalkyl ring, wherein the heterocycloalkyl ring comprises one or more atoms selected form O and N, preferably one or two atoms selected from O and N, more preferably exactly one atom selected from O and N, wherein each bridging cycloalkyl or bridging heterocycloalkyl is optionally substituted by one or more -C₁-C₃ alkyl or -C₁-C₃ alkoxy, more preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H, R² and R⁵ combine to form a first bridging C₅-C₇ cycloalkyl ring or a first bridging 5-7 membered heterocycloalkyl ring, wherein the heterocycloalkyl ring comprises one or more atoms selected form O and N, preferably one or two atoms selected from O and N, more preferably exactly one atom selected from O and N, wherein each bridging cycloalkyl or bridging heterocycloalkyl is optionally substituted by one or more -C₁-C₃ alkyl or -C₁-C₃ alkoxy, more preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging 5-7 membered heterocycloalkyl ring wherein the heterocycloalkyl ring comprises one or more atoms selected form O and N, preferably one or two atoms selected from O and N, wherein the bridging heterocycloalkyl is optionally substituted by one or more -C₁-C₃ alkyl or -C₁-C₃ alkoxy, more preferably -C₁-C₂ alkyl or -C₁-C₂ alkoxy; wherein
   when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl wherein the second bridging heterocycloalkyl comprises one or more atoms selected form O and N, preferably one or two atoms selected from O and N and is optionally substituted by one or more -C₁-C₃ alkyl or -C₁-C₃ alkoxy, more preferably -C₁-C₂ alkyl or -C₁-C₂ alkoxy; or
(iv) exactly one of X³ and X⁶ is a chemical bond.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging cyclobutyl, cyclopentyl, cyclohexyl, or piperidinyl ring, wherein each cyclobutyl, cyclopentyl, cyclohexyl, or piperidinyl ring is optionally substituted with one or more -C₁-C₃ alkyl or -C₁-C₃ alkoxy, more preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H; R² and R⁵ combine to form a first bridging cyclohexyl or cycloheptyl ring, wherein each cyclohexyl or cycloheptyl is optionally substituted with one or more -C₁-C₃ alkyl or -C₁-C₃ alkoxy, more preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging pyrrolidinyl, piperidinyl, or oxazinanyl preferably a bridging pyrrolidinyl, piperidinyl, or 1,4 oxazinanyl, wherein each pyrrolidinyl, piperidinyl, or oxazinanyl is optionally substituted with one or more -C₁-C₃ alkyl or -C₁-C₃ alkoxy, more preferably -C₁-C₂ alkyl or -C₁-C₂ alkoxy; wherein
   when R¹ and R⁴ or R² and R⁵ combine to form a first bridging cyclohexyl or piperidinyl, the first bridging cyclohexyl or piperidinyl can independently combine with R⁵¹ to form a second bridging piperidinyl or 1,3-diazinanyl, wherein the second bridging piperidinyl or 1,3-diazinanyl is optionally substituted by one or more -C₁-C₃ alkyl or -C₁-C₃ alkoxy, more preferably -C₁-C₂ alkyl or -C₁-C₂ alkoxy; or
(iv) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging cyclobutyl, cyclopentyl, cyclohexyl, or piperidinyl ring, wherein each cyclobutyl, cyclopentyl, cyclohexyl, or piperidinyl ring is optionally substituted with one or more -C₁-C₃ alkyl or -C₁-C₃ alkoxy, more preferably -C₁-C₂ alkyl or -C₁-C₂ alkoxy; or
(ii) X³ and X⁶ are both chemical bonds; R^{1A}, R¹, R^{2A}, R^{4A}, R⁴, and R^{5A} are -H; R² and R⁵ combine to form a first bridging cyclohexyl or cycloheptyl ring, wherein each cyclohexyl or cycloheptyl is optionally substituted with one or more -C₁-C₃ alkyl or -C₁-C₃ alkoxy, more preferably -C₁-C₂ alkyl or -C₁-C₂ alkoxy; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging pyrrolidinyl, piperidinyl, or oxazinanyl preferably a bridging pyrrolidinyl, piperidinyl, or 1,4 oxazinanyl, wherein each pyrrolidinyl, piperidinyl, or oxazinanyl is optionally substituted with one or more -C₁-C₃ alkyl or -C₁-C₃ alkoxy, more preferably -C₁-C₂ alkyl or -C₁-C₂ alkoxy; wherein
   when R¹ and R⁴ or R² and R⁵ combine to form a first bridging cyclohexyl or piperidinyl, the first bridging cyclohexyl or piperidinyl can independently combine with R⁵¹ to form a second bridging piperidinyl or 1,3-diazinanyl, wherein the second bridging piperidinyl or 1,3-diazinanyl is optionally substituted by one or more -C₁-C₃ alkyl or -C₁-C₃ alkoxy, more preferably -C₁-C₂ alkyl or -C₁-C₂ alkoxy; or
(iv) exactly one of X³ and X⁶ is a chemical bond.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging cyclobutyl, cyclopentyl, cyclohexyl, or piperidinyl ring, wherein each cyclobutyl, cyclopentyl, cyclohexyl, or piperidinyl ring is optionally substituted with one or more -C₁-C₃ alkyl, preferably one or two -C₁-C₂ alkyl; or
(ii) X³ and X⁶ are both chemical bonds; R^{1A}, R¹, R^{2A}, R^{4A}, R⁴, and R^{5A} are -H; R² and R⁵ combine to form a first bridging cyclohexyl or cycloheptyl ring, wherein each cyclohexyl or cycloheptyl is optionally substituted with one or more -C₁-C₃ alkyl, preferably one or two -C₁-C₂ alkyl; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging pyrrolidinyl, piperidinyl, or oxazinanyl, preferably a bridging pyrrolidinyl, piperidinyl, or 1,4 oxazinanyl, wherein each pyrrolidinyl, piperidinyl, or oxazinanyl is optionally substituted with one or more -C₁-C₃ alkyl, preferably one or two -C₁-C₂ alkyl; wherein
   when R¹ and R⁴ or R² and R⁵ combine to form a first bridging cyclohexyl or piperidinyl, the first bridging cyclohexyl or piperidinyl can independently combine with R⁵¹ to form a second bridging piperidinyl or 1,3-diazinanyl, wherein the second bridging piperidinyl or 1,3-diazinanyl is optionally substituted by one or more -C₁-C₃ alkyl, preferably one or two -C₁-C₂ alkyl; or
(iv) exactly one of X³ and X⁶ is a chemical bond, optionally wherein any of R¹, R², R⁴, R⁵ and the remaining R³ or R⁶ can combine to form a bridging C₄-C₈ cycloalkyl or bridging 5 to 8-membered heterocycloalkyl.

In some embodiments, X¹ is CR¹R^{1A}; X² is CR²R^{2A}; X⁴ is CR⁴R^{4A}; X⁵ is CR⁵R^{5A};
(i) X³ and X⁶ are both chemical bonds; R^{1A}, R², R^{2A}, R^{4A}, R⁵, and R^{5A} are -H; R¹ and R⁴ combine to form a first bridging cyclobutyl, cyclopentyl, cyclohexyl, or piperidinyl ring, wherein each cyclobutyl, cyclopentyl, cyclohexyl, or piperidinyl ring is optionally substituted with one or more -C₁-C₃ alkyl, preferably one or two -C₁-C₂ alkyl; or
(ii) X³ and X⁶ are both chemical bonds; R^{1A}, R¹, R^{2A}, R^{4A}, R⁴, and R^{5A} are -H; R² and R⁵ combine to form a first bridging cyclohexyl or cycloheptyl ring, wherein each cyclohexyl or cycloheptyl is optionally substituted with one or more -C₁-C₃ alkyl, preferably one or two -C₁-C₂ alkyl; or
(iii) X³ and X⁶ are both chemical bonds; R^{1A}, R², R^{2A}, R^{4A}, R⁵, and R^{5A} are -H; one of R¹ and R⁴ combine with R⁵¹ to form a bridging pyrrolidinyl, piperidinyl, or oxazinanyl, preferably a bridging pyrrolidinyl, piperidinyl, or 1,4 oxazinanyl, wherein each pyrrolidinyl, piperidinyl, or oxazinanyl is optionally substituted with one or more -C₁-C₃ alkyl, preferably one or two -C₁-C₂ alkyl; wherein
   when R¹ and R⁴ or R² and R⁵ combine to form a first bridging cyclohexyl or piperidinyl, the first bridging cyclohexyl or piperidinyl can independently combine with R⁵¹ to form a second bridging piperidinyl or 1,3-diazinanyl, wherein the second bridging piperidinyl or 1,3-diazinanyl is optionally substituted by one or more -C₁-C₃ alkyl, preferably one or two -C₁-C₂ alkyl; or
(iv) exactly one of X³ and X⁶ is a chemical bond.

In some embodiments, R⁵¹ is selected from -H, -C₁-C₄ alkyl, and -C₅-C₆ cycloalkyl.

In some embodiments, R⁵¹ is selected from -H, methyl, ethyl, propyl, and *tert*-butyl.

In some embodiments, exactly one of X³ and X⁶ is a chemical bond.

In some embodiments, exactly one of X², X³, X⁵, and X⁶ is a chemical bond.

In some embodiments, exactly one of X³ and X⁶ is a chemical bond and X¹, X², X⁴, and X⁵ are each CR¹R^{1A}, CR²R^{2A}, CR⁴R^{4A}, CR⁵R^{5A}

In some embodiments, X³ is a chemical bond, and R⁵¹ is -H, -C₁-C₆ alkyl, preferably - H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R¹, R^{1A}, R², R^{2A}, R⁴, R^{4A}, R⁵, R^{5A}, R⁶ and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R² and R⁶ combine to form a bridging -C₅-C₈ cycloalkyl, preferably a bridging -C₇-C₈ cycloalkyl; and wherein R¹, R^{1A}, R^{2A}, R⁴, R^{4A}, R⁵, R^{5A}, and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R² and R⁵ combine to form a bridging -C₅-C₈ cycloalkyl, preferably a bridging -C₆-C₇ cycloalkyl; and wherein R¹, R^{1A}, R^{2A}, R⁴, R^{4A}, R^{5A}, R⁶ and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R² and R⁴ combine to form a bridging -C₅-C₈ cycloalkyl, preferably a bridging -C₅-C₆ cycloalkyl; and wherein R¹, R^{1A}, R^{2A}, R^{4A}, R⁵, R^{5A}, R⁶ and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R¹ and R⁶ combine to form a bridging -C₅-C₈ cycloalkyl, preferably a bridging -C₆-C₇ cycloalkyl; and wherein R^{1A}, R², R^{2A}, R⁴, R^{4A}, R⁵, R^{5A}, and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R¹ and R⁵ combine to form a bridging -C₅-C₈ cycloalkyl, preferably a bridging -C₅-C₆ cycloalkyl; and wherein R^{1A}, R², R^{2A}, R⁴, R^{4A}, R^{5A}, R⁶ and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ is a chemical bond; X¹, X², X⁴, X⁵, and X⁶ are each CR¹R^{1A}, CR²R^{2A}, CR⁴R^{4A}, CR⁵R^{5A} and CR⁶R^{6A}, wherein R¹ and R⁴ combine to form a bridging -C₅-C₈ cycloalkyl, preferably a bridging -C₅ cycloalkyl; and wherein R^{1A}, R², R^{2A}, R^{4A}, R⁵, R^{5A}, R⁶ and R^{6A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond, and R⁵¹ is -H, -C₁-C₆ alkyl, preferably - H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R¹, R^{1A}, R², R^{2A}, R³, R^{3A}, R⁴, R^{4A}, R⁵ and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R³ and R⁵ combine to form a bridging -C₅-C₈ cycloalkyl, preferably a bridging -C₇-C₈ cycloalkyl; and wherein R¹, R^{1A}, R², R^{2A}, R^{3A}, R⁴, R^{4A}, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R² and R⁵ combine to form a bridging -C₅-C₈ cycloalkyl, preferably a bridging -C₆-C₇ cycloalkyl; and wherein R¹, R^{1A}, R^{2A}, R³, R^{3A}, R⁴, R^{4A}, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R¹ and R⁵ combine to form a bridging -C₅-C₈ cycloalkyl, preferably a bridging -C₅-C₆ cycloalkyl; and wherein R^{1A}, R², R^{2A}, R³, R^{3A}, R⁴, R^{4A}, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R³ and R⁴ combine to form a bridging -C₅-C₈ cycloalkyl, preferably a bridging -C₆-C₇ cycloalkyl; and wherein R¹, R^{1A}, R², R^{2A}, R^{3A}, R^{4A}, R⁵, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R² and R⁴ combine to form a bridging -C₅-C₈ cycloalkyl, preferably a bridging -C₅-C₆ cycloalkyl; and wherein R¹, R^{1A}, R^{2A}, R³, R^{3A}, R^{4A}, R⁵, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X⁶ is a chemical bond; X¹, X², X³, X⁴, and X⁵ are each CR¹R^{1A}, CR²R^{2A}, CR³R^{3A}, CR⁴R^{4A} and CR⁵R^{5A}, wherein R¹ and R⁴ combine to form a bridging -C₅-C₈ cycloalkyl, preferably a bridging -C₅ cycloalkyl; and wherein R^{1A}, R², R^{2A}, R³, R^{3A}, R^{4A}, R⁵, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₃ alkyl, more preferably -H or -C₁-C₂ alkyl.

In some embodiments, X³ and X⁶ are both chemical bonds.

In some embodiments, X³ and X⁶ are both chemical bonds and X¹, X², X⁴, and X⁵ are each CR¹R^{1A}, CR²R^{2A}, CR⁴R^{4A}, and CR⁵R^{5A}.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging C₄-C₈ cycloalkyl, preferably a C₄-C₆ cycloalkyl, more preferably a C₅-C₆ cycloalkyl, and wherein R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is -H, -C₁-C₆ alkyl, or -C₃-C₈ cycloalkyl, preferably -H, -C₁-C₄ alkyl or C₅-C₆ cycloalkyl, more preferably -H, -C₁-C₂ alkyl or -C₅-C₆ cycloalkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging C₄-C₈ cycloalkyl, preferably a C₄-C₆ cycloalkyl, more preferably a C₅-C₆ cycloalkyl, and wherein R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is -H or -C₁-C₆ alkyl, preferably -H or -C₁-C₄ alkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging C₅-C₈ heterocycloalkyl, preferably a bridging C₆ heterocycloalkyl, wherein said bridging heterocycloalkyl is optionally substituted with one or more -C₁-C₆ alkyl, preferably -C₁-C₃ alkyl; R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is -H, or -C₁-C₆ alkyl, preferably -H, or -C₁-C₄ alkyl, more preferably -H, or -C₁-C₃ alkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging C₅-C₈ heterocycloalkyl, preferably a bridging C₆ heterocycloalkyl, wherein said bridging heterocycloalkyl comprises one or more heteroatoms selected from N and O, preferably one or two heteroatoms selected from N and O, preferably one heteroatom selected from N and O and is optionally substituted with one or more -C₁-C₆ alkyl, preferably -C₁-C₃ alkyl; R^{1A}, R², R^{2A}, R^{4A}, R⁵, and R^{5A} are each -H or -C₁-C₆ alkyl, preferably -H or -C₁-C₂ alkyl, more preferably - H; and R⁵¹ is -H, or -C₁-C₆ alkyl, preferably -H, or -C₁-C₄ alkyl, more preferably -H, or -C₁-C₃ alkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging piperidine, wherein said piperidine is optionally substituted at N with -C₁-C₆ alkyl, preferably -C₁-C₃ alkyl; R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is -H, or -C₁-C₆ alkyl, preferably -H, or -C₁-C₄ alkyl, more preferably -H, or -C₁-C₃ alkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging C₄-C₈ cycloalkyl or bridging C₄-C₈ heterocycloalkyl, preferably a C₄-C₆ cycloalkyl or C₄-C₆ heterocycloalkyl, and wherein R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is connected to the bridging C₄-C₆ cycloalkyl or C₄-C₆ heterocycloalkyl formed by R¹ and R⁴.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging cyclohexane or cyclopentane, and wherein R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is connected to the bridging cyclohexane or cyclopentane formed by R¹ and R⁴ by a -C₁-C₂ alkylene, preferably by a -C₁ alkylene.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R¹ and R⁴ combine to form a bridging piperidine or pyrrolidine, and wherein R^{1A}, R², R^{2A}, R^{4A}, R⁵, and R^{5A} are each -H or -C₁-C₆ alkyl, preferably -H or -C₁-C₂ alkyl, more preferably -H; and R⁵¹ is connected to the bridging piperidine or pyrrolidine formed by R¹ and R⁴ by a -C₁-C₂ alkylene, preferably by a -C₁ alkylene.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₄-C₈ cycloalkyl, preferably a C₆-C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is -H, -C₁-C₆ alkyl, or -C₃-C₈ cycloalkyl, preferably -H, -C₁-C₄ alkyl or C₅-C₆ cycloalkyl, more preferably -H, -C₁-C₃ alkyl or -C₅-C₆ cycloalkyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is methyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is isopropyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is propyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₇ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is cyclopentyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein R² and R⁵ combine to form a bridging C₆ cycloalkyl; R¹, R^{1A}, R^{2A}, R⁴, R^{4A}, and R^{5A} are each -H or -C₁-C₆ alkyl, preferably -H or -C₁-C₂ alkyl, more preferably -H; and R⁵¹ is methyl.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine to form a bridging C₄-C₈ cycloalkyl or 4-8 membered heterocycloalkyl with R⁵¹, preferably a C₅-C₆ cycloalkyl or 5-6 membered heterocycloalkyl, and wherein R^{1A}, R², R^{2A}, R^{4A}, R⁵, and R^{5A} are each -H or - C₁-C₆ alkyl, preferably -H or -C₁-C₂ alkyl, more preferably -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine to form a bridging C₄-C₈ cycloalkyl with R⁵¹, preferably a C₅-C₆ cycloalkyl, and wherein R^{1A}, R², R^{2A}, R^{4A}, R⁵, and R^{5A} are each -H or -C₁-C₆ alkyl, preferably -H or -C₁-C₂ alkyl, more preferably -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine to form a bridging 4-8 membered heterocycloalkyl with R⁵¹, preferably a 5-6 membered heterocycloalkyl, and wherein R^{1A}, R², R^{2A}, R^{4A}, R⁵, and R^{5A} are each -H or -C₁-C₆ alkyl, preferably -H or -C₁-C₂ alkyl, more preferably -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine to form a bridging 4-8 membered heterocycloalkyl with R⁵¹, preferably a 5-6 membered heterocycloalkyl, wherein the bridging heterocycloalkyl comprises one or more heteroatoms selected from N and O, preferably one or two heteroatoms selected from N and O, preferably one heteroatom selected from N and O and wherein R^{1A}, R², R^{2A}, R^{4A}, R⁵, and R^{5A} are each -H or -C₁-C₆ alkyl, preferably -H or -C₁-C₂ alkyl, more preferably -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine to form a bridging 1,3 oxazine ring, and wherein R^{1A}, R², R^{2A}, R^{4A}, R⁵, and R^{5A} are each -H or -C₁-C₆ alkyl, preferably -H or -C₁-C₂ alkyl, more preferably -H.

In some embodiments, X³ and X⁶ are both chemical bonds; X¹, X², X⁴, and X⁵ are each CR¹R^{1A}, CR²R^{2A}, CR⁴R^{4A}, and CR⁵R^{5A}, wherein one of R¹ and R⁴ combine with R⁵¹ to form a bridging 1,4 oxazine ring, and wherein R^{1A}, R², R^{2A}, R^{4A}, R⁵, and R^{5A} are each -H or -C₁-C₆ alkyl, preferably -H or -C₁-C₂ alkyl, more preferably -H.

In some embodiments, the compound is of Formula IA, IB, IC, ID, or IE: 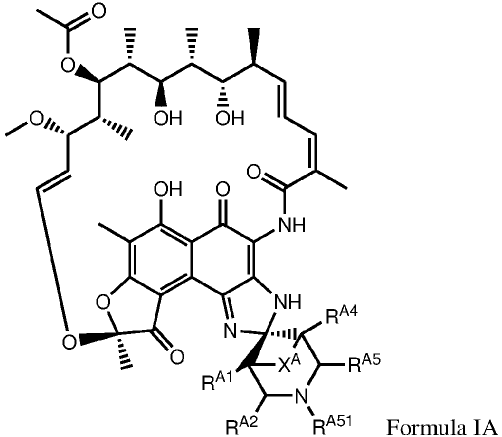 wherein:
R^{A1}, R^{A2}, R^{A4} and R^{A5} are each independently -H or -C₁-C₄ alkyl;
X^{A} is independently selected from -CH₂-, -CH₂CH₂-, -CH₂CH₂CH₂-, -CH₂OCH₂-, - CH₂NHCH₂-, or -CH₂N(C₁-C₄ alkyl)CH₂;
R^{A51} is independently -H or -C₁-C₄ alkyl; or
X^{A} and R^{A51}, together with the atoms to which they are attached, combine to form a 5 to 6-membered heterocycloalkyl; 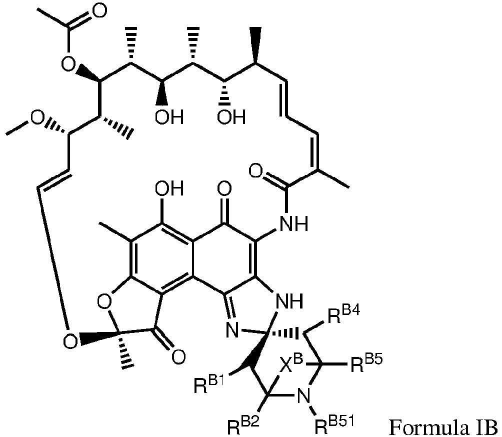 wherein:
   R^{B1}, R^{B2}, R^{B4} and R^{B5} are each independently -H or -C₁-C₄ alkyl;
   X^{B} is independently selected from -CH₂-, and -CH₂CH₂-;
   R^{B51} is independently -H or -C₁-C₄ alkyl; 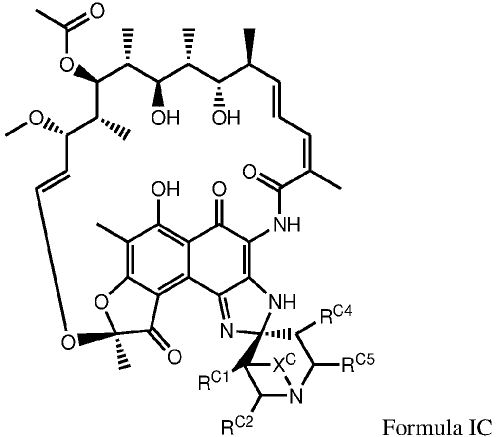 wherein:
      R^{C1}, R^{C2}, R^{C4} and R^{C5} are each independently -H or -C₁-C₄ alkyl;
      X^{c} is independently selected from -CH₂CH₂-, -CH₂CH₂CH₂-, -OCH₂CH₂-, and - NHCH₂CH₂-; 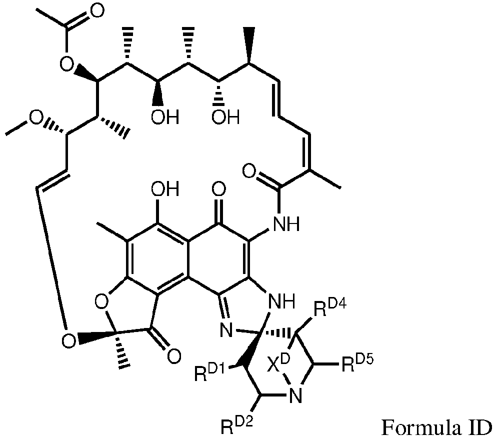 wherein:
         R^{D1}, R^{D2}, R^{D4} and R^{D5} are each independently -H or -C₁-C₄ alkyl;
         X^{D} is independently selected from -CH₂CH₂-, -CH₂CH₂CH₂-, -OCH₂CH₂-, and - NHCH₂CH₂-; 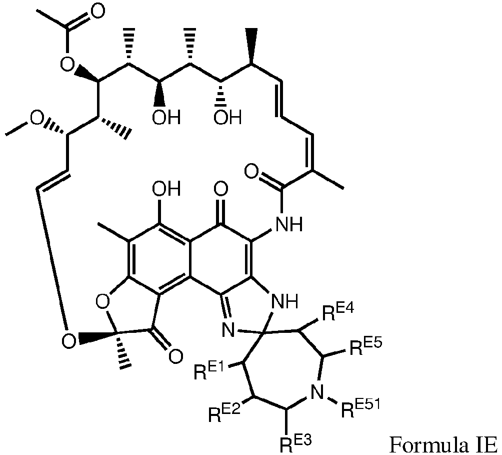 wherein:
            R^{E1}, R^{E2}, and R^{E4} are each -H or C₁-C₄ alkyl; or
            R^{E3} and R^{E5} are H, or combine to form a bridging cyclooctane; and;
            R^{E51} is -H or -C₁-C₄ alkyl.

In some embodiments, the compound is of Formula IA. In some embodiments, the compound is of Formula IB. In some embodiments, the compound is of Formula IC. In some embodiments, the compound is of Formula ID. In some embodiments, the compound is of Formula IE.

In some embodiments, X¹, X², X³, X⁴, X⁵, X⁶ and NR⁵¹ form any structure selected from: 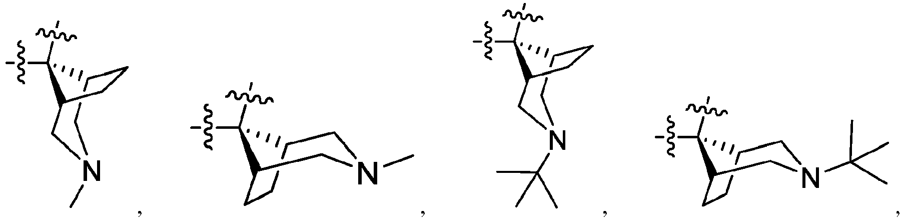 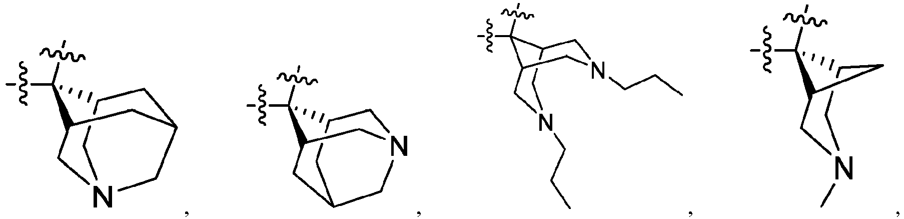 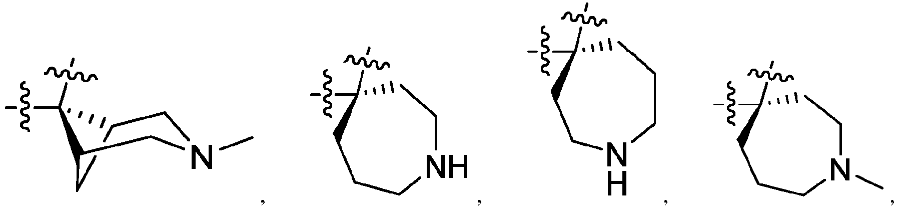 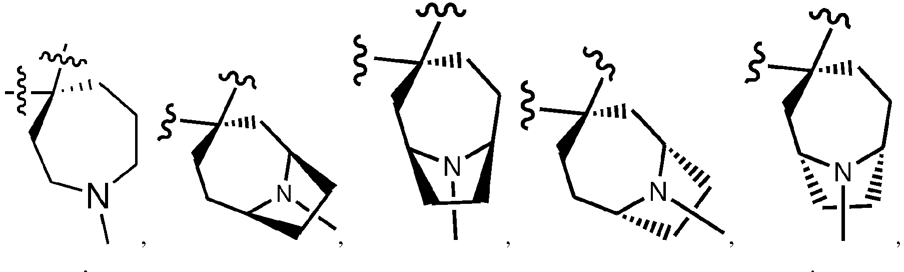 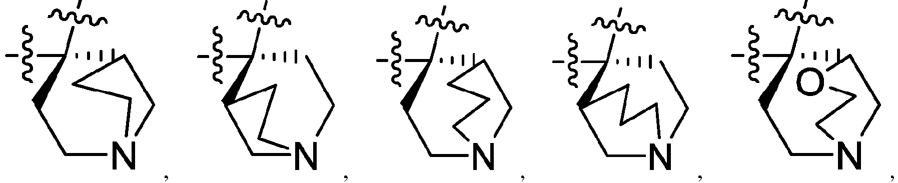 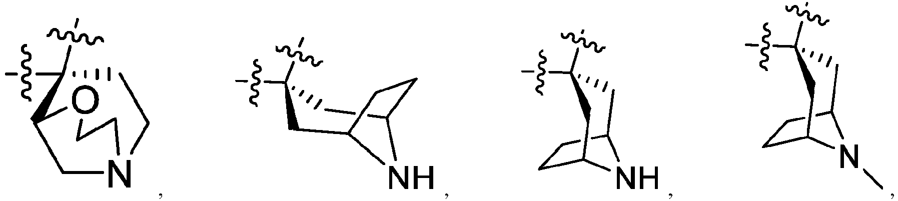 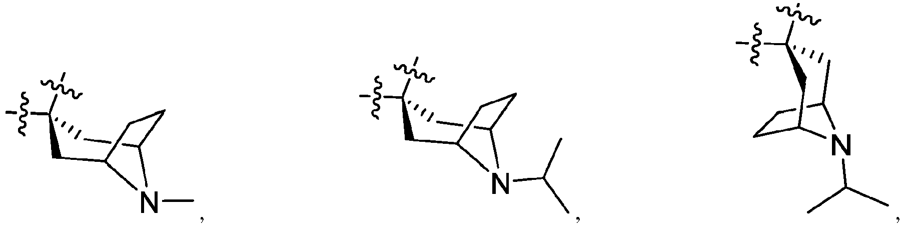 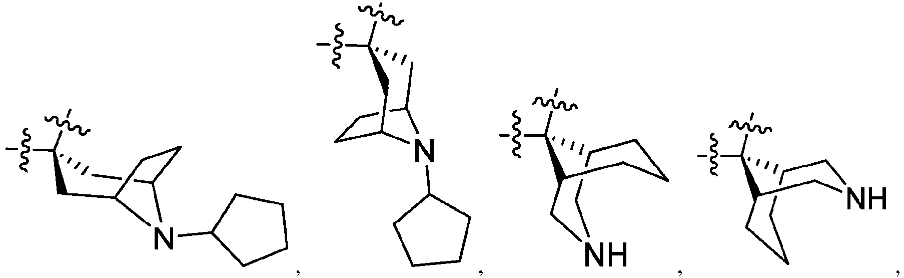 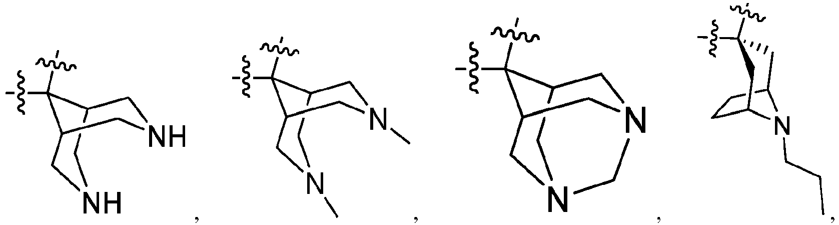 and

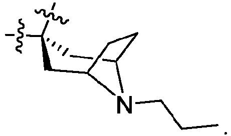

In some embodiments, the compound is selected from the group consisting of: 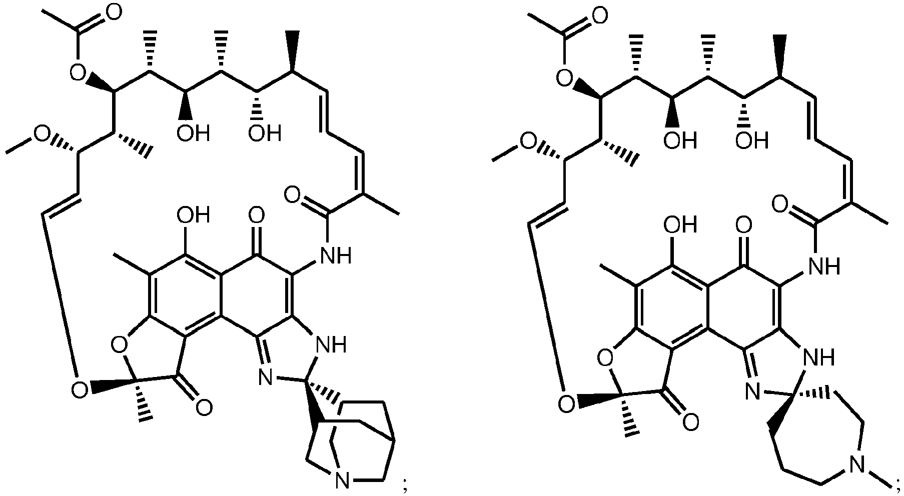 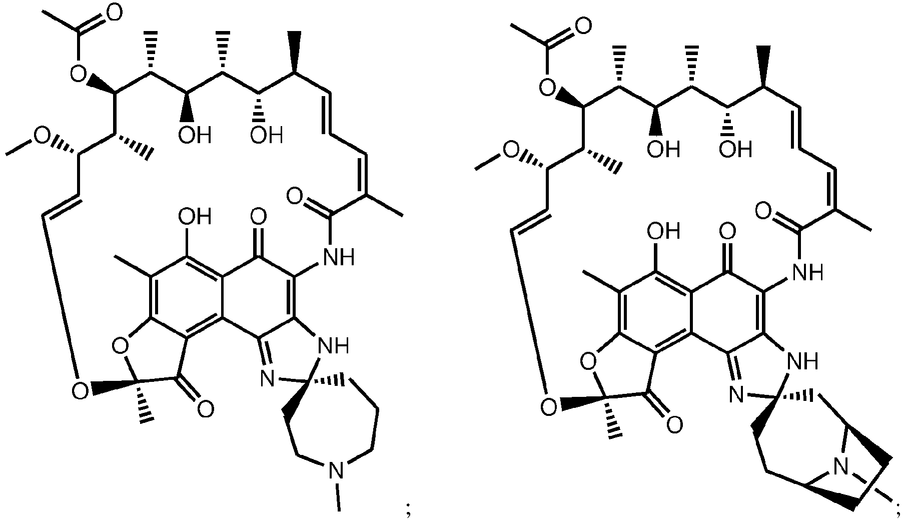 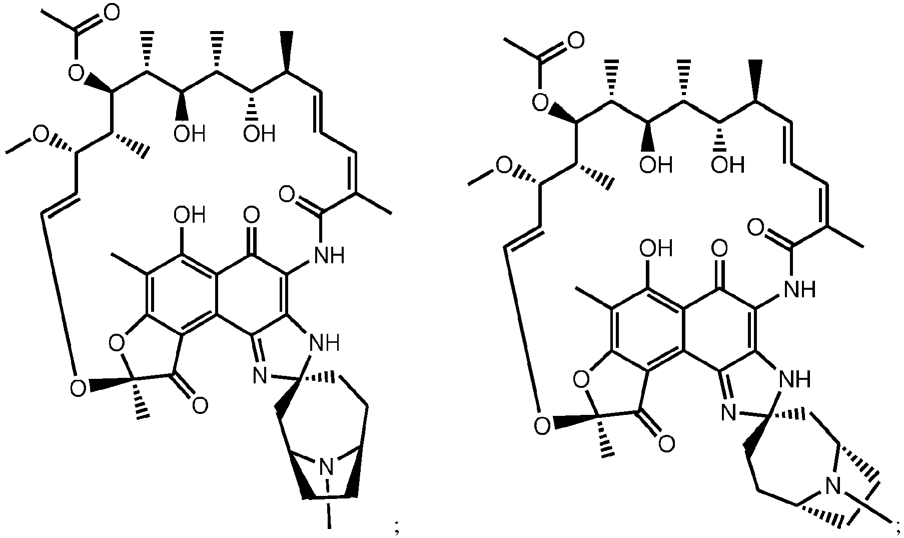 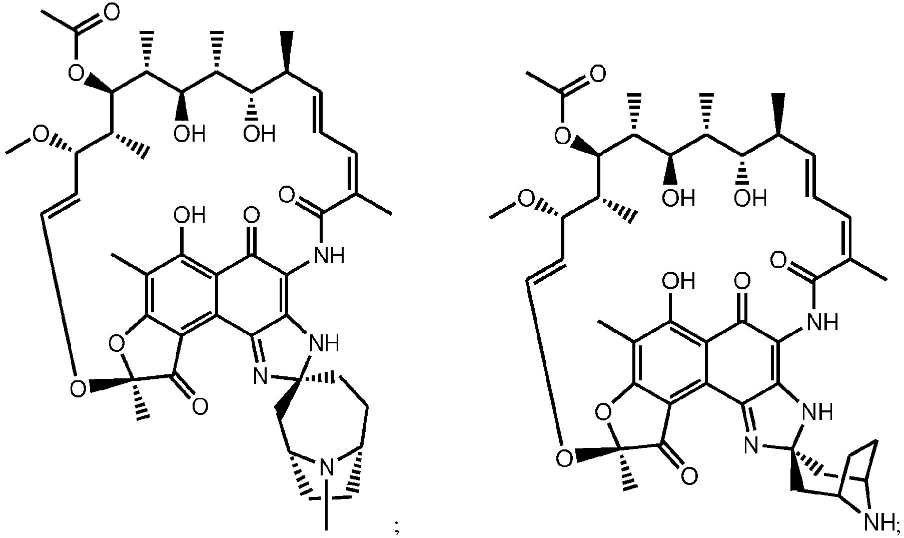 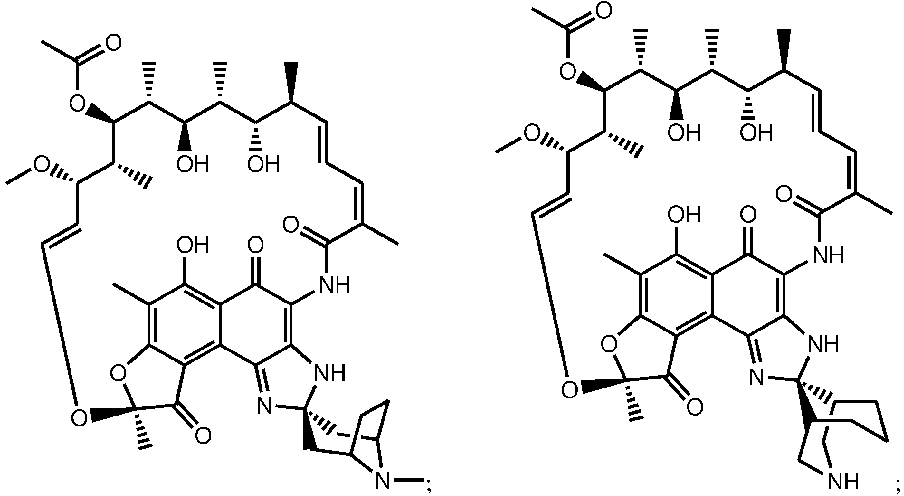 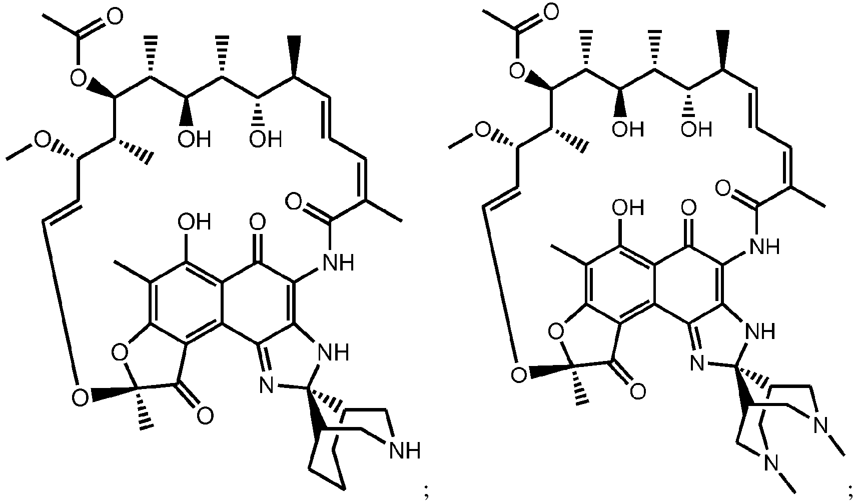 and

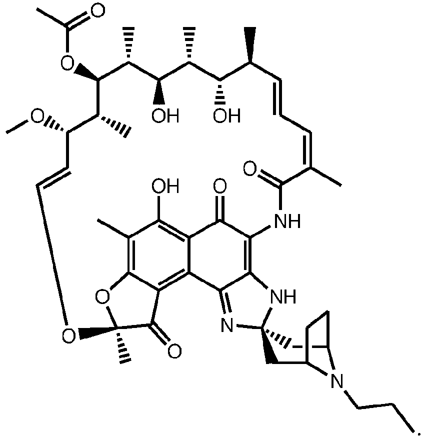

In some embodiments, the compound is selected from the group consisting of: 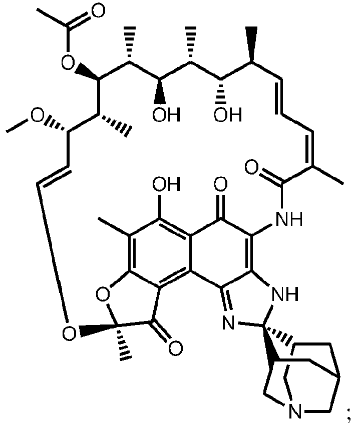 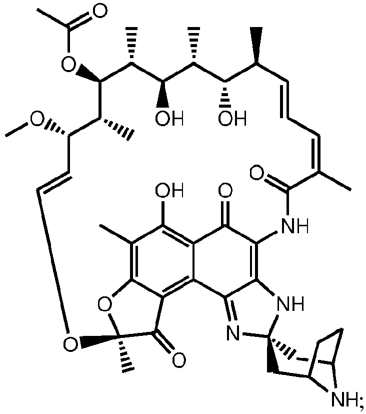 and

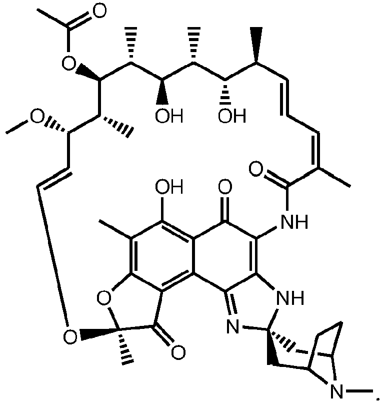

In some embodiments, the compound is:

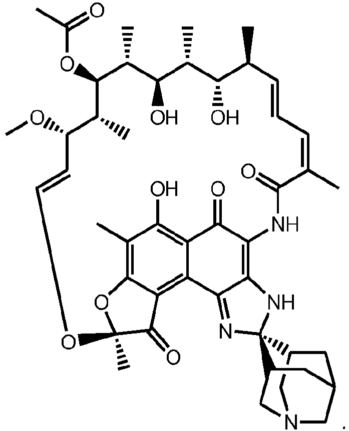

In some embodiments, the compound is:

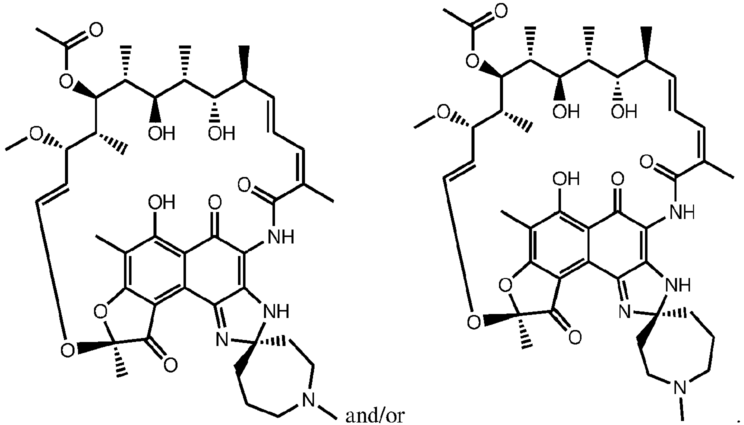

In some embodiments, the compound is:

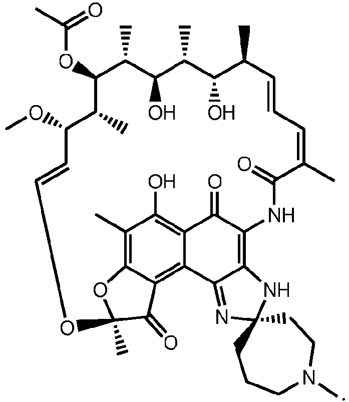

In some embodiments, the compound is:

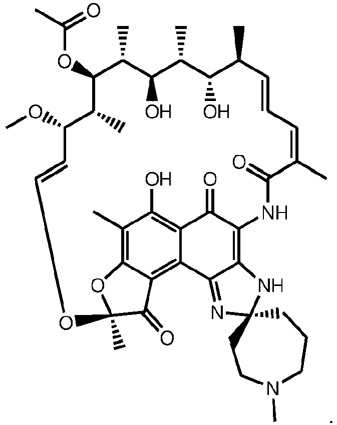

In some embodiments, the compound is:

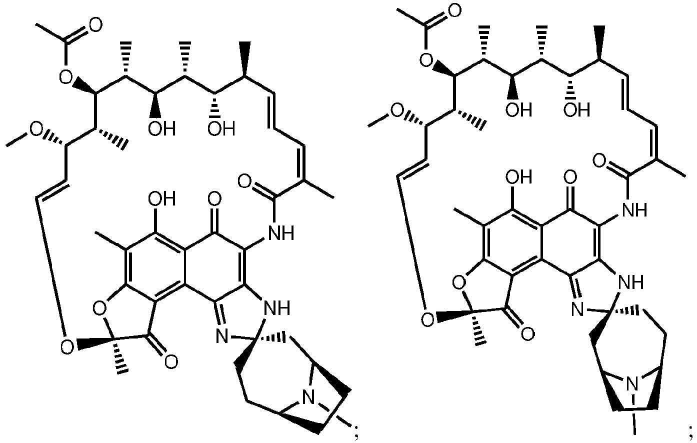

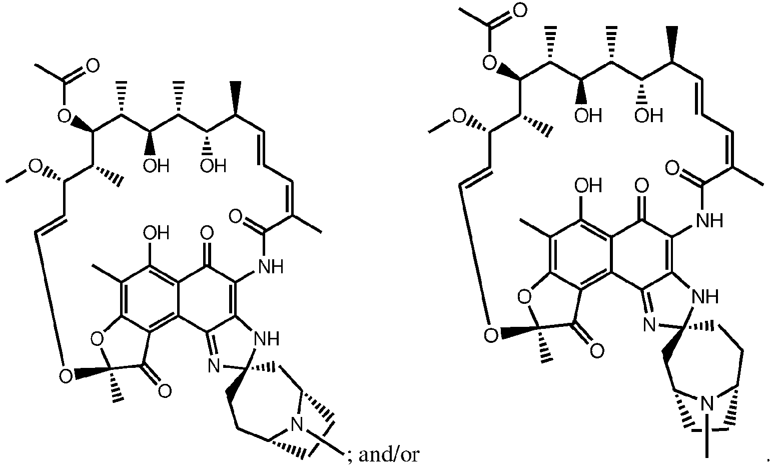

In some embodiments, the compound is:

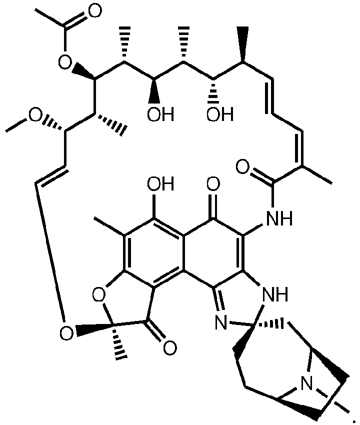

In some embodiments, the compound is:

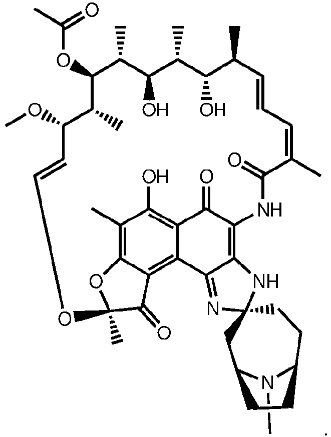

In some embodiments, the compound is:

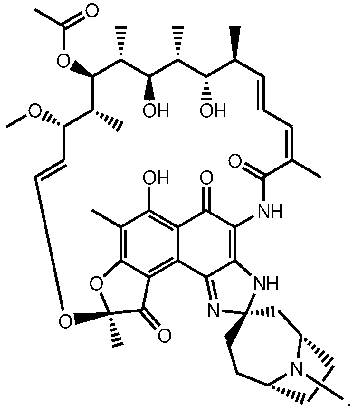

In some embodiments, the compound is:

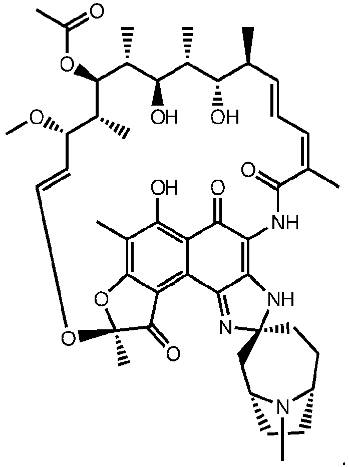

In some embodiments, the compound is:

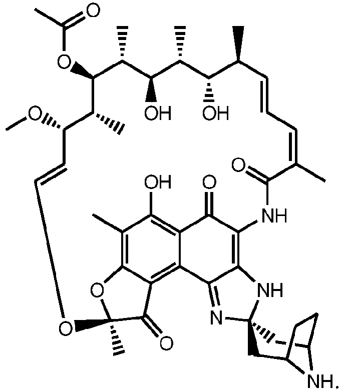

In some embodiments, the compound is:

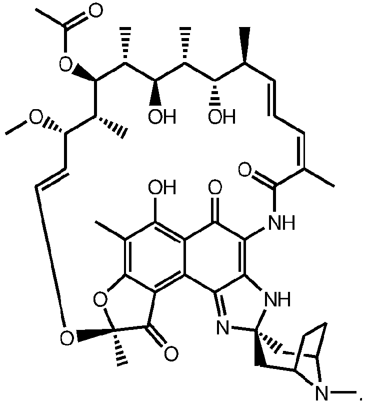

In some embodiments, the compound is:

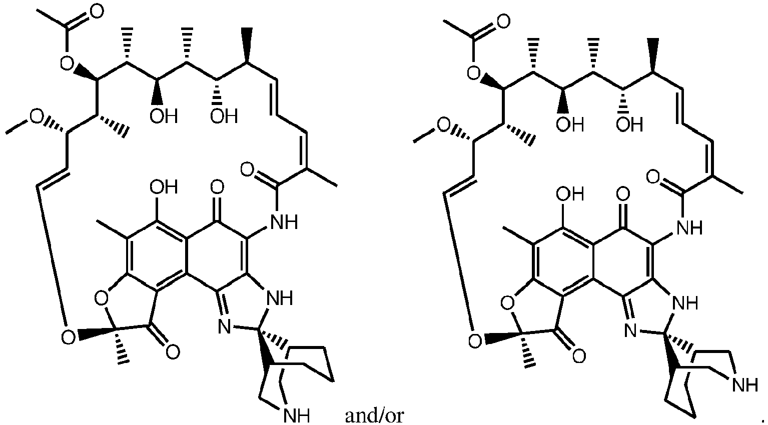

In some embodiments, the compound is:

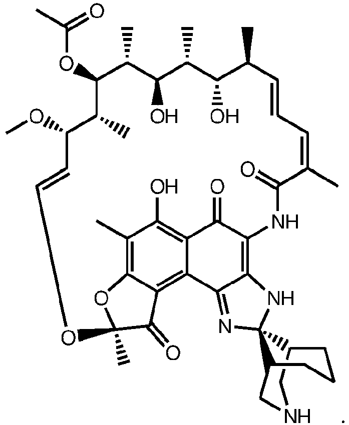

In some embodiments, the compound is:

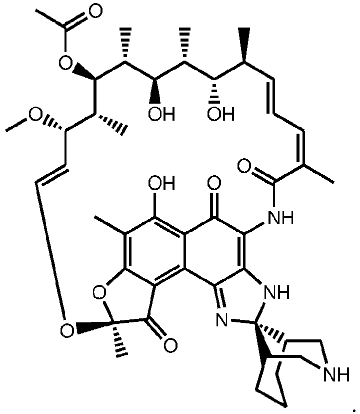

In some embodiments, the compound is:

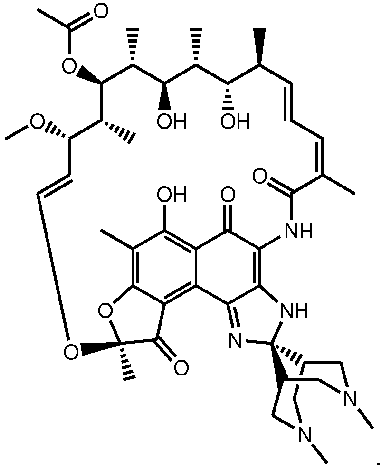

In some embodiments, the compound is:

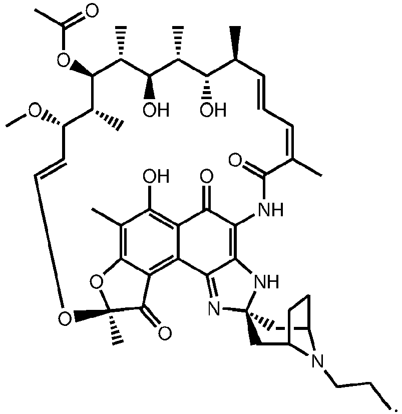

In some embodiments, the compound is Compound 1A.

In some embodiments, the compound is Compound 1B.

In some embodiments, the compound is Compound 2A.

In some embodiments, the compound is Compound 2B.

In some embodiments, the compound is Compound 3A.

In some embodiments, the compound is Compound 3B.

In some embodiments, the compound is Compound 4.

In some embodiments, the compound is Compound 5A.

In some embodiments, the compound is Compound 5B.

In some embodiments, the compound is Compound 6A.

In some embodiments, the compound is Compound 6B.

In some embodiments, the compound is Compound 7A.

In some embodiments, the compound is Compound 7B.

In some embodiments, the compound is Compound 8A.

In some embodiments, the compound is Compound 8B.

In some embodiments, the compound is Compound 8C.

In some embodiments, the compound is Compound 8D.

In some embodiments, the compound is Compound 9A.

In some embodiments, the compound is Compound 9B.

In some embodiments, the compound is Compound 10A.

In some embodiments, the compound is Compound 10B.

In some embodiments, the compound is Compound 11A.

In some embodiments, the compound is Compound 11B.

In some embodiments, the compound is Compound 12A.

In some embodiments, the compound is Compound 12B.

In some embodiments, the compound is Compound 13A.

In some embodiments, the compound is Compound 13B.

In some embodiments, the compound is Compound 14A.

In some embodiments, the compound is Compound 14B.

In some embodiments, the compound is Compound 15A.

In some embodiments, the compound is Compound 15B.

In some embodiments, the compound is Compound 16A.

In some embodiments, the compound is Compound 16B.

In some embodiments, the compound is Compound 17.

In some embodiments, the compound is Compound 18.

In some embodiments, the compound is Compound 19.

In some embodiments, the compound is Compound 20A.

In some embodiments, the compound is Compound 20B.

### Methods of Synthesizing the Disclosed Compounds

The compounds of the present disclosure can be prepared by methods known in the art of organic synthesis as set forth in part by the following synthetic schemes and examples below, together with synthetic methods known in the art of synthetic organic chemistry, or variations thereon as appreciated by those skilled in the art. The methods include but are not limited to those methods described below. Starting materials are either commercially available or made by known procedures reported in the literature or as illustrated.

In the schemes and examples described below, it is well understood that protecting groups for sensitive or reactive groups are employed where necessary in accordance with general principles of chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of the present disclosure.

Those skilled in the art will recognize if a stereocenter exists in the compounds of Formula I. Accordingly, the present disclosure includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley - Interscience, 1994).

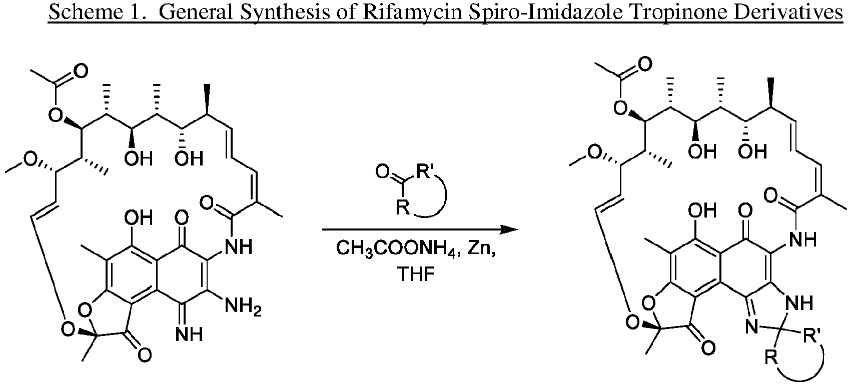

A general method of preparing compounds of Formula (I) is outlined in Scheme 1. To a suspension of ammonium acetate (2.5eq) and zinc (2.5eq) in THF was added 3-amino-4-deoxy-4-imino-rifamycin S (leq), ketone (2.5eq) and acetic acid (20eq). The reaction mixture was stirred at room temperature or elevated temperature and monitored by LC/MS. Upon completion, the mixture was filtered and evaporated. The desired compounds were either tested for antimicrobial activity without further purification or purified by flash chromatography and tested for antimicrobial activity.

### Methods of Using the Disclosed Compounds

The inventive compounds are rifamycin derivatives containing modified spiro-imidazole moieties at the 3,4-imino-amino positions and that exhibit broad spectrum antibacterial activity characteristic of the rifamycin class. Additionally, the inventive compounds unexpectedly showed enhanced antibacterial activity against bacteria of the genu s *Acinetobacter.*

Without wishing to be bound by theory, compounds of the rifamycin class other than rifabutin are not generally active against infections caused by *A. baumannii.* Surprisingly, the compounds set forth herein showed activity against *A. baumannii* that is as good as or better than activity shown by rifabutin. Also surprisingly, the compounds set forth herein showed better antibacterial activity than rifabutin in *A. baumannii* strains carrying a mutation in the RNA polymerase (RpoB) gene.

As shown in Example 3, Tables 2 and 3A & 3B, the compounds of the invention are effective at inhibiting growth of a broad range of bacteria including *S*. *aureus, A. baumannii, E. faecium, E. faecalis, S. epidermidis, S. pneumoniae, S. pyogenes, H. influenzae, M. abscessus, M. tuberculosis,* and *M. smegmatis.* Moreover, as shown in Tables 4 and 5, compounds of the invention were effective at inhibiting the growth of strains of *A. baumannii* carrying mutations in the RpoB gene that confer reduced activity of rifamycins such as rifampicin and/or rifabutin. For example, Table 4 demonstrates that compounds of the invention exhibited a multi-fold increase in activity compared with rifabutin and rifampicin against *A. baumannii* strains carrying an H535L mutation in the RpoB gene. Table 5 shows additional examples in which compounds of the invention exhibited multi-fold increases in activity against *A*. *baumannii* strains with reduced rifabutin and/or rifampicin activity.

In one aspect, the invention provides a compound or a pharmaceutical composition as described herein, for use as a medicament. In one aspect, the invention provides a compound or a pharmaceutical composition as described herein, for use in a method of preventing or treating a disease in a subject, preferably an infection, further preferably a bacterial infection.

In some embodiments, the present invention provides a method of treating or preventing a disease in a subject in need thereof, the method comprising administering to the subject an effective amount of a compound or pharmaceutical composition of the present invention. In some embodiments, the disease to be treated is an infection, further preferably a bacterial infection.

In some embodiments, the present invention provides the use of a compound or pharmaceutical composition of the present invention in the manufacture of a medicament for treating or preventing a disease in a subject in need thereof. In some embodiments, the disease to be treated is an infection, further preferably a bacterial infection.

Accordingly, in some embodiments, the inventive compounds are used to inhibit bacterial infection. In some embodiments, the infection is caused by a bacteria belonging to *Acinetobacter* spp., *Clostridium* spp., *Enterococcus* spp., *Hemophilus* spp., *Legionella* spp., *Mycobacterium* spp. (tuberculous and non-tuberculous *Mycobacteria*), *Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and/or *Toxoplasma gondii.*

More preferably, the bacterial infection caused by *S*. *aureus, A. baumannii, E. faecium, E. faecalis, S. epidermidis, S. pneumoniae, S. pyogenes, H. influenzae, M. abscessus, M. tuberculosis,* and/or *M. smegmatis.* Yet more preferably the bacterial infection caused by one or more bacterium belonging to the genus *Acinetobacter.* Yet more preferably the bacterial infection is caused by *A. baumannii.* Even more preferably the bacterial infection is caused by *A. baumannii* that carries one or more resistance mechanisms against rifamycins such as rifabutin and/or rifampicin.

In some preferred embodiments, the inventive compounds are used to treat a bacterial infection caused by a strain of bacteria that carries one or more resistance genes or resistance mechanisms against currently available antibiotics (e.g., rifampicin and/or rifabutin). In some preferred embodiments, the infection is caused by a bacteria of the species *A. baumannii,* wherein the *A. baumannii* has a mutation in the RNA polymerase B (RpoB) gene, preferably a mutation that confers reduced activity of an antibiotic, preferably an antibiotic of the rifamycin class, more preferably rifabutin and/or rifampicin. In some preferred embodiments the RpoB mutation is selected from I581M; H535Q, S521T; S583L; N527D; H535C; H535L; L542F; H535N; and combinations thereof. In some preferred embodiments, the mutation in RpoB is H535L.

### Pharmaceutical Compositions

In one aspect, the invention provides a pharmaceutical composition comprising at least one compound according to any of the preceding claims, or a pharmaceutically acceptable salt, tautomer, solvate or hydrate thereof, and a pharmaceutically acceptable excipient.

Illustrative pharmaceutical compositions are tablets and gelatin capsules comprising a compound of the disclosure and a pharmaceutically acceptable carrier, such as a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algiic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

In some embodiments the disclosed compounds are also formulated as a suppository that is prepared from fatty emulsions or suspensions; using polyalkylene glycols such as propylene glycol, as the carrier.

In some embodiments the disclosed compounds are administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Another aspect of the disclosure relates to a pharmaceutical composition comprising a compound of the present disclosure and a pharmaceutically acceptable carrier. The pharmaceutically acceptable carrier can further include an excipient, diluent, or surfactant.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about 0.5 mg to about 5000 mg of the disclosed compound as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

The inventive compounds and pharmaceutical compositions may be administered by any suitable route, depending on the nature of the disease or disorder to be treated, e.g. orally (as syrups, tablets, capsules, lozenges, controlled-release preparations, fast-dissolving preparations, lozenges, etc.); topically (as creams, ointments, lotions, nasal sprays or aerosols, etc.); by injection (subcutaneous, intradermic, intramuscular, intravenous, etc.) or by inhalation (as a dry powder, a solution, a dispersion, etc.).

### Equivalents

While the present technology has been described in conjunction with the specific embodiments set forth above, many alternatives, modifications and other variations thereof will be apparent to those of ordinary skill in the art. All such alternatives, modifications and variations are intended to fall within the scope of the present disclosure.

### EXAMPLES

The invention is now illustrated by way of the following non-limiting examples. While particular embodiments of the invention are described below, a skilled person will appreciate that various changes and modifications can be made. References to preparations carried out in a similar manner to, or by the general method of, other preparations, may encompass variations in routine parameters such as time, temperature, workup conditions, minor changes in reagents amounts, and the like. These alternatives for the synthesis of the derivatives are within the scope of this invention.

### Abbreviations

The following list provides definitions of certain abbreviations and symbols as used herein. It will be appreciated that the list is not exhaustive, but the meaning of those abbreviations and symbols not herein below defined will be readily apparent to those skilled in the art. In describing the invention, chemical elements are identified in accordance with the Periodic Table of the Elements.
- ACN: acetonitrile
- AcOH: acetic acid
- aq.: aqueous
- Ar: argon
- DCM: dichloromethane
- DMF: dimethylformamide
- eq.: equivalent
- EtOAc: ethyl acetate
- EtOH: ethanol
- Et₂O: diethylether
- h: hour(s)
- HPLC: high performance liquid chromatography
- i-PrOH: isopropanol
- LC: liquid chromatography
- M: molar
- min: minutes
- MIC: minimum inhibitory concentration
- MS: mass spectroscopy
- NMR: nuclear magnetic resonance spectroscopy
- rt: room temperature
- T_{R}: Retention time
- sat.: saturated

### Analytical Methods

Unless specified otherwise, the purity and identity of intermediate or example compounds were assessed by HLPC-MS as set forth below.

HPLC Analysis: Reaction monitoring and compound purity determination were performed by liquid chromatography/mass spectrometry (LC/MS). The HPLC system was a Waters 2695 LC with a photodiode array detector Waters 996; and an XBridge C18 column (3.5µm particle size, dimensions 50mm x 4.6mm). The mobile phases were: phase A (H₂O/ammonium formate, pH 3.75) and phase B (CH₃CN + 5% H₂O/ammonium formate, pH 3.75) and compounds were eluted according to the following method:

| Time (min) | Phase A | Phase B | Flow (mL/min) |
|---|---|---|---|
| 0.00 | 100.0 | 0.0 | 2.00 |
| 0.25 | 100.0 | 0.0 | 2.00 |
| 3.50 | 0.0 | 100.0 | 2.00 |
| 4.00 | 0.0 | 100.0 | 2.00 |
| 4.05 | 100.0 | 0.0 | 2.00 |
| 5.00 | 100.0 | 0.0 | 2.00 |

Mass Spectrometry Analysis: The mass spectrometer was a Waters Alliance Micromass ZQ 2000 using electrospray ionization (polarity: negative and positive). Percent purity of compounds was determined by reversed phase HPLC using UV detection (254 nm). Structures were confirmed by MS using electro spray ionization positive (ESI+) method and reported as M+H, referring to the protonated molecular ion.

NMR Analysis: NMR spectra were recorded on a Bruker 500 MHz spectrometer with a TXI probe. Chemical shifts are given in parts per million (ppm). The assignments were made using one-dimensional (1D) ¹H and ¹³C spectra and two-dimensional (2D) HSQC, HMBC and COSY spectra. Stereochemical confirmation was performed by ROESY experiment.

Compound Synthesis: All starting materials were either purchased from commercial sources or prepared according to published procedures. Reagents were purchased from commercial sources and used without further purification. The starting material 3-amino-4-deoxy-4-imino-rifamycin S was purchased from commercial sources.

Unless otherwise described, flash column chromatography purification was performed on prepacked columns (Grace Resolv^{™} flash cartridges, from Grace^{®}, or FlashPure, from BUCHI) using PuriFlash430 system from Interchim. The mobile phase used was CH₃Cl/NH₃ in MeOH (1N) from 100/0 to 95/5. Preparative thin-layer chromatography (TLC) was performed using glass plates (20x20 cm) of silica gel (silica gel 60 with fluorescent indicator UV254, thickness 1mm or 2 mm, from MACHEREY-NAGEL). The mobile phase used was cyclohexane/DCM/MeOH (3/6/1).

Antibacterial Activity: For examples below in which the corresponding starting ketone does not present an axis of symmetry, the condensation reaction can lead to the formation of multiple (e.g., two or four) diastereoisomers of the 3,4-spiro-rifamycin derivatives. The antibacterial activity of the diastereoisomeric compounds were either tested as a mixture (e.g, "A+B"), or isomers were separated by preparative TLC or flash chromatography and tested separately.

### EXAMPLE 1: SYNTHESIS AND ISOLATION OF COMPOUNDS 13A AND 13B

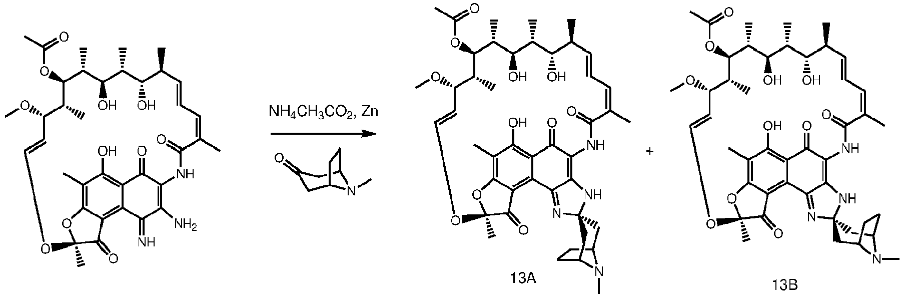

To a suspension of ammonium acetate (136mg, 1.76mmol, 2.5eq) and zinc (115mg, 1.76mmol, 2.5eq) in THF (5mL) was added 3-Amino-4-Imino-Rifamycin S (500mg, 0.70mmol, 1eq), acetic acid (807µL, 14.10mmol, 20eq) and tropinone hydrochloride hydrate (245mg, 1.76mmol, 2.5eq). The reaction was stirred at 40°C. After 1h, the mixture was filtered over sintered glass and the THF was evaporated. The obtained oil was dissolved in EtOAc (50mL) and washed with an aqueous solution of HCl (0.5N, 3x50mL), a saturated solution of NaHCO₃ (3x50mL) and brine (50mL). The organic layer was dried over MgSO₄ and evaporated under vacuum. The obtained solid was purified by flash chromatography (CH₃Cl/NH₃ in MeOH (1N): 100/0 to 94/6) to give two products: 13A, as a purple solid (72mg, yield=12.3%, purity (254nm)=99%) and 13B, as a purple solid (28mg, yield=4.7%, purity (254nm)=99%).

The compounds shown in Table 1 were prepared according to Example 1. The compounds comprise a 3,4 spiro-rifamycin core as shown in the general structure below, and the substitution pattern shown in Table 1.

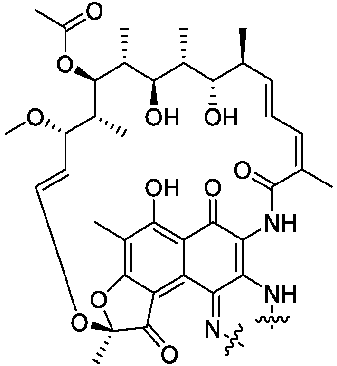

**Table 1. Compounds Prepared According to Example 1.**

| Compound | Structure | MW (g/mol) | T_{R} (min) | ESI+ [M+H]+ |
|---|---|---|---|---|
| 1 (A+B) | 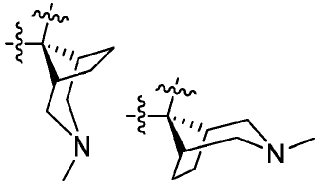 | 830.41 | 2.60 | 831.50 |
| 2 (A+B) | 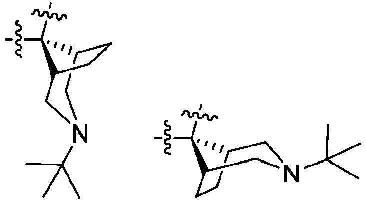 | 872.45 | 2.80 | 873.57 |
| 3A¹ | 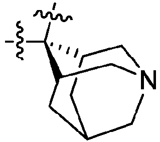 | 842.41 | 2.65 | 843.52 |
| 3B¹ | 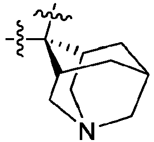 | 842.41 | 2.65 | 843.52 |
| 4 | 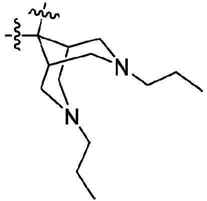 | 915.49 | 2.97 | 916.58 |
| 5 (A+B) | 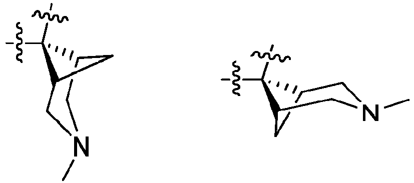 | 816.39 | 2.88 | 817.46 |
| 6 (A+B)² | 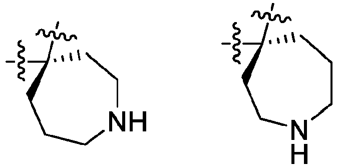 | 804.39 | 2.47 | 805.37 |
| 7 (A+B)² | 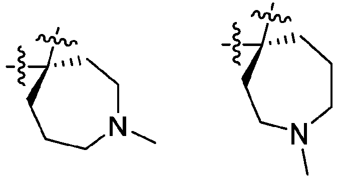 | 818.41 | 2.52 | 819.42 |
| 8 (A, B, C, D) | 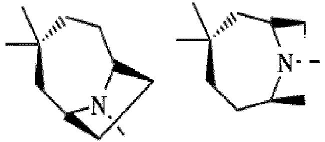 | 844.42 | 2.63 | 845.54 |
| | 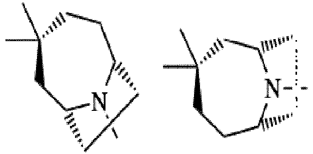 | | | |
| 9 (A+B) | 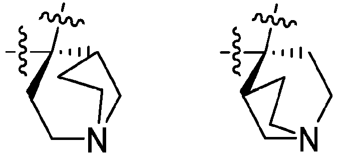 | 816.39 | 2.48 | 817.33 |
| 10 (A+B) | 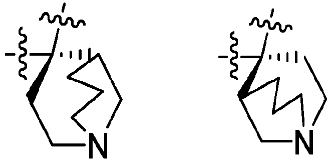 | 830.41 | 2.48 | 831.37 |
| 11 (A+B) | 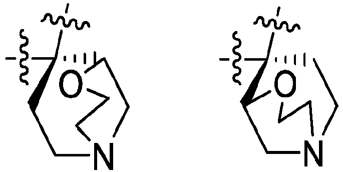 | 842.41 | 2.53 | 833.46 |
| 12A¹ | 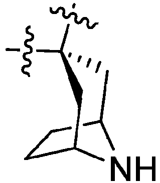 | 816.39 | 2.58 | 817.33 |
| 12B¹ | 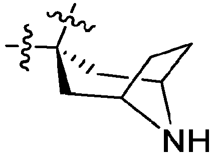 | 816.39 | 2.58 | 817.33 |
| 13A¹ | 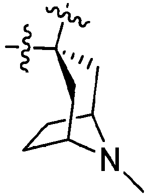 | 830.41 | 2.62 | 831.37 |
| 13B¹ | 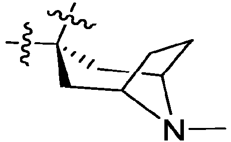 | 830.41 | 2.62 | 831.37 |
| 14A¹ | 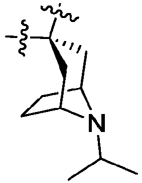 | 858.44 | 2.68 | 859.59 |
| 14B¹ | 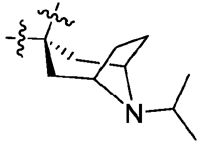 | 858.44 | 2.68 | 859.59 |
| 15 (A+B) | 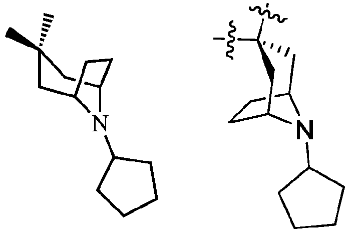 | 884.45 | 2.75 | 885.60 |
| 16 (A+B) | 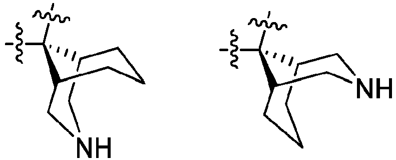 | 830.41 | 2.68 | 831.56 |
| 17 | 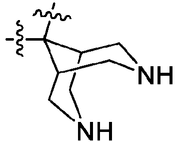 | 831.40 | 2.57 | 832.57 |
| 18 | 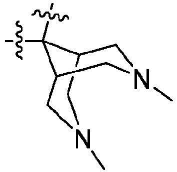 | 859.43 | 2.70 | 860.60 |
| 19 | 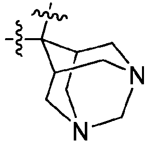 | 843.40 | 2.49 | 844.60 |
| 20A¹ | 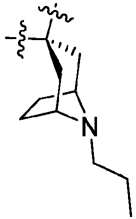 | 858.44 | 2.80 | 859.59 |
| 20B¹ | 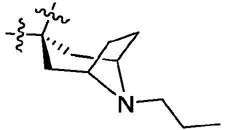 | 858.44 | 2.75 | 859.59 |

| | | | | |
|---|---|---|---|---|
| ¹: Configuration attributed by NMR (¹H, ¹³C, 2D and ROESY) ²: 6A and 7A are the least polar diastereoisomer in TLC using cyclohexane/DCM/MeOH 3/6/1 as eluent, otherwise the definitive configuration has not been attributed for isomers shown in the Table. | | | | |

### EXAMPLE 2: STEREOCHEMICAL ELUCIDATION OF COMPOUNDS 13A AND 13B

The structural characterization of the new rifabutin analogs 13A and 13B was achieved using 1D and 2D NMR spectroscopic experiments through the analysis of their HSQC and HMBC spectra. Atom numbering is shown in the structure below:

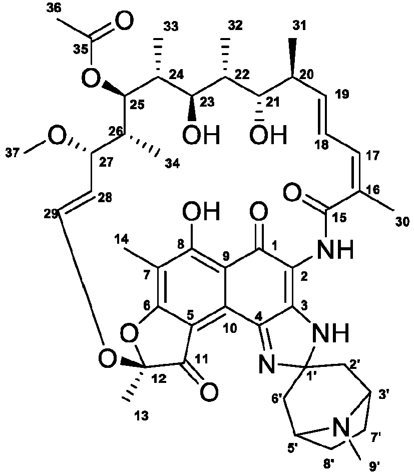

### 1D experiment: ¹H NMR & ¹³C NMR

### Compound 13A

¹H NMR (500 MHz, CDCl₃): -0.04 (d, *J =* 7.08 Hz, 3H), 0.60 (d, *J =* 6.95 Hz, 3H), 0.82 (d, *J* = 7.06 Hz, 3H), 1.02 (d, *J =* 7.06 Hz, 3H), 1.32-1.39 (m, 2H), 1.60 (d, *J =* 13.86 Hz, 1H), 1.73-1.76 (m, 4H), 1.83 (m, 1H), 1.99 (s, 3H), 2.01 (s, 3H), 2.20-2.25 (m, 2H), 2.32 (s, 3H), 2.36 (m, 1H), 2.51 (s, 3H), 2.67 (m, 2H), 2.76 (m, 2H), 2.99 (d, *J =* 9.94 Hz, 1H), 3.08 (s, 3H), 3.31 (dd, *J* = 7.90 Hz, *J =* 2.76 Hz, 1H), 3.36 (bs, 1H), 3.43-3.47 (m, 2H), 3.54 (bs, 1H), 3.64 (d, *J* = 9.94 Hz, 1H), 4.73 (d, *J* = 10.73 Hz, 1H), 5.20 (dd, *J =* 12.69 Hz, *J* = 7.97 Hz , 1H), 5.94 (dd, *J =* 15.69 Hz, *J =* 6.50 Hz, 1H), 6.18 (d, *J =* 12.56 Hz, 1H), 6.21 (d, *J* = 10.35 Hz, 1H), 6.31 (dd, *J =* 15.64 Hz, *J =* 10.18 Hz, 1H), 8.09 (s, 1H), 14.75 (s, 1H) (chemical shifts in ppm).

¹³C NMR (500 MHz, CDCl₃): 7.7, 8.9, 11.3, 11.4, 17.4, 20.4, 21.2, 22.0, 25.3, 25.5, 27.8, 33.1, 37.6, 37.8, 38.6, 39.2, 39.6, 39.8, 56.9, 70.6, 72.5, 73.0, 81.2, 91.0, 104.6, 107.3, 108.9, 111.8, 114.9, 115.7, 124.3, 124.9, 131.4, 133.2, 140.7, 141.3, 144.5, 155.5, 168.2, 168.3, 171.5, 172.1, 181.6, 193.2 (chemical shifts in ppm).

### Compound 13B

¹H NMR (500 MHz, CDCl₃): -0.15 (d, *J =* 7.09 Hz, 3H), 0.53 (d, *J =* 6.94 Hz, 3H), 0.77 (d, J= 6.94 Hz, 3H), 0.96 (d, *J =* 7.01 Hz, 3H), 1.18 (m, 1H), 1.38 (m, 1H), 1.47 (d, *J =* 14.65 Hz, 1H), 1.66-1.70 (m, 5H), 1.95 (s, 3H), 1.99 (s, 3H), 2.02 (m, 1H), 2.14 (m, 1H), 2.25 (s, 3H), 2.32-2.38 (m, 3H), 2.62 (s, 3H), 2.93 (d, *J* = 9.90 Hz, 1H), 2.99-3.05 (m, 6H), 3.31 (dd, *J =* 6.99 Hz, *J* = 2.1 Hz, 1H), 3.53-3.61 (m, 4H), 4.68 (d, *J =* 10.49 Hz, 1H), 5.05 (dd, *J =* 12.43 Hz, *J =* 7.22 Hz, 1H), 5.94 (dd, *J =* 15.93 Hz, *J =* 6.77 Hz, 1H), 6.07 (d, *J* = 12.62 Hz, 1H), 6.18 (d, *J* = 10.49 Hz, 1H), 6.29 (dd, *J =* 15.73 Hz, *J =* 10.48 Hz, 1H), 8.16 (s, 1H), 9.70 (s, 1H), 14.56 (s, 1H) (chemical shifts in ppm).

¹³C NMR (500 MHz, CDCl₃): 7.6, 8.8, 10.8, 11.2, 17.4, 20.2, 21.0, 21.8, 25.4, 25.6, 33.0, 35.9, 36.8, 37.5, 37.7, 37.9, 56.9, 58.6, 58.7, 73.0, 73.1, 76.8, 80.4, 92.0, 104.3, 107.2, 109.0, 111.5, 114.5, 116.0, 123.9, 124.6, 131.3, 132.8, 141.0, 142.2, 144.0, 155.2, 168.1, 168.9, 171.4, 172.2, 180.9,192.5 (chemical shifts in ppm).

### 2D experiment - ROESY NMR (Compound 13B)

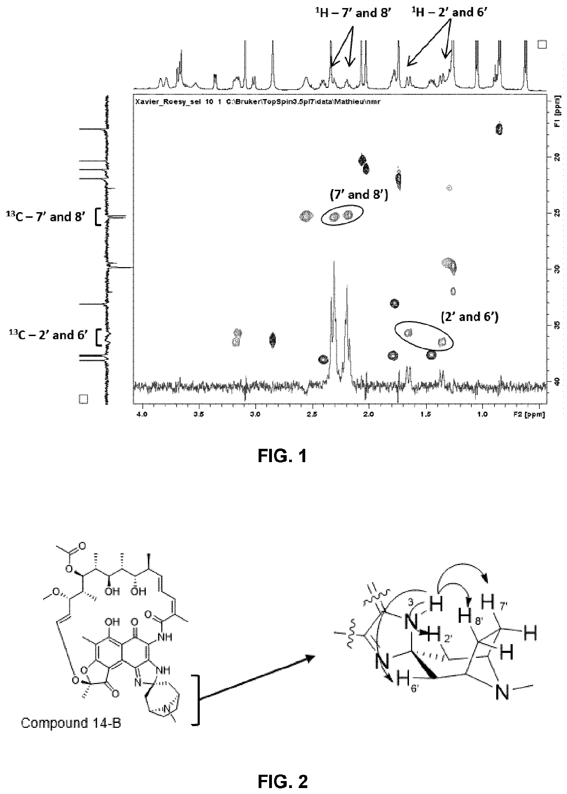

The stereochemistry of the diastereoisomers was assigned by studying the ROESY spectra. ROESY analysis show a correlation between NH-3 and H-7', H-8', H-2' and H-6'. For the minor isomer 13B, the proposed disposition of the ethylenic bridge was supported by NOESY correlation peaks between the amine proton NH-3 and the protons H-7' and H-8' of the ethylenic part of the tropane moiety as shown in FIG. 1. This observation leads to the conclusion that the compound 13B is the diastereomer with the ethylenic moiety oriented toward the NH-3, as depicted in FIG 2.

### EXAMPLE 3: ANTIBACTERIAL ACTIVITY

The *in vitro* antibacterial activity of the compounds disclosed was measured according to the following protocols:
MIC values were determined by broth microdilution method according to the CLSI guidelines. Unless otherwise mentioned, MIC against *Acinetobacter baumannii* was performed in RPMI medium supplemented with 10% FCS. MIC against *Streptococcus pneumoniae* and *Streptococcus pyogenes* was performed in cation adjusted Mueller Hinton broth supplemented with 5% laked horse blood. MIC against *Hemophilus influenzae* was performed in Hemophilus test medium broth. MIC against *Mycobacterium abscessus, Mycobacterium tuberculosis* and *Mycobacterium smegmatis* was performed in Middlebrook 7H9 broth supplemented with Middlebrook ADC growth supplement. All other MIC were performed in standard cation adjusted Mueller Hinton broth.

The following procedure applies to all species tested except *M. tuberculosis* and *M. smegmatis.* From an overnight culture plate, cells were resuspended in 0.9% (w/v) saline solution and bacterial inoculum was prepared in the respective testing medium with 5x10⁵ CFU/mL. The appropriate volumes of a 10 mg/mL compound solution were directly dispensed in the 96-well assay plate using a digital dispenser to create two-fold dilution series from 32 to 0.002 µg/mL final concentration. 100 µL of bacterial suspension was finally added to the compounds. Plates were covered and incubated without shaking at 35 °C for 20 hours. Every experiment contained an antibiotic as quality control. MIC was determined visually as the lowest concentration of a compound that prevents visible growth of the bacteria and the plates were scanned for documentation.

A similar procedure was used for *M. tuberculosis* and *M. smegmatis,* except that the inoculum was prepared from an exponentially growing culture and set to an OD₆₀₀ of 0.02 and 0.002 for *M. tuberculosis* and *M. smegmatis,* respectively. The MIC was determined at 90% growth inhibition using a GFP reporter after 5 days incubation for *M. tuberculosis* and using the BacTiter-Glo kit (Promega) after 20 hours incubation for *M. smegmatis.*

The results are shown below in the following Tables.

**Table 2. In vitro antibacterial activity of compounds.**

| MIC (mg/mL) | *S. aureus* | *A. baumannii* |
|---|---|---|
| Rifabutin | 0.016 | ≤ 0.002 |
| 1 (A+B) | 0.016 | 0.25 |
| 2 (A+B) | 0.008 | 2 |
| 3A | 0.008 | 0.25 |
| 3B | 0.008 | ≤ 0.002 |
| 4 | 0.016 | 0.03 |
| 5 (A+B) | 0.125 | 8 |
| 6A | 0.016 | 0.125 |
| 6B | 0.016 | 0.125 |
| 7A | 0.008 | 0.004 |
| 7B | 0.008 | 0.125 |
| 8 (A+B+C+D) | 0.03 | 0.008 |
| 9 (A+B) | 0.016 | 0.25 |
| 10 (A+B) | 0.008 | 0.06 |
| 11 (A+B) | 0.004 | 4 |
| 12A | 0.06 | 0.06 |
| 12B | 0.016 | ≤ 0.002 |
| 13A | 0.03 | 0.03 |
| 13B | 0.016 | ≤ 0.002 |
| 14A | 0.016 | 0.25 |
| 14B | 0.008 | 0.016 |
| 15 (A+B) | 0.03 | 0.25 |
| 16 (A+B) | 0.004 | ≤ 0.002 |
| 17 | 0.008 | 0.5 |
| 18 | 0.016 | 0.008 |
| 19 | 0.004 | 1 |
| 20A | 0.008 | 0.25 |
| 20B | 0.004 | ≤ 0.002 |

Compounds 3B, 7A, 8(A+B+C+D), 12B, 13B, 16 (A+B), 18 and 20B were tested against the following other representative species: *Enterococcus faecium* (strain NCTC 12204), *Enterococcus faecalis* (strain ATCC 51575), *Staphylococcus epidermidis* (strain ATCC 12228), *S. pneumoniae* (strain ATCC 49619), *S. pyogenes* (strain IHMA 1117021), *H. influenzae* (strain ATCC 49247), *M. abscessus* (strain ATCC 19977), *M. tuberculosis* (H37Rv strain ATCC 27294) and *M. smegmatis* (strain ATCC 700084). The results are given in Table 3A and Table 3B.

**Table 3A. In vitro antibacterial activity of selected compounds against listed species.**

| MIC (mg/mL) | *E. faecium* | *E. faecalis* | *S. epidermidis* | *S. pneumoniae* |
|---|---|---|---|---|
| Rifampicin | 0.5 | 1 | ≤ 0.002 | 0.016 |
| Rifabutin | 0.125 | 4 | 0.004 | ≤ 0.002 |
| 3B | 0.03 | 0.5 | ≤ 0.002 | ≤ 0.002 |
| 7A | 0.06 | 0.5 | ≤ 0.002 | ≤ 0.002 |
| 8 (A+B+C+D) | 0.125 | 2 | ≤ 0.002 | ≤ 0.002 |
| 12B | 0.03 | 0.125 | ≤ 0.002 | ≤ 0.002 |
| 13B | 0.125 | 0.5 | ≤ 0.002 | ≤ 0.002 |
| 16(A+B) | 0.125 | 0.5 | ≤ 0.002 | ≤ 0.002 |
| 18 | 0.125 | 2 | ≤ 0.002 | ≤ 0.002 |
| 20B | 0.5 | 1 | ≤ 0.002 | ≤ 0.002 |

**Table 3B. In vitro antibacterial activity of selected compounds against listed species.**

| MIC (mg/mL) | *S. pyogenes* | *H. influenzae* | *M. abscessus* | *M. tuberculosis* | *M*. *smegmatis* |
|---|---|---|---|---|---|
| Rifampicin | 0.03 | 0.25 | > 32 | 0.0041 | 1.12 |
| Rifabutin | 0.004 | 0.5 | 8 | 0.0035 | 0.24 |
| 3B | ≤ 0.002 | 0.125 | 8 | 0.010 | 0.65 |
| 7A | ≤ 0.002 | 0.06 | 16 | 0.010 | 0.54 |
| 8 (A+B+C+D) | 0.016 | 0.125 | 32 | 0.010 | 1.19 |
| 12B | ≤ 0.002 | 0.06 | 8 | 0.010 | 0.32 |
| 13B | ≤ 0.002 | 0.125 | 8 | 0.010 | 0.30 |
| 16(A+B) | ≤ 0.002 | 0.06 | 4 | ND | 0.33 |
| 18 | ≤ 0.002 | 0.125 | 32 | 0.010 | 1.11 |
| 20B | ≤ 0.002 | 0.25 | 8 | 0.010 | 0.29 |

The same compounds were tested against representative *A. baumannii* clinical isolates with mutations in RpoB known to confer reduced rifabutin activity (Trebosc et al. J. Antimicrob. Chemother. 2020; 75(12):3552-3562). The results are given in Table 4.

**Table 4. In vitro antibacterial activity of selected compounds against A. baumannii strains carrying RpoB mutations.**

| Compound | *A. baumannii* MIC (mg/L) in RPMI + FCS | | | |
|---|---|---|---|---|
| | WT | RpoB | RpoB | RpoB |
| | | H535Q | H535C | H535L |
| Rifampicin^{a} | 8 | > 32 | > 32 | > 32 |
| Rifabutin | ≤ 0.002 | 0.004 | 0.03 | > 32 |
| 3B | ≤ 0.002 | ≤ 0.002 | ≤ 0.002 | 0.016 |
| 7A | 0.004 | 0.016 | 0.03 | 1 |
| 8(A+B) | 0.008 | 0.06 | 0.125 | 1 |
| 12B | ≤ 0.002 | ≤ 0.002 | ≤ 0.002 | 0.03 |
| 13B | ≤ 0.002 | ≤ 0.002 | ≤ 0.002 | 0.03 |
| 16(A+B) | ≤ 0.002 | 0.008 | 0.016 | 1 |
| 18 | 0.008 | 0.03 | 0.25 | 4 |
| 20B | ≤ 0.002 | 0.004 | 0.008 | 0.5 |

| | | | | |
|---|---|---|---|---|
| ^{a} Rifampicin MIC determined in cation-adjusted Mueller Hinton broth | | | | |

The *in vitro* activity of compounds 3B, 12B and 13B was further tested against all known *A. baumannii* clinical isolates carrying an RpoB mutation (n =20) from a collection of 353 *A. baumannii* strains. The results are given in Table 5.

**Table 5: In vitro antibacterial activity (MIC mg/L) against A. baumannii isolates with various RpoB mutations.**

| Compound | RpoB mutation | | | | | | |
|---|---|---|---|---|---|---|---|
| | I581M | H535Q | H535Q | H535Q | H535Q / S521T | H535Q / S521T | |
| Rifampicin^{a} | > 32 | > 32 | > 32 | > 32 | ND | ND | |
| Rifabutin | ≤ 0.002 | 0.03 | > 32 | 16 | 32 | 16 | |
| 3B | ≤ 0.002 | ≤ 0.002 | 2 | 1 | 1 | 0.5 | |
| 12B | 0.004 | ≤ 0.002 | 4 | 1 | 0.5 | 1 | |
| 13B | ≤ 0.002 | ≤ 0.002 | 4 | 2 | 0.5 | 1 | |
| | | | | | | | |

| Compound | RpoB mutation | | | | | | |
|---|---|---|---|---|---|---|---|
| | H535C | H535C | H535C | H535C | H535L | H535L | |
| Rifampicin^{a} | ND | > 32 | > 32 | > 32 | > 32 | > 32 | |
| Rifabutin | 0.06 | 0.016 | 0.25 | > 32 | 0.06 | 0.008 | |
| 3B | ≤ 0.002 | ≤ 0.002 | 0.004 | 0.008 | 0.008 | ≤ 0.002 | |
| 12B | ≤ 0.002 | 0.002 | 0.004 | 0.016 | 0.004 | ≤ 0.002 | |
| 13B | ≤ 0.002 | 0.002 | 0.004 | 0.016 | 0.004 | ≤ 0.002 | |
| | | | | | | | |

| Compound | RpoB mutation | | | | | | |
|---|---|---|---|---|---|---|---|
| | H535L | L542F | L542F | S583L | N527D | H535L | H535L |
| Rifampicin^{a} | > 32 | 8 | 8 | > 32 | > 32 | > 32 | > 32 |
| Rifabutin | > 32 | 0.004 | 1 | 0.03 | 0.03 | > 32 | > 32 |
| 3B | 0.125 | ≤ 0.002 | 0.125 | ≤ 0.002 | ≤ 0.002 | 0.016 | 0.016 |
| 12B | 0.125 | 0.002 | 0.125 | 0.004 | ≤ 0.002 | 0.03 | 0.06 |
| 13B | 0.25 | 0.002 | 0.125 | 0.004 | ≤ 0.002 | 0.03 | 0.03 |

| | | | | | | | |
|---|---|---|---|---|---|---|---|
| ^{a} Rifampicin MIC determined in cation-adjusted Mueller Hinton broth ND: not determined | | | | | | | |

## Claims

1. A compound of Formula I or a pharmaceutically acceptable salt, tautomer, solvate, hydrate, enantiomer or diastereomer thereof: 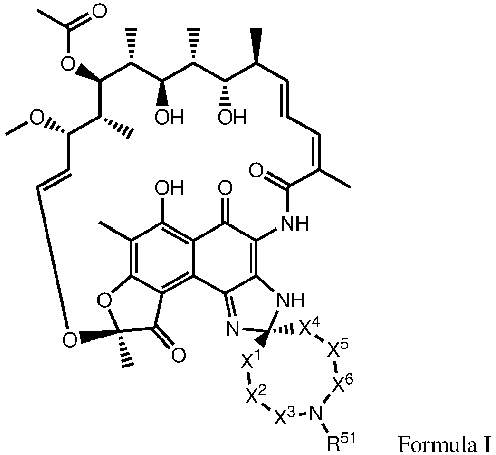 wherein:
X¹ is -CR¹R^{1A}-;
X² is independently, at each occurrence, selected from -CR²R^{2A}-, -NR^{2B}-, -O-, and a chemical bond;
X³ is independently, at each occurrence, selected from -CR³R^{3A}-, -NR^{3B}-, -O-, and a chemical bond;
X⁴ is -CR⁴R^{4A}-;
X⁵ is independently, at each occurrence, selected from -CR⁵R^{5A}-, -NR^{5B}-, -O-, and a chemical bond;
X⁶ is independently, at each occurrence, selected from -CR⁶R^{6A}-, -NR^{6B}-, -O-, and a chemical bond;
R⁵¹ is independently, at each occurrence, selected from -H, -C₁-C₆ alkyl, and -C₃-C₈ cycloalkyl;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R^{2B}, R^{3B}, R^{5B} and R^{6B} are independently, at each occurrence, selected from -H and -C₁-C₆ alkyl; or
any of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹, together with the atom to which they are attached, can independently combine with any other member of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹ to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₆ alkyl or -C₁-C₆ alkoxy;
wherein when any of R¹, R^{1A}, R², R^{2A}, R^{2B}, R³, R^{3A}, R^{3B}, R⁴, R^{4A}, R⁵, R^{5A}, R^{5B}, R⁶, R^{6A} and R^{6B} combine to form said first bridging cycloalkyl ring or said first bridging heterocycloalkyl ring, said first bridging cycloalkyl or first bridging heterocycloalkyl can independently combine with R⁵¹ to form a second bridging 5-7 membered heterocycloalkyl, wherein said second bridging heterocycloalkyl is optionally substituted by one or more -C₁-C₆ alkyl, or -C₁-C₆ alkoxy; and
provided that at least two of R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹ combine to form a bridging cycloalkyl or heterocycloalkyl; or no more than one of X², X³, X⁵, and X⁶ is a chemical bond.

2. The compound of claim 1, wherein:
X¹, X², X³, X⁴, X⁵, X⁶ and NR⁵¹ combine to form an azepane, wherein said azepane optionally further comprises a bridging C₄-C₈ cycloalkyl or bridging 5 to 8-membered heterocycloalkyl; or
X¹, X², X³, X⁴, X⁵, X⁶ and NR⁵¹ combine to form a piperidine further comprising a bridging C₄-C₈ cycloalkyl or bridging 5 to 8-membered heterocycloalkyl.

3. The compound of any of the preceding claims, wherein:
X¹ is CR¹R^{1A};
X² is CR²R^{2A};
X³ is independently, at each occurrence, selected from CR³R^{3A} and a chemical bond;
X⁴ is CR⁴R^{4A};
X⁵ is CR⁵R^{5A};
X⁶ is independently, at each occurrence, selected from CR⁶R^{6A} and a chemical bond;
R¹, R^{1A}, R², R^{2A}, R³, R^{3A}, R⁴, R^{4A}, R⁵, R^{5A}, R⁶ and R^{6A} are independently, at each occurrence, selected from -H, -C₁-C₆ alkyl, and -C₁-C₆ alkoxy;
R⁵¹ is independently, at each occurrence, selected from -H, -C₁-C₆ alkyl, and -C₃-C₈ cycloalkyl; or
any of R¹, R² and R³, together with the carbon atoms to which they are attached, can independently combine with any of R⁴, R⁵, R⁶, and R⁵¹ to form a bridging C₄-C₈ cycloalkyl ring or a bridging 5-8 membered heterocycloalkyl ring; or any of R⁴, R⁵, R⁶, together with the carbon atoms to which they are attached, can independently combine with R⁵¹ to form a bridging 5-8 membered heterocycloalkyl ring, wherein said heterocycloalkyl is optionally substituted by one or more -C₁-C₆ alkyl or -C₁-C₆ alkoxy;
wherein when any of R¹, R², R³, R⁴, R⁵, and R⁶ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted by one or more -C₁-C₆ alkyl, or -C₁-C₆ alkoxy; and
provided that at least two of R¹, R², R³, R⁴, R⁵, R⁶ and R⁵¹ combine to form a bridging cycloalkyl or heterocycloalkyl; or no more than one of X³ and X⁶ is a chemical bond.

4. The compound of any of the preceding claims, wherein:
X¹ is CR¹R^{1A}; X² is CR²R^{2A}; X³ is independently, at each occurrence, selected from CR³R^{3A} and a chemical bond; X⁴ is CR⁴R^{4A}; X⁵ is CR⁵R^{5A}; X⁶ is independently, at each occurrence, selected from CR⁶R^{6A} and a chemical bond; wherein
(i) R^{1A}, R², R^{2A}, R^{4A}, R⁵ and R^{5A} are -H; R³, R^{3A}, R⁶ and R^{6A} are absent or -H; R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(ii) R^{1A}, R¹, R^{2A}, R^{4A}, R⁴ and R^{5A} are -H; R³, R^{3A}, R⁶ and R^{6A} are absent or -H; R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iii) R^{1A}, R², R^{2A}, R⁴, R^{4A}, R⁵ and R^{5A} are -H; R³, R^{3A}, R⁶ and R^{6A} are absent or -H; R¹ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(iv) R¹, R^{1A}, R², R^{2A}, R^{4A}, R⁵, and R^{5A} are -H; R³, R^{3A}, R⁶ and R^{6A} are absent or -H; R⁴ and R⁵¹ combine to form a bridging 5-8 membered heterocycloalkyl ring, wherein said bridging heterocycloalkyl is optionally substituted by one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; wherein
when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl, wherein each bridging cycloalkyl or heterocycloalkyl is optionally substituted with one or more -C₁-C₄ alkyl or -C₁-C₄ alkoxy, preferably -C₁-C₃ alkyl or -C₁-C₃ alkoxy, more preferably -C₁-C₂ alkyl or C₁-C₂ alkoxy; or
(v) exactly one of X³ and X⁶ is a chemical bond.

5. The compound of any of the preceding claims, wherein:
X¹ is CR¹R^{1A}; X² is CR²R^{2A}; X⁴ is CR⁴R^{4A}; X⁵ is CR⁵R^{5A};
(i) X³ and X⁶ are both chemical bonds, R^{1A}, R², R^{2A}, R^{4A}, R⁵, and R^{5A} are -H, R¹ and R⁴ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(ii) X³ and X⁶ are both chemical bonds, R^{1A}, R¹, R^{2A}, R^{4A}, R⁴, and R^{5A} are -H, R² and R⁵ combine to form a first bridging C₄-C₈ cycloalkyl ring or a first bridging 5-8 membered heterocycloalkyl ring, wherein each bridging cycloalkyl or bridging heterocycloalkyl is optionally substituted by one or more -C₁-C₃ alkyl or -C₁-C₃ alkoxy, preferably -C₁-C₂ alkyl or -C₁-C₂ alkoxy; or
(iii) X³ and X⁶ are both chemical bonds, R^{1A}, R², R^{2A}, R^{4A}, R⁵, and R^{5A} are -H, one of R¹ and R⁴ combine with R⁵¹ to form a bridging 5-8 membered heterocycloalkyl ring, wherein said bridging heterocycloalkyl is optionally substituted by one or more -C₁-C₃ alkyl or -C₁-C₃ alkoxy, preferably -C₁-C₂ alkyl or -C₁-C₂ alkoxy; wherein
when R¹ and R⁴ or R² and R⁵ combine to form a first bridging cycloalkyl ring or first bridging heterocycloalkyl ring, the first bridging cycloalkyl or first bridging heterocycloalkyl can independently combine with R⁵¹ to form a second bridging 5-7 membered heterocycloalkyl wherein the second bridging heterocycloalkyl is optionally substituted by one or more -C₁-C₃ alkyl or -C₁-C₃ alkoxy, preferably -C₁-C₂ alkyl or -C₁-C₂ alkoxy; or
(iv) exactly one of X³ and X⁶ is a chemical bond.

6. The compound of any of the preceding claims, wherein
X¹ is CR¹R^{1A}; X² is CR²R^{2A}; X⁴ is CR⁴R^{4A}; X⁵ is CR⁵R^{5A};
(i) X³ and X⁶ are both chemical bonds, R^{1A}, R², R^{2A}, R^{4A}, R⁵, and R^{5A} are -H, R¹ and R⁴ combine to form a first bridging cyclobutyl, cyclopentyl, cyclohexyl, or piperidinyl ring, wherein each cyclobutyl, cyclopentyl, cyclohexyl, or piperidinyl ring is optionally substituted with one or more -C₁-C₃ alkyl, preferably one or two -C₁-C₂ alkyl; or
(ii) X³ and X⁶ are both chemical bonds, R^{1A}, R¹, R^{2A}, R^{4A}, R⁴, and R^{5A} are -H, R² and R⁵ combine to form a first bridging cyclohexyl or cycloheptyl ring, wherein each cyclohexyl or cycloheptyl is optionally substituted with one or more -C₁-C₃ alkyl, preferably one or two -C₁-C₂ alkyl; or
(iii) X³ and X⁶ are both chemical bonds, R^{1A}, R², R^{2A}, R^{4A}, R⁵, and R^{5A} are -H, one of R¹ and R⁴ combine with R⁵¹ to form a bridging pyrrolidinyl, piperidinyl, or oxazinanyl, wherein each pyrrolidinyl, piperidinyl, or oxazinanyl is optionally substituted with one or more -C₁-C₃ alkyl, preferably one or two -C₁-C₂ alkyl; wherein
when R¹ and R⁴ or R² and R⁵ combine to form a first bridging cyclohexyl or piperidinyl, the first bridging cyclohexyl or piperidinyl can independently combine with R⁵¹ to form a second bridging piperidinyl or 1,3-diazinanyl, wherein the second bridging piperidinyl or 1,3-diazinanyl is optionally substituted by one or more -C₁-C₃ alkyl, preferably one or two -C₁-C₂ alkyl; or
(iv) exactly one of X³ and X⁶ is a chemical bond.

7. The compound of any of the preceding claims, wherein R⁵¹ is selected from -H, -C₁-C₄ alkyl, and -C₅-C₆ cycloalkyl.

8. The compound of any of the preceding claims, wherein exactly one of X³ and X⁶ is a chemical bond.

9. The compound of any of the preceding claims, wherein X³ and X⁶ are both chemical bonds.

10. The compound of claim 1, wherein the compound is of Formula IA, IB, IC, ID, or IE: 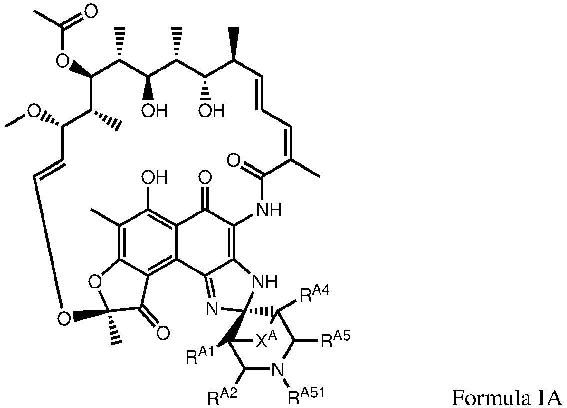 wherein:
R^{A1}, R^{A2}, R^{A4} and R^{A5} are each independently -H or -C₁-C₄ alkyl;
X^{A} is independently selected from -CH₂-, -CH₂CH₂-, -CH₂CH₂CH₂-, -CH₂OCH₂-, - CH₂NHCH₂-, or -CH₂N(C₁-C₄ alkyl)CH₂;
R^{A51} is independently -H or -C₁-C₄ alkyl; or
X^{A} and R^{A51}, together with the atoms to which they are attached, combine to form a 5 to 6-membered heterocycloalkyl; 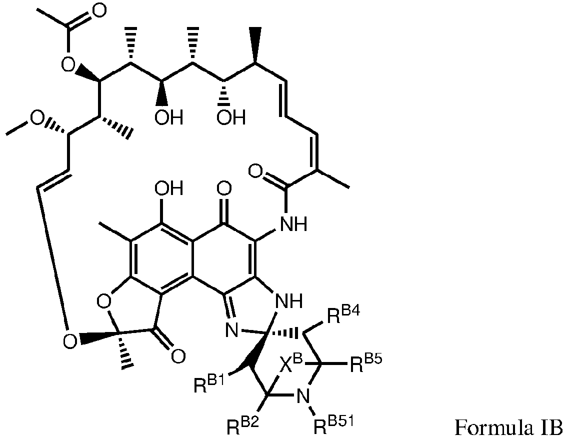 wherein:
R^{B1}, R^{B2}, R^{B4} and R^{B5} are each independently -H or -C₁-C₄ alkyl;
X^{B} is independently selected from -CH₂-, and -CH₂CH₂-;
R^{B51} is independently -H or -C₁-C₄ alkyl; 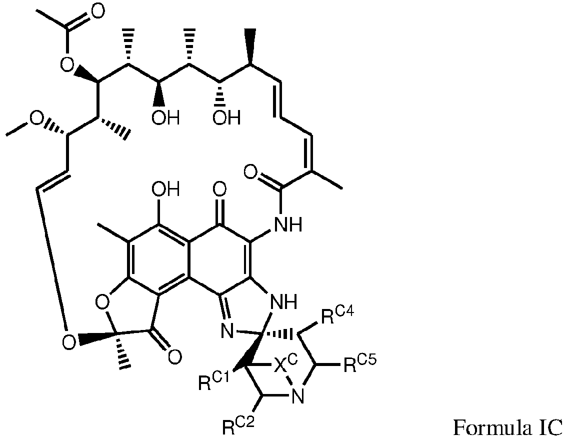
wherein:
R^{C1}, R^{C2}, R^{C4} and R^{C5} are each independently -H or -C₁-C₄ alkyl;
X^{c} is independently selected from -CH₂CH₂-, -CH₂CH₂CH₂-, -OCH₂CH₂-, and - NHCH₂CH₂-; 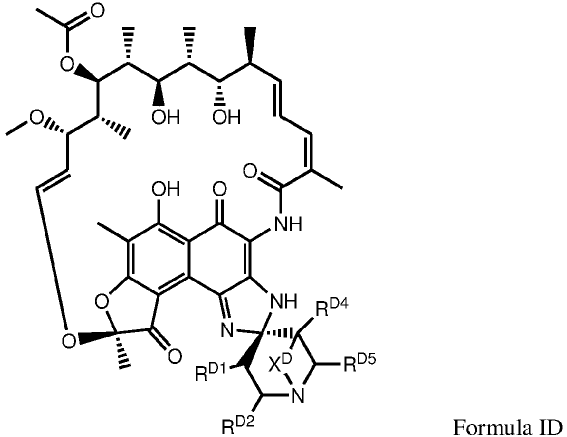 wherein:
R^{D1}, R^{D2}, R^{D4} and R^{D5} are each independently -H or -C₁-C₄ alkyl;
X^{D} is independently selected from -CH₂CH₂-, -CH₂CH₂CH₂-, -OCH₂CH₂-, and - NHCH₂CH₂-; 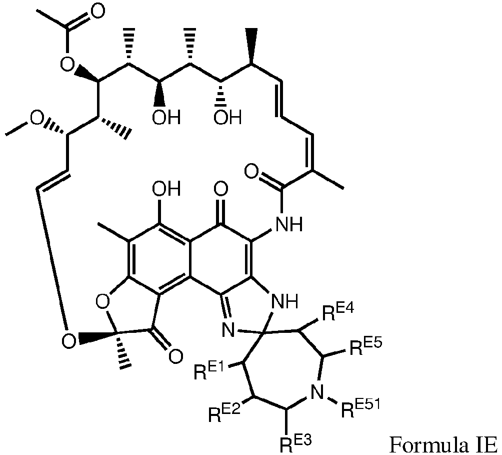 wherein:
R^{E1}, R^{E2}, and R^{E4} are each -H or C₁-C₄ alkyl; or
R^{E3} and R^{E5} are -H or combine to form a bridging cyclooctane; and;
R^{E51} is -H or -C₁-C₄ alkyl.

11. The compound of claim 1, wherein X¹, X², X³, X⁴, X⁵, X⁶ and NR⁵¹ form a structure selected from: 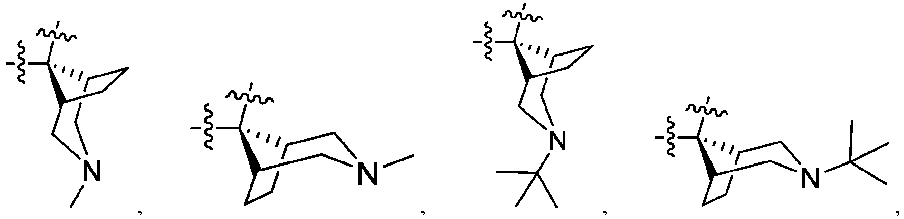 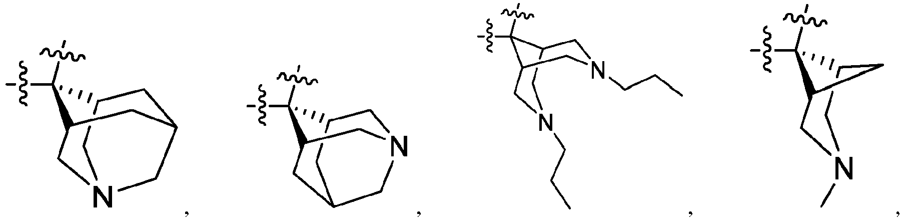 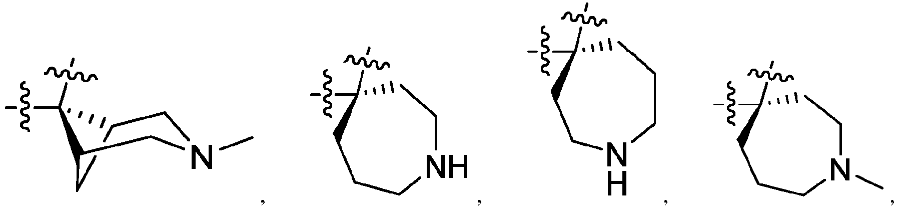 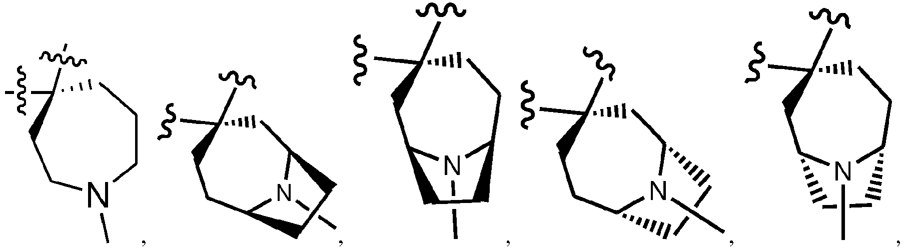 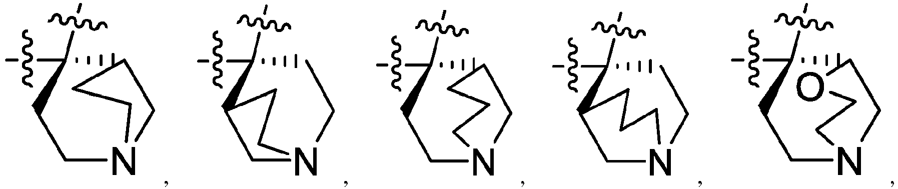 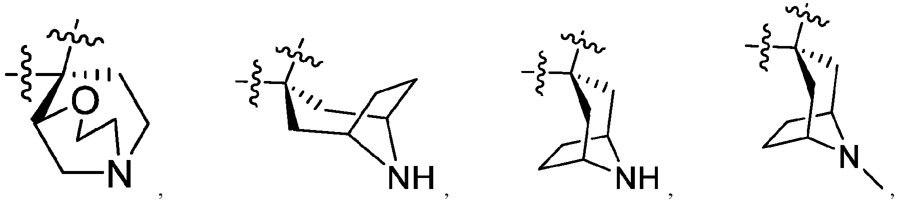 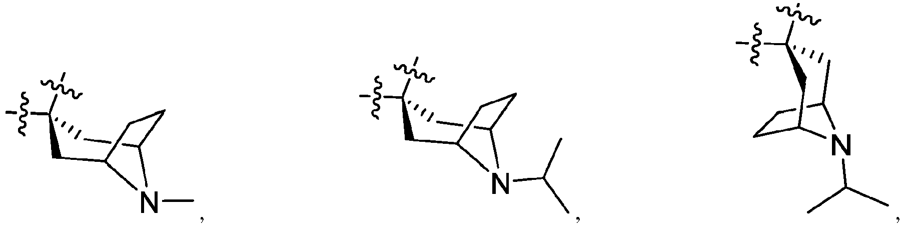 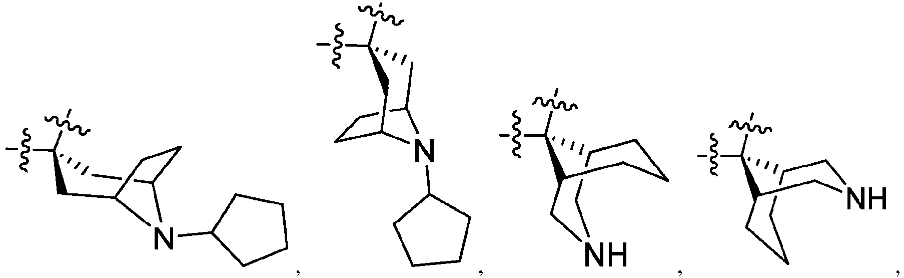 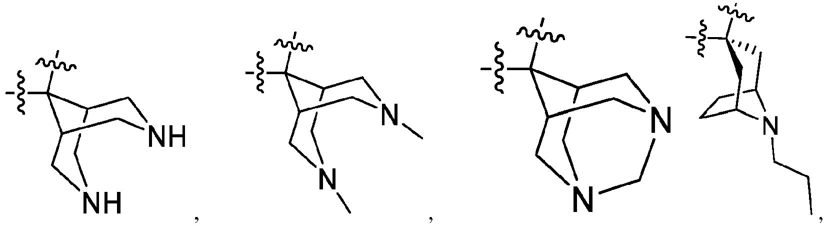 and

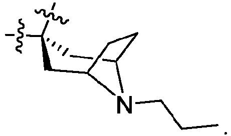

12. The compound of claim 1, selected from the group consisting of: 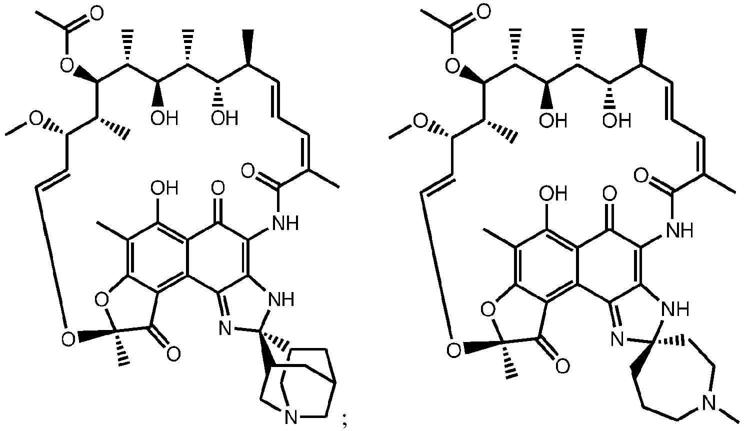 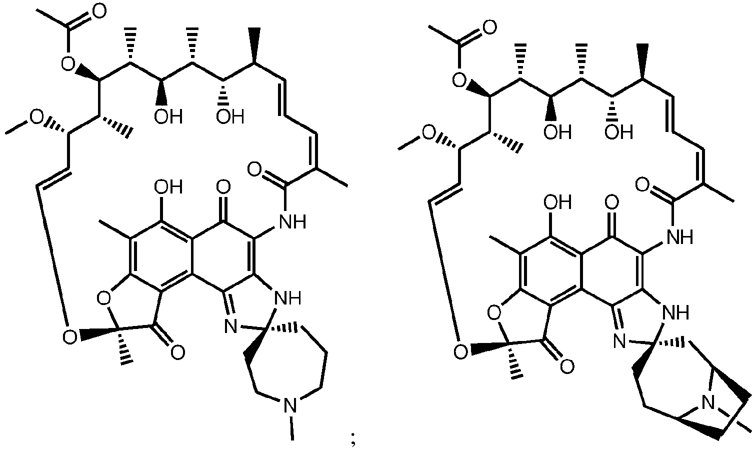 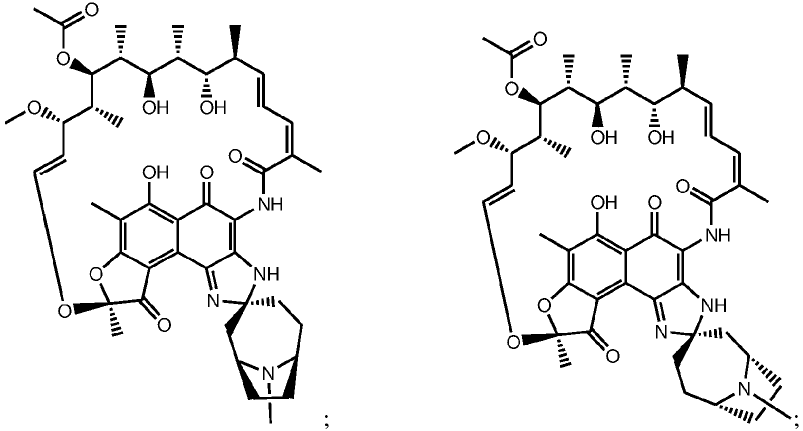 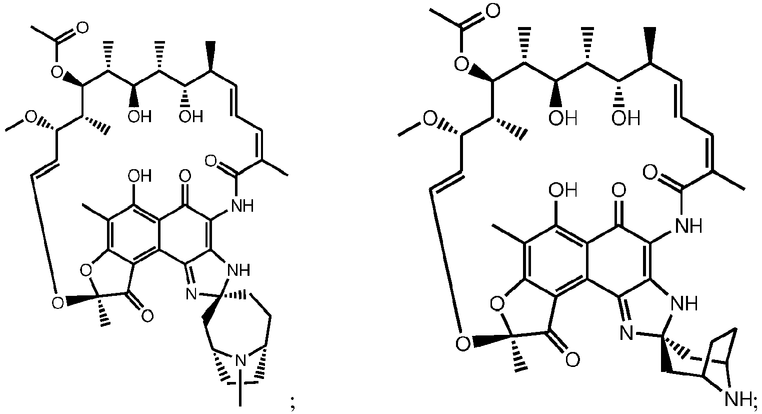 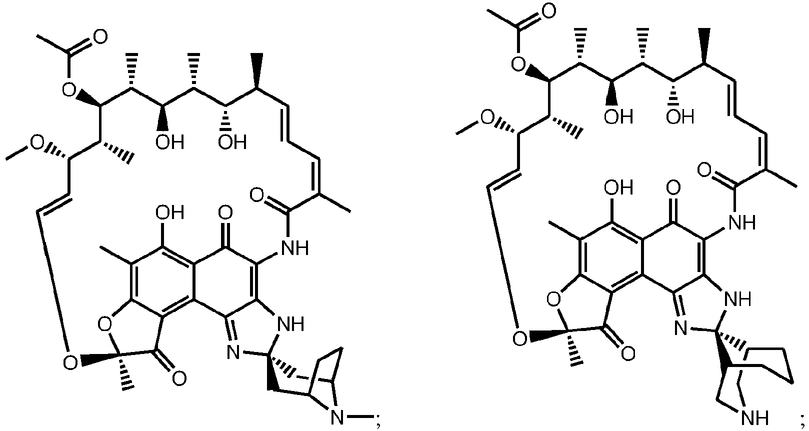 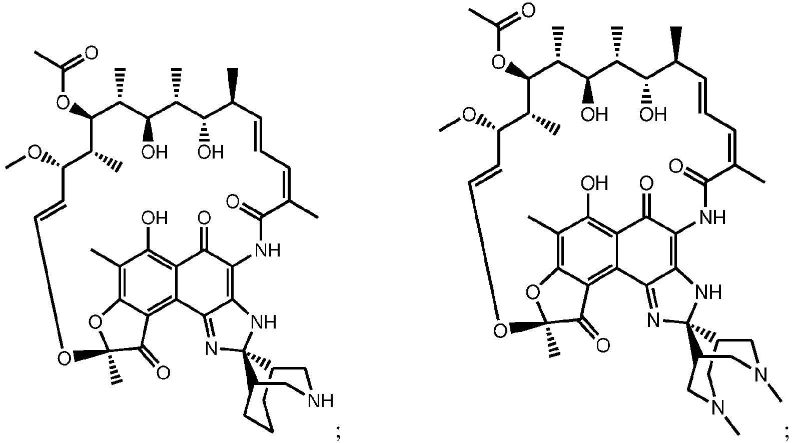 and 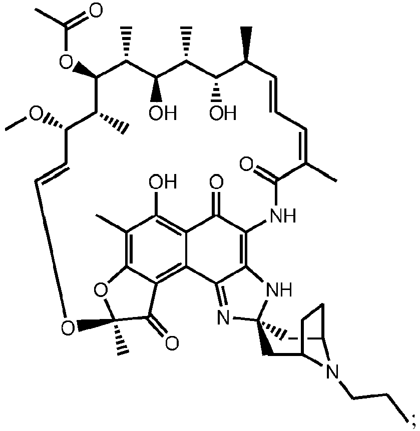 wherein preferably said compound is selected from the group consisting of: 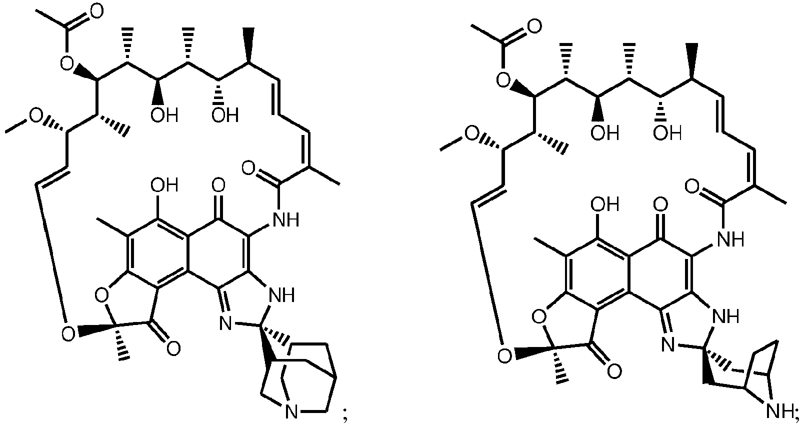 and

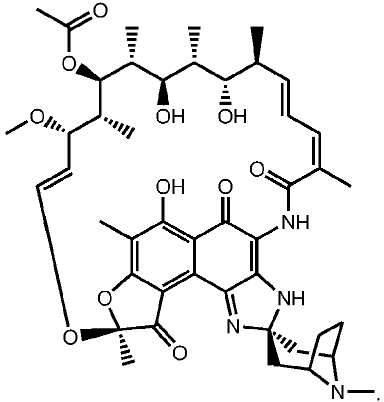

13. A pharmaceutical composition comprising at least one compound according to any of the preceding claims, or a pharmaceutically acceptable salt, tautomer, solvate or hydrate thereof, and a pharmaceutically acceptable excipient.

14. The compound according to any of claims 1-12, or a pharmaceutical composition according to claim 13, for use as a medicament, preferably for use in a method of preventing or treating a disease in a subject, wherein said disease is an infection, wherein preferably said disease is a bacterial infection.

15. The compound or pharmaceutical composition for use of claim 14, wherein said disease is a bacterial infection, wherein said bacterial infection is caused by a bacteria belonging to *Acinetobacter* spp., *Clostridium* spp., *Enterococcus* spp., *Hemophilus* spp., *Legionella* spp., *Mycobacterium* spp. (tuberculous and non-tuberculous *Mycobacteria*), *Neisseria* spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni, Chlamydia trachomatis* and/or *Toxoplasma gondii;* more preferably a bacterial infection caused by S. *aureus, A. baumannii, E. faecium, E. faecalis, S. epidermidis, S. pneumoniae, S. pyogenes, H. influenzae, M. abscessus, M. tuberculosis,* and/or *M. smegmatis;* and wherein preferably said bacterial infection is caused by one or more bacterium belonging to the genus *Acinetobacter;* further preferably by A. *baumannii;* and even more preferably by A. *baumannii* that carries one or more resistance mechanisms against rifamycins such as rifabutin and/or rifampicin.

## Patentansprüche

1. Verbindung von Formel I oder ein pharmazeutisch verträgliches Salz, Tautomer, Solvat, Hydrat, Enantiomer oder Diastereomer davon: 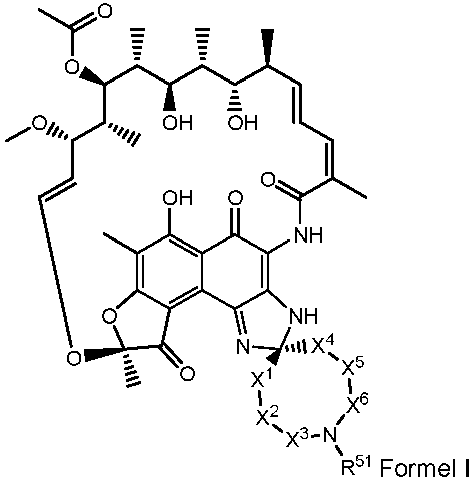 wobei:
X¹ -CR¹R^{1A}- ist;
X², bei jedem Vorkommen, aus -CR²R^{2A}-, -NR^{2B}-, -O- und einer chemischen Bindung unabhängig ausgewählt ist;
X³, bei jedem Vorkommen, aus -CR³R^{3A}-, -NR^{3B}-, -O- und einer chemischen Bindung unabhängig ausgewählt ist;
X⁴ -CR⁴R^{4A}- ist;
X⁵, bei jedem Vorkommen, aus -CR⁵R^{5A}-, -NR^{5B}-, -O- und einer chemischen Bindung unabhängig ausgewählt ist;
X⁶, bei jedem Vorkommen, aus -CR⁶R^{6A}-, -NR^{6B}-, -O- und einer chemischen Bindung unabhängig ausgewählt ist;
R⁵¹, bei jedem Vorkommen, aus -H, -C₁-C₆-Alkyl und -C₃-C₈-Cycloalkyl unabhängig ausgewählt ist;
R¹, R^{1A}, R², R^{2A}, R³, R^{3A}, R⁴, R^{4A}, R⁵, R^{5A}, R⁶ und R^{6A}, bei jedem Vorkommen, aus -H, -C₁-C₆-Alkyl und -C₁-C₆-Alkoxy unabhängig ausgewählt sind;
R^{2B}, R^{3B}, R^{5B} und R^{6B}, bei jedem Vorkommen, aus -H und -C₁-C₆-Alkyl unabhängig ausgewählt sind; oder
beliebige von R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} und R⁵¹, zusammen mit dem Atom, an das sie gebunden sind, sich mit einem beliebigen anderen Glied von R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} und R⁵¹ unabhängig kombinieren können, um einen ersten überbrückenden C₄-C₈-Cycloalkylring oder einen ersten überbrückenden 5-8-gliedrigen Heterocycloalkylring auszubilden, wobei jedes überbrückende Cycloalkyl oder überbrückende Heterocycloalkyl optional durch ein oder mehrere -C₁-C₆-Alkyl oder -C₁-C₆-Alkoxy substituiert ist;
wobei, wenn beliebige von R¹, R^{1A}, R², R^{2A}, R^{2B}, R³, R^{3A}, R^{3B}, R⁴, R^{4A}, R⁵, R^{5A}, R^{5B}, R⁶, R^{6A} und R^{6B} sich kombinieren, um den ersten überbrückenden Cycloalkylring oder den ersten überbrückenden Heterocycloalkylring auszubilden, das erste überbrückende Cycloalkyl oder erste überbrückende Heterocycloalkyl mit R⁵¹ unabhängig kombiniert werden kann, um ein zweites überbrückendes 5-7-gliedriges Heterocycloalkyl auszubilden, wobei das zweite überbrückende Heterocycloalkyl optional durch ein oder mehrere -C₁-C₆-Alkyl oder -C₁-C₆-Alkoxy substituiert ist; und
vorausgesetzt, dass mindestens zwei von R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} und R⁵¹ sich kombinieren, um ein überbrückendes Cycloalkyl oder Heterocycloalkyl auszubilden; oder nicht mehr als eines von X², X³, X⁵ und X⁶ eine chemische Bindung ist.

2. Verbindung nach Anspruch 1, wobei:
X¹, X², X³, X⁴, X⁵, X⁶ und NR⁵¹ kombinieren, um ein Azepan auszubilden, wobei das Azepan optional ferner ein überbrückendes C₄-C₈-Cycloalkyl oder überbrückendes 5- bis 8-gliedriges Heterocycloalkyl umfasst; oder
X¹, X², X³, X⁴, X⁵, X⁶ und NR⁵¹ sich kombinieren, um ein Piperidin auszubilden, ferner umfassend ein überbrückendes C₄-C₈-Cycloalkyl oder überbrückendes 5- bis 8-gliedriges Heterocycloalkyl.

3. Verbindung nach einem der vorstehenden Ansprüche, wobei:
X¹ CR¹R^{1A} ist;
X² CR²R^{2A} ist;
X³, bei jedem Vorkommen, aus CR³R^{3A} und einer chemischen Bindung unabhängig ausgewählt ist;
X⁴ CR⁴R^{4A} ist;
X⁵ CR⁵R^{5A} ist;
X⁶, bei jedem Vorkommen, aus CR⁶R^{6A} und einer chemischen Bindung unabhängig ausgewählt ist;
R¹, R^{1A}, R², R^{2A}, R³, R^{3A}, R⁴, R^{4A}, R⁵, R^{5A}, R⁶ und R^{6A}, bei jedem Vorkommen, aus -H, -C₁-C₆-Alkyl und -C₁-C₆-Alkoxy unabhängig ausgewählt sind;
R⁵¹, bei jedem Vorkommen, aus -H, -C₁-C₆-Alkyl und -C₃-C₈-Cycloalkyl unabhängig ausgewählt ist; oder
beliebige von R¹, R² und R³, zusammen mit den Kohlenstoffatomen, an die sie gebunden sind, sich mit einem beliebigen von R⁴, R⁵, R⁶ und R⁵¹ unabhängig kombinieren können, um einen überbrückenden -C₄-C₈-Cycloalkylring oder einen überbrückenden 5-8-gliedrigen Heterocycloalkylring auszubilden; oder beliebige von R⁴, R⁵, R⁶, zusammen mit den Kohlenstoffatomen, an die sie gebunden sind, mit R⁵¹ unabhängig kombinieren können, um einen überbrückenden 5-8-gliedrigen Heterocycloalkylring auszubilden, wobei das Heterocycloalkyl optional durch ein oder mehrere -C₁-C₆-Alkyl oder -C₁-C₆-Alkoxy substituiert ist;
wobei, wenn beliebige von R¹, R², R³, R⁴, R⁵ und R⁶ sich kombinieren, um einen ersten überbrückenden Cycloalkylring oder ersten überbrückenden Heterocycloalkylring auszubilden, das erste überbrückende Cycloalkyl oder erste überbrückende Heterocycloalkyl sich mit R⁵¹ unabhängig kombinieren kann, um ein zweites überbrückendes 5-7-gliedriges Heterocycloalkyl auszubilden, wobei jedes überbrückende Cycloalkyl oder Heterocycloalkyl optional durch ein oder mehrere -C₁-C₆-Alkyl oder -C₁-C₆-Alkoxy substituiert ist; und
vorausgesetzt, dass mindestens zwei von R¹, R², R³, R⁴, R⁵, R⁶ und R⁵¹ sich kombinieren, um ein überbrückendes Cycloalkyl oder Heterocycloalkyl auszubilden; oder nicht mehr als eines von X³ und X⁶ eine chemische Bindung ist.

4. Verbindung nach einem der vorstehenden Ansprüche, wobei:
X¹ CR¹R^{1A} ist; X² CR²R^{2A} ist; X³, bei jedem Vorkommen, aus CR³R^{3A} und einer chemischen Bindung unabhängig ausgewählt ist; X⁴ CR⁴R^{4A} ist; X⁵ CR⁵R^{5A} ist; X⁶, bei jedem Vorkommen, aus CR⁶R^{6A} und einer chemischen Bindung unabhängig ausgewählt ist; wobei
(i) R^{1A}, R², R^{2A}, R^{4A}, R⁵ und R^{5A} -H sind; R³, R^{3A}, R⁶ und R^{6A} fehlen oder -H sind; R¹ und R⁴ sich kombinieren, um einen ersten überbrückenden C₄-C₈-Cycloalkylring oder einen ersten überbrückenden 5-8-gliedrigen Heterocycloalkylring auszubilden, wobei jedes überbrückende Cycloalkyl oder überbrückende Heterocycloalkyl optional durch ein oder mehrere -C₁-C₄-Alkyl oder -C₁-C₄-Alkoxy, vorzugsweise -C₁-C₃-Alkyl oder -C₁-C₃-Alkoxy, mehr bevorzugt -C₁-C₂-Alkyl oder C₁-C₂-Alkoxy substituiert ist; oder
(ii) R^{1A}, R¹, R^{2A}, R^{4A}, R⁴ und R^{5A} -H sind; R³, R^{3A}, R⁶ und R^{6A} fehlen oder -H sind; R² und R⁵ sich kombinieren, um einen ersten überbrückenden C₄-C₈-Cycloalkylring oder einen ersten überbrückenden 5-8-gliedrigen Heterocycloalkylring auszubilden, wobei jedes überbrückende Cycloalkyl oder überbrückende Heterocycloalkyl optional durch ein oder mehrere -C₁-C₄-Alkyl oder -C₁-C₄-Alkoxy, vorzugsweise -C₁-C₃-Alkyl oder -C₁-C₃-Alkoxy, mehr bevorzugt -C₁-C₂-Alkyl oder C₁-C₂-Alkoxy substituiert ist; oder
(iii) R^{1A}, R², R^{2A}, R⁴, R^{4A}, R⁵ und R^{5A} -H sind; R³, R^{3A}, R⁶ und R^{6A} fehlen oder -H sind; R¹ und R⁵¹ sich kombinieren, um einen überbrückenden 5-8-gliedrigen Heterocycloalkylring auszubilden, wobei das überbrückende Heterocycloalkyl optional durch ein oder mehrere -C₁-C₄-Alkyl oder -C₁-C₄-Alkoxy, vorzugsweise -C₁-C₃-Alkyl oder -C₁-C₃-Alkoxy, mehr bevorzugt -C₁-C₂-Alkyl oder C₁-C₂-Alkoxy substituiert ist; oder
(iv) R¹, R^{1A}, R², R^{2A}, R^{4A}, R⁵ und R^{5A} -H sind; R³, R^{3A}, R⁶ und R^{6A} fehlen oder -H sind; R⁴ und R⁵¹ sich kombinieren, um einen überbrückenden 5-8-gliedrigen Heterocycloalkylring auszubilden, wobei das überbrückende Heterocycloalkyl optional durch ein oder mehrere -C₁-C₄-Alkyl oder -C₁-C₄-Alkoxy, vorzugsweise -C₁-C₃-Alkyl oder -C₁-C₃-Alkoxy, mehr bevorzugt -C₁-C₂-Alkyl oder C₁-C₂-Alkoxy substituiert ist; wobei
wenn R¹ und R⁴ oder R² und R⁵ sich kombinieren, um einen ersten überbrückenden Cycloalkylring oder ersten überbrückenden Heterocycloalkylring auszubilden, das erste überbrückende Cycloalkyl oder erste überbrückende Heterocycloalkyl sich mit R⁵¹ unabhängig kombinieren kann, um ein zweites überbrückendes 5-7-gliedriges Heterocycloalkyl auszubilden, wobei jedes überbrückende Cycloalkyl oder Heterocycloalkyl optional mit einem oder mehreren -C₁-C₄-Alkyl oder -C₁-C₄-Alkoxy, vorzugsweise -C₁-C₃-Alkyl oder -C₁-C₃-Alkoxy, mehr bevorzugt -C₁-C₂-Alkyl oder C₁-C₂-Alkoxy substituiert ist; oder
(v) genau eines von X³ und X⁶ eine chemische Bindung ist.

5. Verbindung nach einem der vorstehenden Ansprüche, wobei:
X¹ CR¹R^{1A} ist; X² CR²R^{2A} ist; X⁴ CR⁴R^{4A} ist; X⁵ CR⁵R^{5A} ist;
(i) X³ und X⁶ beide chemische Bindungen sind, R^{1A}, R², R^{2A}, R^{4A}, R⁵ und R^{5A} -H sind, R¹ und R⁴ sich kombinieren, um einen ersten überbrückenden C₄-C₈-Cycloalkylring oder einen ersten überbrückenden 5-8-gliedrigen Heterocycloalkylring auszubilden, wobei jedes überbrückende Cycloalkyl oder überbrückende Heterocycloalkyl optional durch ein oder mehrere -C₁-C₃-Alkyl oder -C₁-C₃-Alkoxy, vorzugsweise -C₁-C₂-Alkyl oder -C₁-C₂-Alkoxy substituiert ist; oder
(ii) X³ und X⁶ beide chemische Bindungen sind, R^{1A}, R¹, R^{2A}, R^{4A}, R⁴ und R^{5A} -H sind, R² und R⁵ sich kombinieren, um einen ersten überbrückenden C₄-C₈-Cycloalkylring oder einen ersten überbrückenden 5-8-gliedrigen Heterocycloalkylring auszubilden, wobei jedes überbrückende Cycloalkyl oder überbrückende Heterocycloalkyl optional durch ein oder mehrere -C₁-C₃-Alkyl oder -C₁-C₃-Alkoxy, vorzugsweise -C₁-C₂-Alkyl oder -C₁-C₂-Alkoxy substituiert ist; oder
(iii) X³ und X⁶ beide chemische Bindungen sind, R^{1A}, R², R^{2A}, R^{4A}, R⁵ und R^{5A} -H sind, eines von R¹ und R⁴ sich mit R⁵¹ kombinieren, um einen überbrückenden 5-8-gliedrigen Heterocycloalkylring auszubilden, wobei das überbrückende Heterocycloalkyl optional durch ein oder mehrere -C₁-C₃-Alkyl oder -C₁-C₃-Alkoxy, vorzugsweise -C₁-C₂-Alkyl oder -C₁-C₂-Alkoxy substituiert ist; wobei
wenn R¹ und R⁴ oder R² und R⁵ sich kombinieren, um einen ersten überbrückenden Cycloalkylring oder ersten überbrückenden Heterocycloalkylring auszubilden, das erste überbrückende Cycloalkyl oder erste überbrückende Heterocycloalkyl sich mit R⁵¹ unabhängig kombinieren kann, um ein zweites überbrückendes 5-7-gliedriges Heterocycloalkyl auszubilden, wobei das zweite überbrückende Heterocycloalkyl optional durch ein oder mehrere -C₁-C₃-Alkyl oder -C₁-C₃-Alkoxy, vorzugsweise -C₁-C₂-Alkyl oder -C₁-C₂-Alkoxy substituiert ist; oder
(iv) genau eines von X³ und X⁶ eine chemische Bindung ist.

6. Verbindung nach einem der vorstehenden Ansprüche, wobei
X¹ CR¹R^{1A} ist; X² CR²R^{2A} ist; X⁴ CR⁴R^{4A} ist; X⁵ CR⁵R^{5A} ist;
(i) X³ und X⁶ beide chemische Bindungen sind, R^{1A}, R², R^{2A}, R^{4A}, R⁵ und R^{5A} -H sind, R¹ und R⁴ sich kombinieren, um einen ersten überbrückenden Cyclobutyl-, Cyclopentyl-, Cyclohexyl- oder Piperidinylring auszubilden, wobei jeder Cyclobutyl-, Cyclopentyl-, Cyclohexyl- oder Piperidinylring optional mit einem oder mehreren -C₁-C₃-Alkyl, vorzugsweise ein oder zwei -C₁-C₂-Alkyl substituiert ist; oder
(ii) X³ und X⁶ beide chemische Bindungen sind, R^{1A}, R¹, R^{2A}, R^{4A}, R⁴ und R^{5A} -H sind, R² und R⁵ sich kombinieren, um einen ersten überbrückenden Cyclohexyl- oder Cycloheptylring auszubilden, wobei jedes Cyclohexyl oder Cycloheptyl optional mit einem oder mehreren -C₁-C₃-Alkyl, vorzugsweise ein oder zwei -C₁-C₂-Alkyl substituiert ist; oder
(iii) X³ und X⁶ beide chemische Bindungen sind, R^{1A}, R², R^{2A}, R^{4A}, R⁵ und R^{5A} -H sind, eines von R¹ und R⁴ sich mit R⁵¹ kombinieren, um ein überbrückendes Pyrrolidinyl, Piperidinyl oder Oxazinanyl auszubilden, wobei jedes Pyrrolidinyl, Piperidinyl oder Oxazinanyl optional mit einem oder mehreren -C₁-C₃-Alkyl, vorzugsweise ein oder zwei -C₁-C₂-Alkyl substituiert ist; wobei
wenn R¹ und R⁴ oder R² und R⁵ sich kombinieren, um ein erstes überbrückendes Cyclohexyl oder Piperidinyl auszubilden, das erste überbrückende Cyclohexyl oder Piperidinyl mit R⁵¹ sich unabhängig kombineren kann, um ein zweites überbrückendes Piperidinyl oder 1,3-Diazinanyl auszubilden, wobei das zweite überbrückende Piperidinyl oder 1,3-Diazinanyl optional durch ein oder mehrere -C₁-C₃-Alkyl, vorzugsweise ein oder zwei -C₁-C₂-Alkyl substituiert ist; oder
(iv) genau eines von X³ und X⁶ eine chemische Bindung ist.

7. Verbindung nach einem der vorstehenden Ansprüche, wobei R⁵¹ aus -H, -C₁-C₄-Alkyl und -C₅-C₆-Cycloalkyl ausgewählt ist.

8. Verbindung nach einem der vorstehenden Ansprüche, wobei genau eines von X³ und X⁶ eine chemische Bindung ist.

9. Verbindung nach einem der vorstehenden Ansprüche, wobei X³ und X⁶ beides chemische Bindungen sind.

10. Verbindung nach Anspruch 1, wobei die Verbindung von Formel IA, IB, IC, ID oder IE stammt: 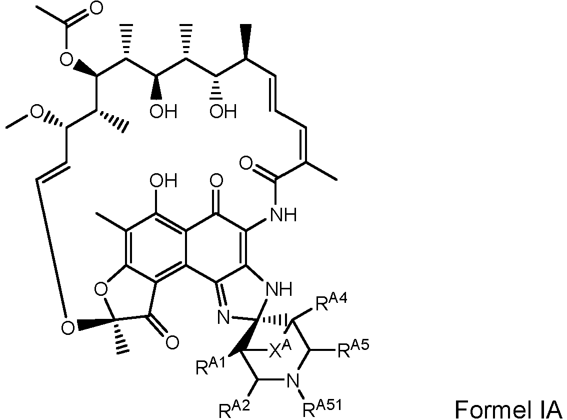 wobei:
R^{A1}, R^{A2}, R^{A4} und R^{A5} jeweils unabhängig -H oder -C₁-C₄-Alkyl sind;
X^{A} aus -CH₂-, -CH₂CH₂-, -CH₂CH₂CH₂-, -CH₂OCH₂-, -CH₂NHCH₂- oder -CH₂N(C₁-C₄-Alkyl)CH₂ unabhängig ausgewählt ist;
R^{A51} unabhängig H oder C₁-C₄-Alkyl ist; oder
X^{A} und R^{A51} zusammen mit den Atomen, an die sie gebunden sind, ein 5-bis 6-gliedriges Heterocycloalkyl ausbilden; 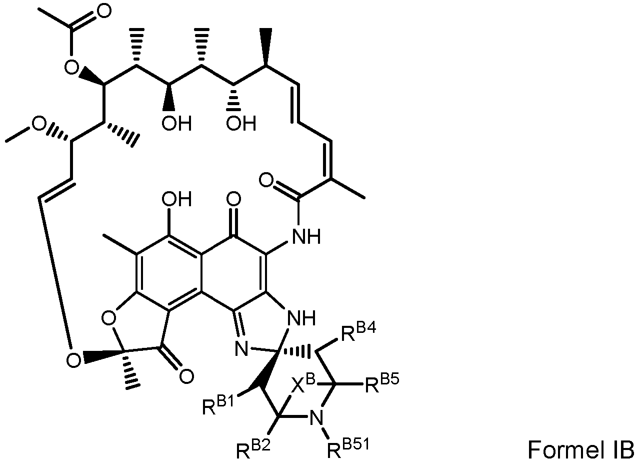 wobei:
R^{B1}, R^{B2}, R^{B4} und R^{B5} jeweils unabhängig -H oder -C₁-C ₄-Alkyl sind;
X^{B} aus -CH₂- und -CH₂CH₂- unabhängig ausgewählt ist;
R^{B51} unabhängig H oder C₁-C₄-Alkyl ist; 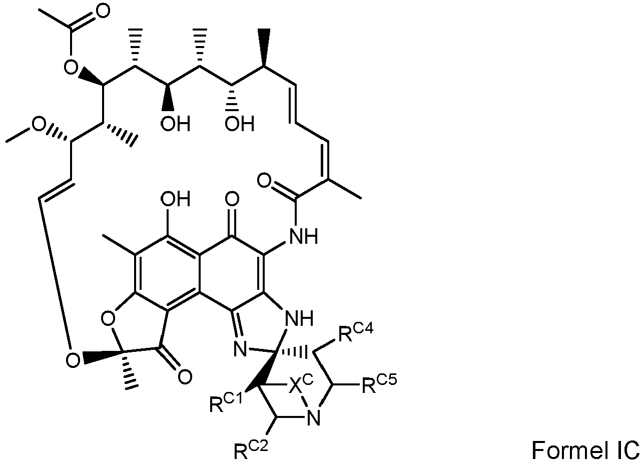 wobei:
R^{C1}, R^{C2}, R^{C4} und R^{C5} jeweils unabhängig -H oder -C₁-C₄-Alkyl sind;
X^{C} aus -CH₂CH₂-, -CH₂CH₂CH₂-, -OCH₂CH₂- und -NHCH₂CH₂-unabhängig ausgewählt ist; 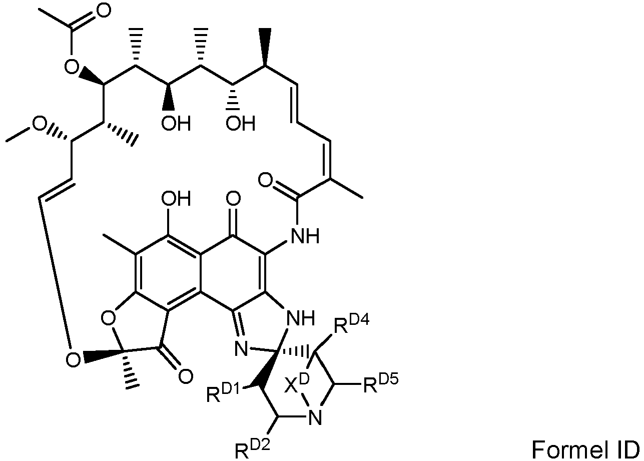 wobei:
R^{D1}, R^{D2}, R^{D4} und R^{D5} jeweils unabhängig -H oder -C₁-C₄-Alkyl sind;
X^{D} aus -CH₂CH₂-, -CH₂CH₂CH₂-, -OCH₂CH₂- und -NHCH₂CH₂-unabhängig ausgewählt ist; 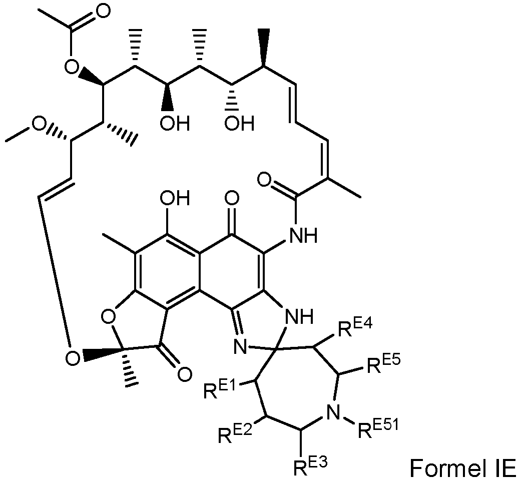 wobei:
R^{E1}, R^{E2} und R^{E4} jeweils -H oder C₁-C₄-Alkyl sind; oder
R^{E3} und R^{E5} -H sind oder sich kombinieren, um ein überbrückendes Cyclooctan auszubilden; und;
R^{E51} H oder C₁-C₄-Alkyl ist.

11. Verbindung nach Anspruch 1, wobei X¹, X², X³, X⁴, X⁵, X⁶ und NR⁵¹ eine Struktur ausbilden, die ausgewählt ist aus:

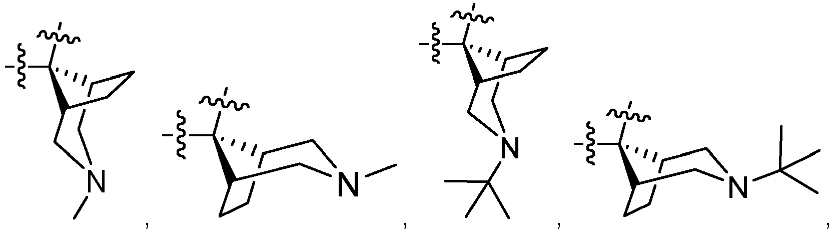

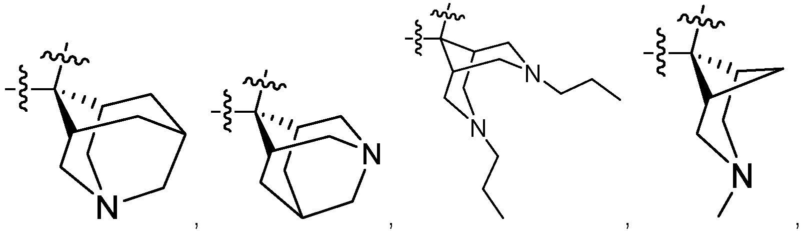

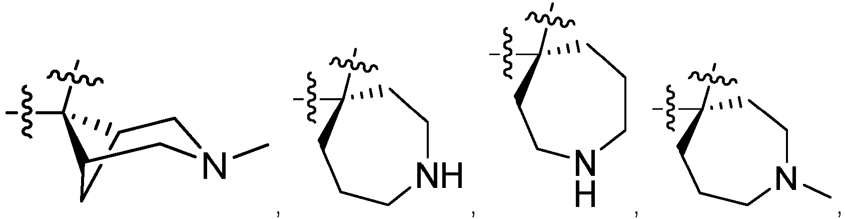

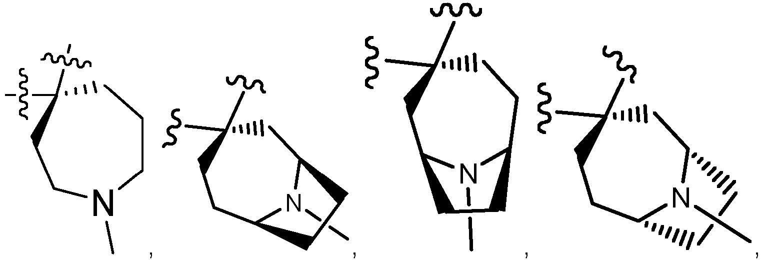

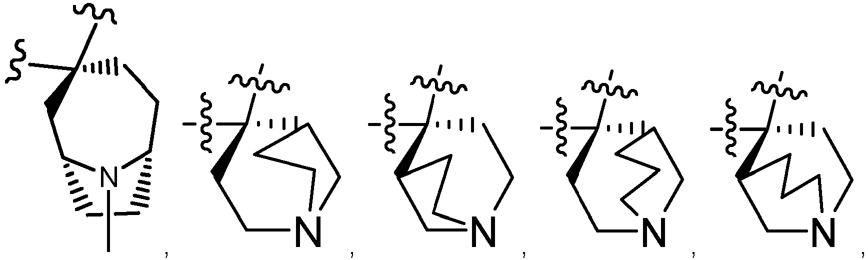

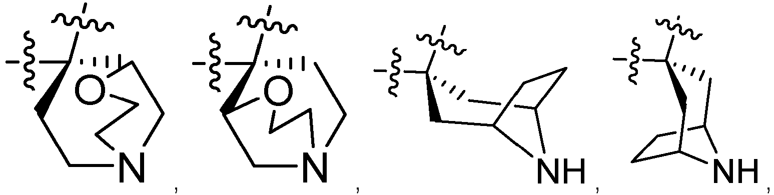

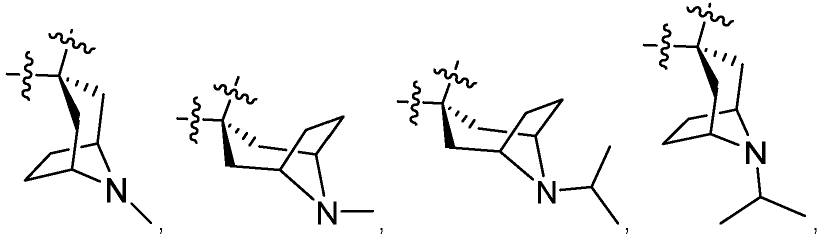

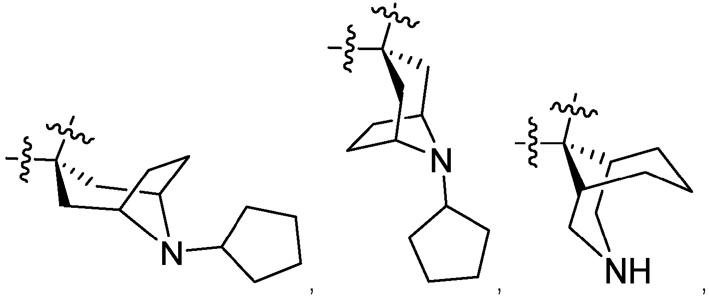

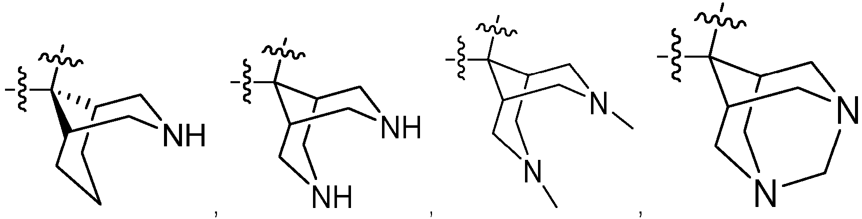

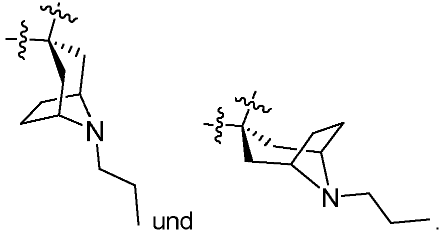

12. Verbindung nach Anspruch 1, die aus der Gruppe ausgewählt ist, bestehend aus: 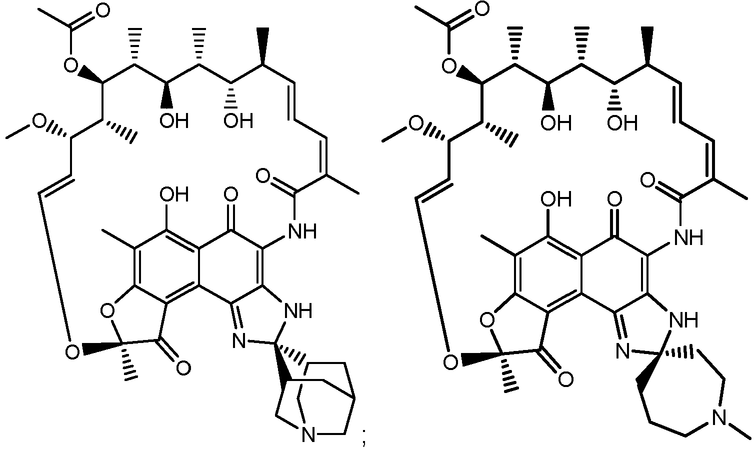 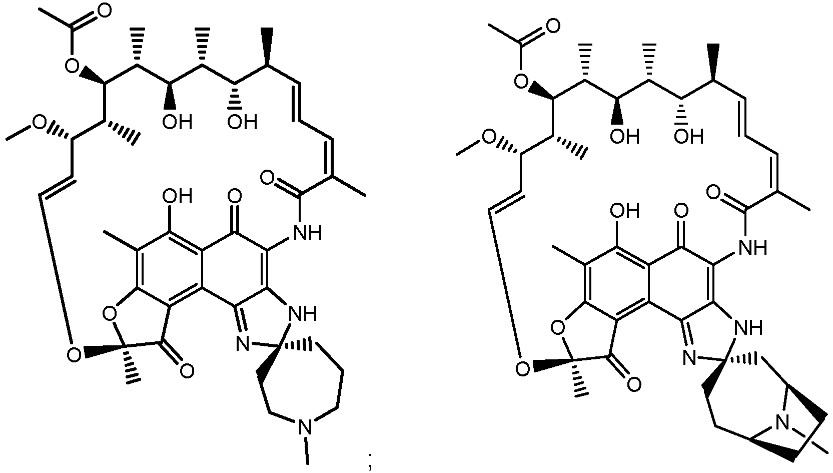 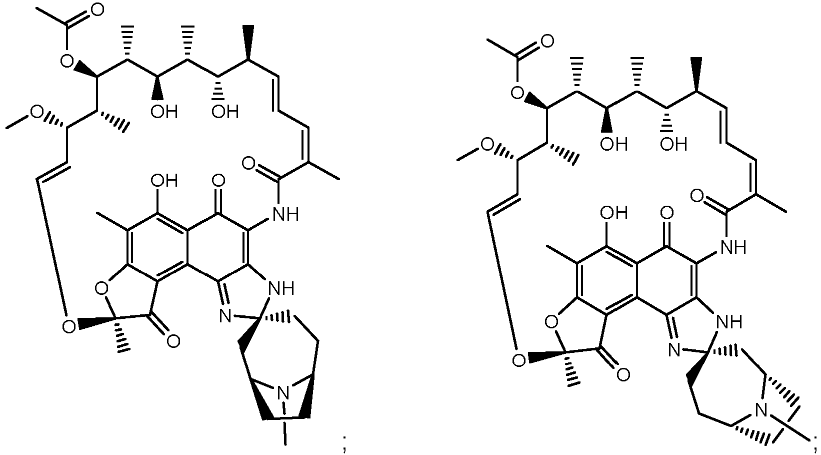 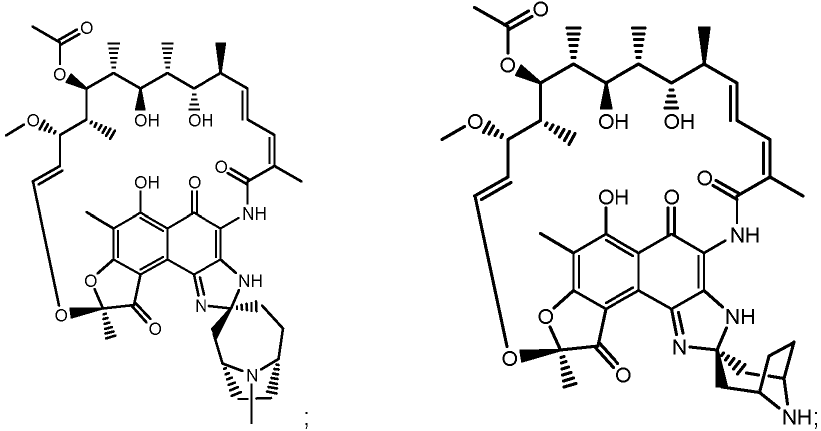 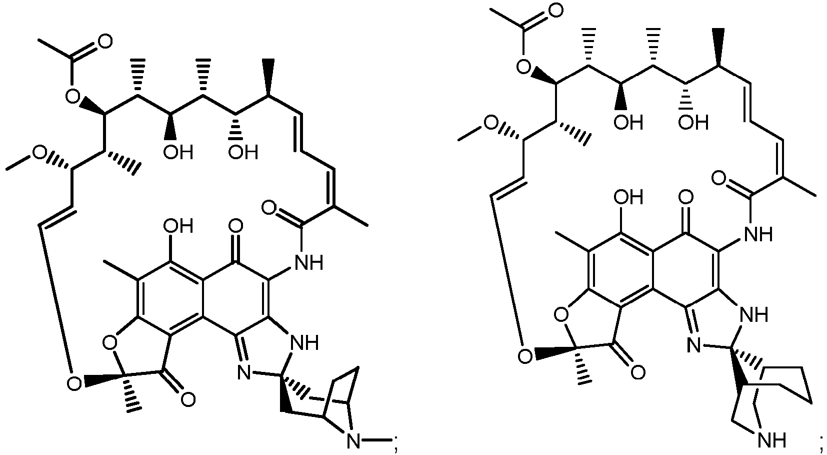 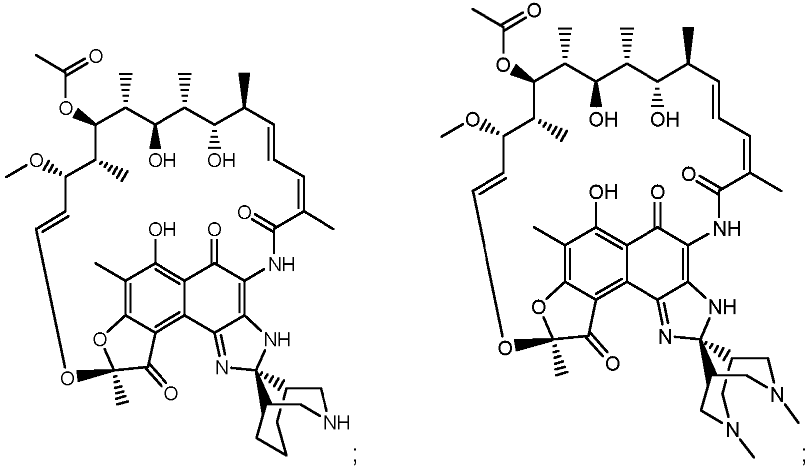 und 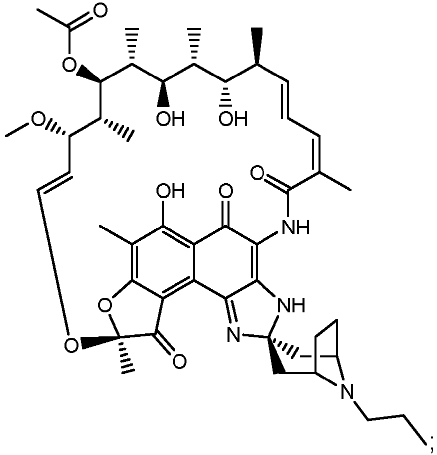 wobei vorzugsweise die Verbindung aus der Gruppe ausgewählt, bestehend aus: 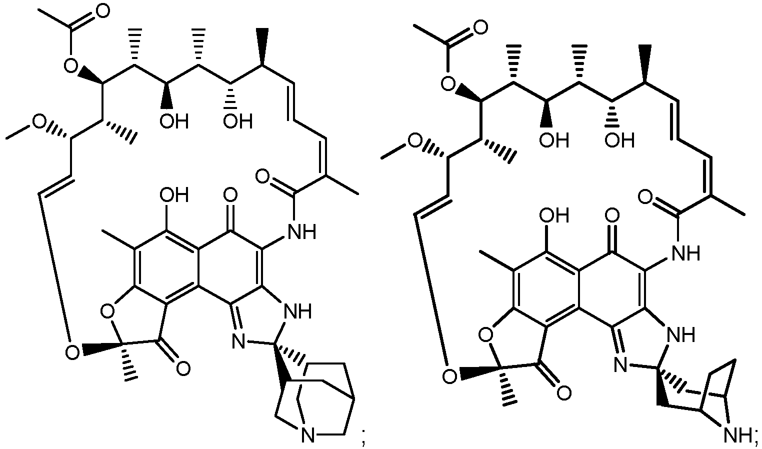 und

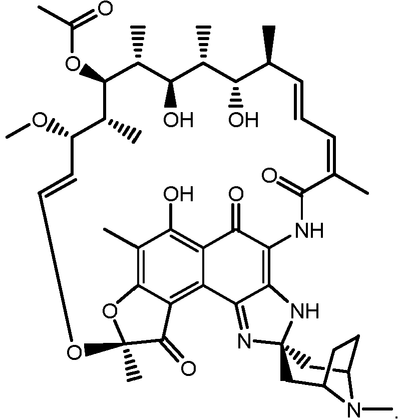

13. Pharmazeutische Zusammensetzung, umfassend mindestens eine Verbindung nach einem der vorstehenden Ansprüche oder ein pharmazeutisch verträgliches Salz, Tautomer, Solvat oder Hydrat davon und einen pharmazeutisch verträglichen Arzneustoffträger.

14. Verbindung nach einem der Ansprüche 1 bis 12 oder pharmazeutische Zusammensetzung nach Anspruch 13 zur Verwendung als ein Arzneimittel, vorzugsweise zur Verwendung in einem Verfahren zum Vorbeugen oder Behandeln einer Krankheit bei einem Subjekt, wobei die Krankheit eine Infektion ist, wobei vorzugsweise die Krankheit eine bakterielle Infektion ist.

15. Verbindung oder pharmazeutische Zusammensetzung zur Verwendung nach Anspruch 14, wobei die Krankheit eine bakterielle Infektion ist, wobei die bakterielle Infektion durch ein Bakterium verursacht wird, das zu *Acinetobacter* spp., *Clostridium* spp., *Enterokokkus* spp., *Hämophilus* spp., *Legionellen* spp., *Mykobakterium* spp. (tuberkulöse und nichttuberkulöse *Mykobakterien*), *Neisseria* spp., *Staphylokokkus* spp., *Streptokokkus* spp., *Listeria monocytogenes, Moraxella catarrhalis, Bazillus* spp., *Bacteroides* spp., *Gardnerella vaginalis, Lactobacillus* spp., *Mobiluncus* spp., *Helicobacter pylori, Campylobacter jejuni, Chlamydia trachomatis* und/oder *Toxoplasma gondii* gehört; mehr bevorzugt wobei eine bakterielle Infektion durch *S. aureus, A. baumannii, E. faecium, E. faecalis, S. epidermidis, S. pneumoniae, S. pyogenes, H. influenzae, M. abscessus, M. tuberculosis* und/oder *M. smegmatis* verursacht wird; und wobei vorzugsweise die bakterielle Infektion durch ein oder mehrere Bakterien verursacht wird, die zu der Gattung *Acinetobacter* gehören; ferner vorzugsweise durch *A. baumannii*; und noch mehr bevorzugt durch *A. baumannii,* das einen oder mehrere Resistenzmechanismen gegen Rifamycine wie Rifabutin und/oder Rifampicin trägt.

## Revendications

1. Composé de formule I ou sel, tautomère, solvate, hydrate, énantiomère ou diastéréomère pharmaceutiquement acceptable de celui-ci : 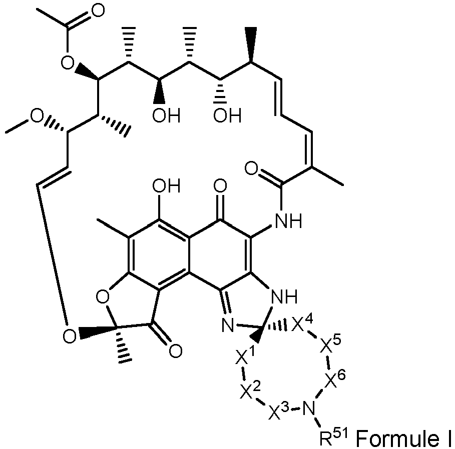 dans lequel :
X¹ est -CR¹R^{1A}- ;
X² est indépendamment, à chaque occurrence, choisi parmi -CR²R^{2A}-, - NR^{2B}-, -O-, et une liaison chimique ;
X³ est indépendamment, à chaque occurrence, choisi parmi -CR³R^{3A}-, - NR^{3B}-, -O-, et une liaison chimique ;
X⁴ est -CR⁴R^{4A}- ;
X⁵ est indépendamment, à chaque occurrence, choisi parmi -CR⁵R^{5A}-, - NR^{5B}-, -O-, et une liaison chimique ;
X⁶ est indépendamment, à chaque occurrence, choisi parmi -CR⁶R^{6A}-, - NR^{6B}-, -O-, et une liaison chimique ;
R⁵¹ est indépendamment, à chaque occurrence, choisi parmi -H, l'alkyle en -C₁ à C₆, et le cycloalkyle en -C₃ à C₈ ;
R¹, R^{1A}, R², R^{2A}, R³, R^{3A}, R⁴, R^{4A}, R⁵, R^{5A}, R⁶ et R^{6A} sont indépendamment, à chaque occurrence, choisis parmi -H-H, l'alkyle en -C₁ à C₆, et l'alkoxy en -C₁ à C₆ ;
R^{2B}, R^{3B}, R^{5B} et R^{6B} sont indépendamment, à chaque occurrence, choisis parmi -H et l'alkyle en -C₁ à C₆ ; ou
l'un quelconque de R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} and R⁵¹, ainsi que l'atome auquel ils sont attachés, peuvent se combiner indépendamment avec tout autre membre de R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} et R⁵¹ pour former un premier cycle cycloalkyle en C₄ à C₈ pontant ou un premier cycle hétérocycloalkyle à 5 à 8 membres pontant, dans lequel chaque cycloalkyle pontant ou hétérocycloalkyle pontant est éventuellement substitué par un ou plusieurs alkyles en -C₁ à C₆ ou l'alkoxy en -C₁à C₆ ;
dans lequel lorsque l'un quelconque de R¹, R^{1A}, R², R^{2A}, R^{2B}, R³, R^{3A}, R^{3B}, R⁴, R^{4A}, R⁵, R^{5A}, R^{5B}, R⁶, R^{6A} et R^{6B} se combinent pour former ledit premier cycle cycloalkyle pontant ou ledit premier cycle hétérocycloalkyle pontant, ledit premier cycloalkyle pontant ou premier hétérocycloalkyle pontant peut indépendamment se combiner avec R⁵¹ pour former un second hétérocycloalkyle pontant à 5 à 7 membres, dans lequel ledit second hétérocycloalkyle pontant est éventuellement substitué par un ou plusieurs alkyles en -C₁ à C₆ alkyl, ou l'alcoxy en -C₁ à C₆ ; et
à condition qu'au moins deux de R¹, R^{1A}, R², R^{2A}, R^{2B}, R³, R^{3A}, R^{3B}, R⁴, R^{4A}, R⁵, R^{5A}, R^{5B}, R⁶, R^{6A}, R^{6B} et R⁵¹ se combinent pour former un cycloalkyle ou un hétérocycloalkyle pontant ; ou pas plus d'un de X², X³, X⁵, et X⁶ n'est une liaison chimique.

2. Composé selon la revendication 1, dans lequel :
X¹, X², X³, X⁴, X⁵, X⁶ et NR⁵¹ se combinent pour former un azépane, dans lequel ledit azépane comprend éventuellement un cycloalkyle pontant en C₄ à C₈ ou un hétérocycloalkyle pontant à 5 à 8 membres ; ou
X¹, X², X³, X⁴, X⁵, X⁶ et NR⁵¹ se combinent pour former une pipéridine comprenant en outre un cycloalkyle pontant en C₄à C₈ ou un hétérocycloalkyle pontant à 5 à 8 membres.

3. Composé selon l'une quelconque des revendications précédentes, dans lequel :
X¹ est CR¹R^{1A} ;
X² est CR²R^{2A} ;
X³ est indépendamment, à chaque occurrence, choisi parmi CR³R^{3A} et une liaison chimique ;
X⁴ est CR⁴R^{4A} ;
X⁵ est CR⁵R^{5A} ;
X⁶ est indépendamment, à chaque occurrence, choisi parmi CR⁶R^{6A} et une liaison chimique ;
R¹, R^{1A}, R², R^{2A}, R³, R^{3A}, R⁴, R^{4A}, R⁵, R^{5A}, R⁶ et R^{6A} sont indépendamment, à chaque occurrence, choisis parmi -H, l'alkyle en -C₁ à C₆, et l'alkoxy en -C₁ à C₆ ;
R⁵¹ est indépendamment, à chaque occurrence, choisi parmi -H, l'alkyle en -C₁ à C₆, et le cycloalkyle en -C₃ à C₈ ; ou
l'un quelconque de R¹, R² et R³, ainsi que les atomes de carbone auxquels ils sont attachés, peuvent indépendamment se combiner avec l'un quelconque de R⁴, R⁵, R⁶, et R⁵¹ pour former un cycle cycloalkyle pontant en C₄ à C₈ ou un cycle hétérocycloalkyle pontant à 5 à 8 membres ; ou l'un quelconque de R⁴, R⁵, R⁶, ainsi que les atomes de carbone auxquels ils sont attachés, peuvent indépendamment se combiner avec R⁵¹ pour former un cycle hétérocycloalkyle pontant de 5 à 8 membres, dans lequel ledit hétérocycloalkyle est éventuellement substitué par un ou plusieurs alkyles en -C₁ à C₆ ou l'alcoxy en -C₁ à C₆ ;
dans lequel lorsque l'un quelconque de R¹, R², R³, R⁴, R⁵, et R⁶ se combinent pour former un premier cycle cycloalkyle pontant ou un premier cycle hétérocycloalkyle pontant, le premier cycloalkyle pontant ou le premier hétérocycloalkyle pontant peut indépendamment se combiner avec R⁵¹ pour former un second hétérocycloalkyle pontant à 5 à 7 membres, dans lequel chaque cycloalkyle ou hétérocycloalkyle pontant est éventuellement substitué par un ou plusieurs alkyles en -C₁à C₆, ou l'alcoxy en -C₁à C₆ ; et
à condition qu'au moins deux de R¹, R², R³, R⁴, R⁵, R⁶ et R⁵¹ se combinent pour former un cycloalkyle ou un hétérocycloalkyle pontant ; ou pas plus d'un de X³ et X⁶ n'est une liaison chimique.

4. Composé selon l'une quelconque des revendications précédentes, dans lequel :
X¹ est CR¹R^{1A} ; ; X² est CR²R^{2A} ; X³ est indépendamment, à chaque occurrence, choisi parmi CR³R^{3A} et une liaison chimique ; X⁴ est CR⁴R^{4A} ; X⁵ est CR⁵R^{5A} ; X⁶ est indépendamment, à chaque occurrence, choisi parmi CR⁶R^{6A} et une liaison chimique ; dans lequel
(i) R^{1A}, R², R^{2A}, R^{4A}, R⁵ et R^{5A} sont -H ; R³, R^{3A}, R⁶ et R^{6A} sont absents ou - H ; R¹ et R⁴ se combinent pour former un premier cycle cycloalkyle pontant en C₄à C₈ ou un premier cycle hétérocycloalkyle pontant à 5 à 8 membres, dans lequel chaque cycloalkyle pontant ou hétérocycloalkyle pontant est éventuellement substitué par un ou plusieurs alkyles en -C₁à C₄ ou l'alcoxy en -C₁à C₄, de préférence l'alkyle en -C₁à C₃ ou l'alcoxy en -C₁à C₃, plus préférablement l'alkyle en -C₁à C₂ ou l'alcoxy en C₁à C₂ ; ou
(ii) R^{1A}, R¹, R^{2A}, R^{4A}, R⁴ et R^{5A} sont-H ; R³, R^{3A}, R⁶ et R^{6A} sont absents ou -H ; R² et R⁵ se combinent pour former un premier cycle cycloalkyle pontant en C₄-C₈ ou un premier cycle hétérocycloalkyle pontant à 5 à 8 membres, dans lequel chaque cycloalkyle pontant ou hétérocycloalkyle pontant est éventuellement substitué par un ou plusieurs alkyles en -C₁à C₄ ou l'alcoxy en -C₁à C₄, de préférence l'alkyle en -C₁à C₃ ou l'alkoxy en -C₁à C₃, plus préférablement l'alkyle en -C₁à C₂ ou l'alkoxy en C₁à C₂ ; ou
(iii) R^{1A}, R², R^{2A}, R⁴, R^{4A}, R⁵ et R^{5A} sont -H ; R³, R^{3A}, R⁶ et R^{6A} sont absents ou -H ; R¹ et R⁵¹ se combinent pour former un cycle hétérocycloalkyle pontant à 5 à 8 membres, dans lequel ledit hétérocycloalkyle pontant est éventuellement substitué par un ou plusieurs alkyles en -C₁à C₄ ou l'alkoxy en -C₁à C₄, de préférence l'alkyle en -C₁à C₃ ou l'alkoxy en -C₁à C₃, plus préférablement l'alkyle en -C₁à C₂ ou l'alkoxy en C₁à C₂ ; ou
(iv) R¹, R^{1A}, R², R^{2A}, R^{4A}, R⁵, et R^{5A} sont -H ; R³, R^{3A}, R⁶ et R^{6A} sont absents ou -H ; R⁴ et R⁵¹ se combinent pour former un cycle hétérocycloalkyle pontant à 5 à 8 membres, dans lequel ledit hétérocycloalkyle pontant est éventuellement substitué par un ou plusieurs alkyles en -C₁à C₄ ou l'alkoxy en -C₁à C₄, de préférence l'alkyle en -C₁à C₃ ou l'alkoxy en -C₁à C₃, plus préférablement l'alkyle en -C₁à C₂ ou l'alkoxy en C₁à C₂ ; dans lequel
lorsque R¹ et R⁴ ou R² et R⁵ se combinent pour former un premier cycle cycloalkyle pontant ou un premier cycle hétérocycloalkyle pontant, le premier cycloalkyle pontant ou le premier hétérocycloalkyle pontant peut indépendamment se combiner avec R⁵¹ pour former un second hétérocycloalkyle pontant à 5 à 7 membres, dans lequel chaque cycloalkyle ou hétérocycloalkyle pontant est éventuellement substitué par un ou plusieurs alkyles en -C₁à C₄ ou l'alkoxy en - C₁à C₄, de préférence l'alkyle en -C₁à C₃ ou l'alkoxy en -C₁à C₃, plus préférablement l'alkyle en -C₁à C₂ ou l'alkoxy en C₁à C₂ ; ou
(v) exactement l'un de X³ et X⁶ est une liaison chimique.

5. Composé selon l'une quelconque des revendications précédentes, dans lequel :
X¹ est CR¹R^{1A} ; ; X² est CR²R^{2A} ; X⁴ est CR⁴R^{4A} ; X⁵ est CR⁵R^{5A} ;
(i) X³ et X⁶ sont tous deux des liaisons chimiques, R^{1A}, R², R^{2A}, R^{4A}, R⁵, et R^{5A} sont -H, R¹ et R⁴ se combinent pour former un premier cycle cycloalkyle pontant en C₄à C₈ ou un premier cycle hétérocycloalkyle pontant à 5 à 8 membres, dans lequel chaque cycloalkyle pontant ou hétérocycloalkyle pontant est éventuellement substitué par un ou plusieurs alkyles en -C₁à C₃ ou l'alkoxy en -C₁à C₃, de préférence l'alkyle en -C₁à C₂ ou l'alkoxy en -C₁à C₂ ; ou
(ii) X³ et X⁶ sont tous deux des liaisons chimiques, R^{1A}, R¹, R^{2A}, R^{4A}, R⁴, et R^{5A} sont -H, R² et R⁵ se combinent pour former un premier cycle cycloalkyle pontant en C₄à C₈ ou un premier cycle hétérocycloalkyle pontant à 5 à 8 membres, dans lequel chaque cycloalkyle pontant ou hétérocycloalkyle pontant est éventuellement substitué par un ou plusieurs alkyles en -C₁à C₃ ou l'alkoxy en -C₁à C₃, de préférence l'alkyle en -C₁àC₂ ou l'alkoxy en -C₁à C₂ ; ou
(iii) X³ et X⁶ sont tous deux des liaisons chimiques, R^{1A}, R², R^{2A}, R^{4A}, R⁵, et R^{5A} sont -H, un de R¹ et R⁴ se combine avec R⁵¹ pour former un cycle hétérocycloalkyle pontant à 5 à 8 membres, dans lequel ledit hétérocycloalkyle pontant est éventuellement substitué par un ou plusieurs alkyles en -C₁à C₃ ou l'alkoxy en -C₁à C₃, de préférence l'alkyle en -C₁à C₂ ou l'alkoxy en -C₁à C₂ ; dans lequel
lorsque R¹ et R⁴ ou R² et R⁵ se combinent pour former un premier cycle cycloalkyle pontant ou un premier cycle hétérocycloalkyle pontant, le premier cycloalkyle pontant ou le premier hétérocycloalkyle pontant peut indépendamment se combiner avec R⁵¹ pour former un second hétérocycloalkyle pontant à 5 à 7 membres, dans lequel le second hétérocycloalkyle pontant est éventuellement substitué par un ou plusieurs alkyles en -C₁-C₃ ou l'alkoxy en -C₁à C₃, de préférence l'alkyle en -C₁à C₂ ou l'alkoxy en -C₁à C₂ ; ou
(iv) exactement l'un de X³ et X⁶ est une liaison chimique.

6. Composé selon l'une quelconque des revendications précédentes, dans lequel
X¹ est CR¹R^{1A} ; ; X² est CR²R^{2A} ; X⁴ est CR⁴R^{4A} ; X⁵ est CR⁵R^{5A} ;
(i) X³ et X⁶ sont tous deux des liaisons chimiques, R^{1A}, R², R^{2A}, R^{4A}, R⁵, et R^{5A} sont -H, R¹ et R⁴ se combinent pour former un premier cycle cyclobutyle, cyclopentyle, cyclohexyle ou pipéridinyle pontant, dans lequel chaque cycle cyclobutyle, cyclopentyle, cyclohexyle ou pipéridinyle est éventuellement substitué par un ou plusieurs alkyles en -C₁à C₃, de préférence un ou deux alkyles en -C₁à C₂ ; ou
(ii) X³ et X⁶ sont des liaisons chimiques, R^{1A}, R¹, R^{2A}, R^{4A}, R⁴, et R^{5A} sont -H, R² et R⁵ se combinent pour former un premier anneau cyclohexyle ou cycloheptyle pontant, dans lequel chaque cyclohexyle ou cycloheptyle est éventuellement substitué par un ou plusieurs alkyles en -C₁à C₃, de préférence un ou deux alkyles en -C₁à C₂ ; ou
(iii) X³ et X⁶ sont tous deux des liaisons chimiques, R^{1A}, R², R^{2A}, R^{4A}, R⁵, et R^{5A} sont -H, un de R¹ et R⁴ se combine avec R⁵¹ pour former un pyrrolidinyle, un pipéridinyle ou un oxazinanyle pontant, dans lequel chaque pyrrolidinyle, pipéridinyle ou oxazinanyle est éventuellement substitué par un ou plusieurs alkyles en -C₁à C₃, de préférence un ou deux alkyles en -C₁à C₂ ; dans lequel
lorsque R¹ et R⁴ ou R² et R⁵ se combinent pour former un premier cyclohexyle ou pipéridinyle pontant, le premier cyclohexyle ou pipéridinyle pontant peut indépendamment se combiner avec R⁵¹ pour former un second pipéridinyle ou 1,3-diazinanyle pontant, dans lequel le second pipéridinyle ou 1,3-diazinanyle pontant est éventuellement substitué par un ou plusieurs alkyles en -C₁à C₃, de préférence un ou deux alkyles en -C₁à C₂ ; ou
(iv) exactement l'un de X³ et X⁶ est une liaison chimique.

7. Composé selon l'une quelconque des revendications précédentes, dans lequel R⁵¹ est choisi parmi -H, l'alkyle en -C₁à C₄, et le cycloalkyle en -C₅à C₆.

8. Composé selon l'une quelconque des revendications précédentes, dans lequel l'un de X³ et X⁶ est une liaison chimique.

9. Composé selon l'une quelconque des revendications précédentes, dans lequel X³ et X⁶ sont tous deux des liaisons chimiques.

10. Composé selon la revendication 1, dans lequel le composé est de formule IA, IB, IC, ID ou IE : 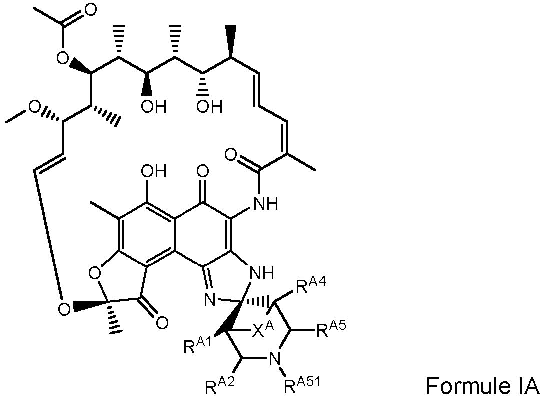 dans lequel :
R^{A1}, R^{A2}, R^{A4} et R^{A5} sont chacun indépendamment -H ou l'alkyle en -C₁à C₄ ;
X^{A} est indépendamment choisi parmi -CH₂-, -CH₂CH₂-, -CH₂CH₂CH₂-, - CH₂OCH₂-, -CH₂NHCH₂-, ou -CH₂N(l'alkyle en C₁à C₄)CH₂ ;
R^{A51} est indépendamment -H ou l'alkyle en -C₁-à ₄ ; ou
X^{A} et R^{A51}, ainsi que les atomes auxquels ils sont attachés, se combinent pour former un hétérocycloalkyle à 5 ou 6 chaînons ; 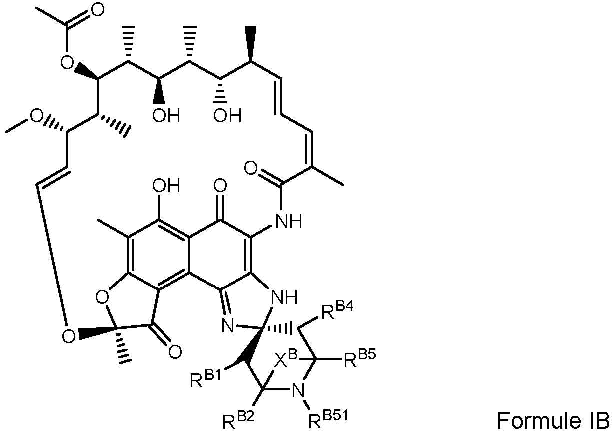 dans lequel :
R^{B1}, R^{B2}, R^{B4} et R^{B5} sont chacun indépendamment -H ou l'alkyle en -C₁à C₄ ;
X^{B} est indépendamment choisi parmi -CH₂-, et -CH₂CH₂- ;
R^{B51} est indépendamment -H ou l'alkyle en -C₁-C₄ ; 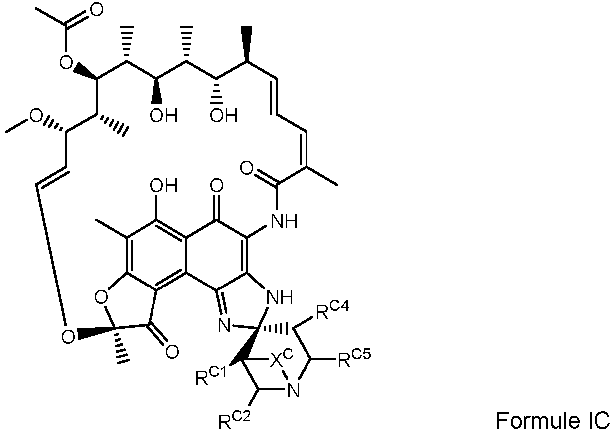 dans lequel :
R^{C1}, R^{C2}, R^{C4} et R^{C5} sont chacun indépendamment -H ou l'alkyle en -C₁à C₄ ;
X^{c} est indépendamment choisi parmi -CH₂CH₂-, -CH₂CH₂CH₂-, - OCH₂CH₂-, et -NHCH₂CH₂- ; 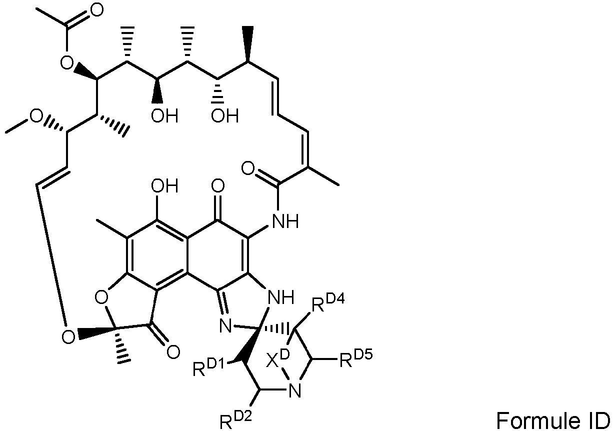 dans lequel :
R^{D1}, R^{D2}, R^{D4} et R^{D5} sont chacun indépendamment -H ou l'alkyle en -C₁à C₄ ;
X^{D} est indépendamment choisi parmi -CH₂CH₂-, -CH₂CH₂CH₂-, - OCH₂CH₂-, et -NHCH₂CH₂- ; 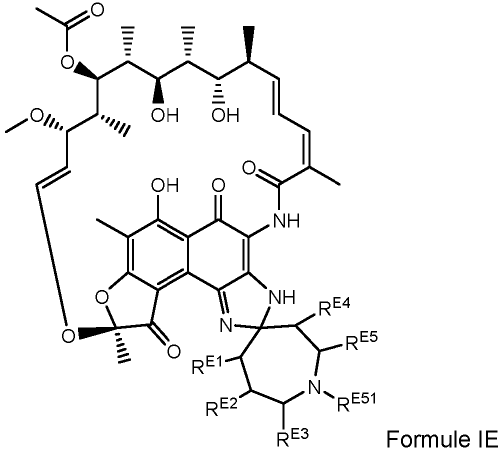 dans lequel :
R^{E1}, R^{E2}, et R^{E4} sont chacun -H ou l'alkyle en C₁à C₄ ; ou
R^{E3} et R^{E5} sont -H ou se combinent pour former un cyclooctane pontant ; et ;
R^{E51} est -H ou l'alkyle en -C₁à C₄.

11. Composé selon la revendication 1, dans lequel X¹, X², X³, X⁴, X⁵, X⁶ et NR⁵¹ forment une structure choisie parmi :

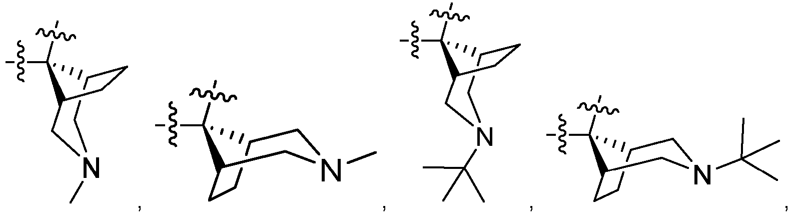

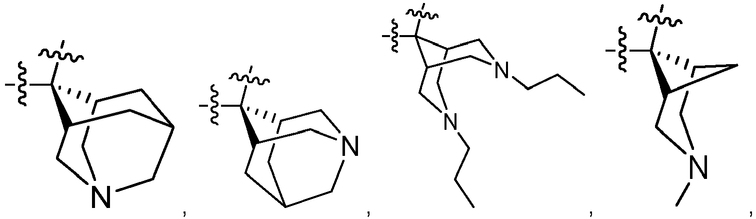

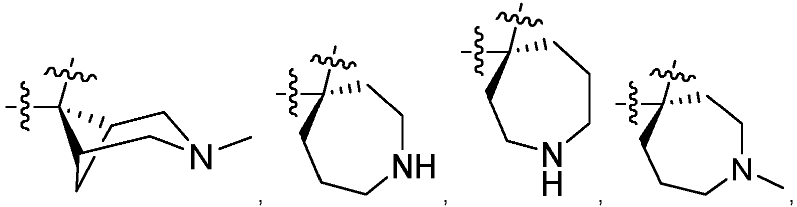

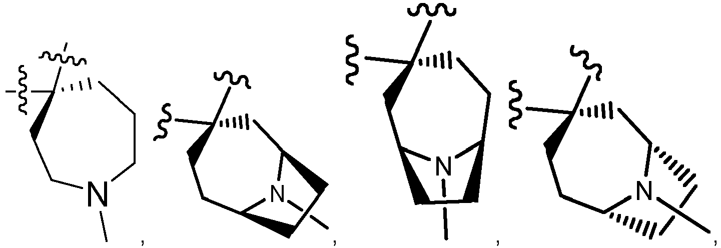

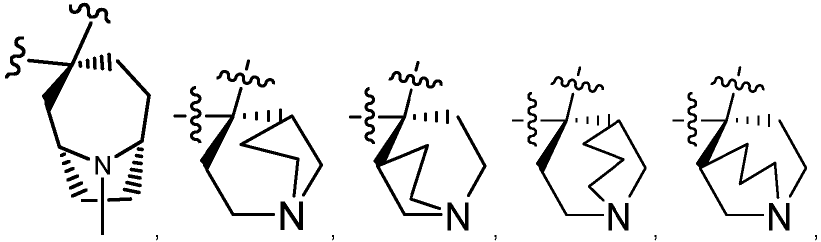

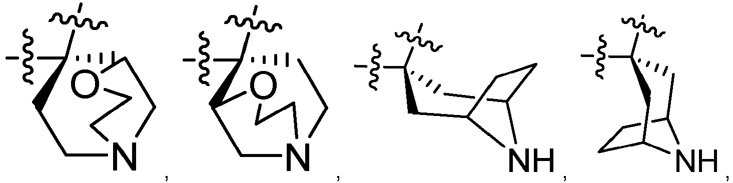

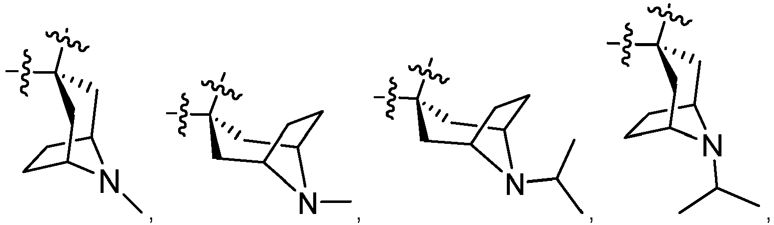

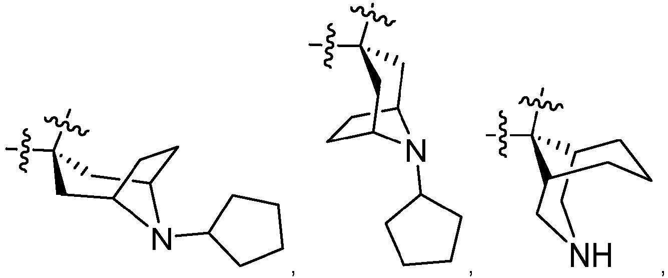

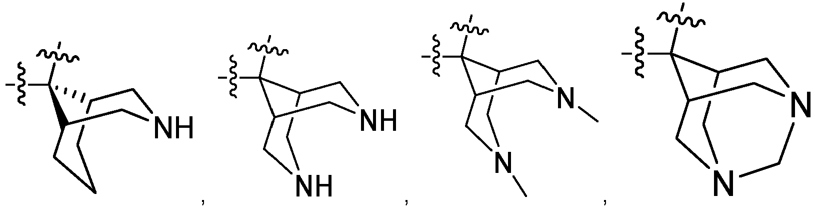

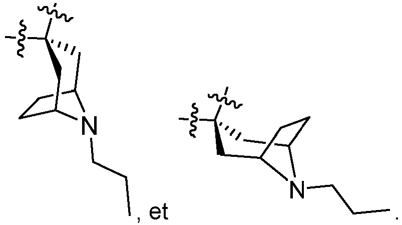

12. Composé selon la revendication 1, choisi dans le groupe constitué de : 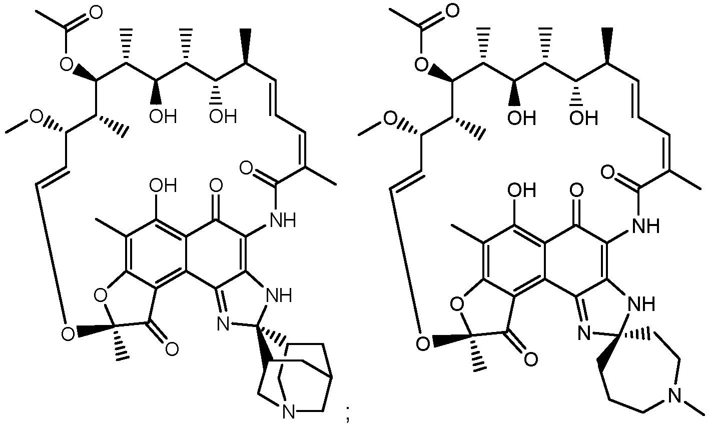 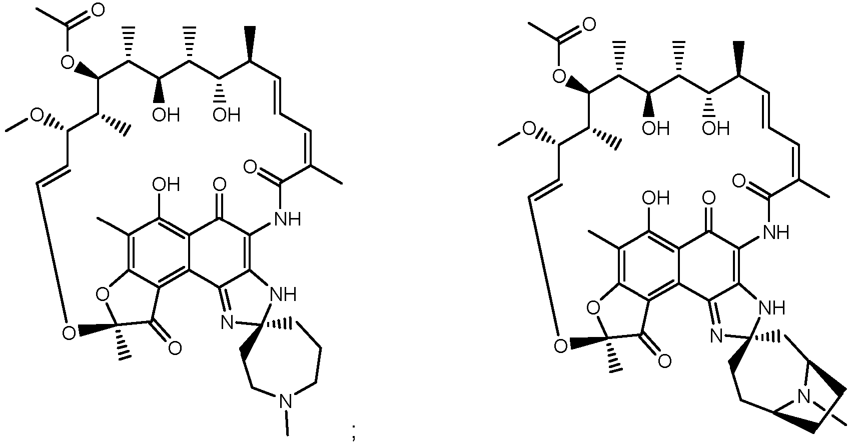 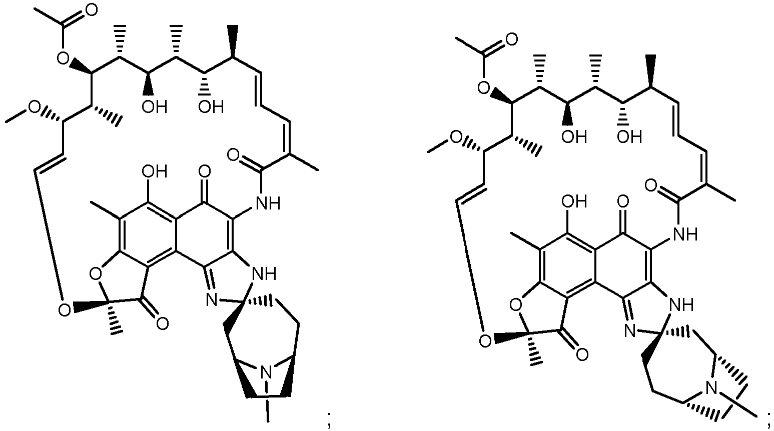 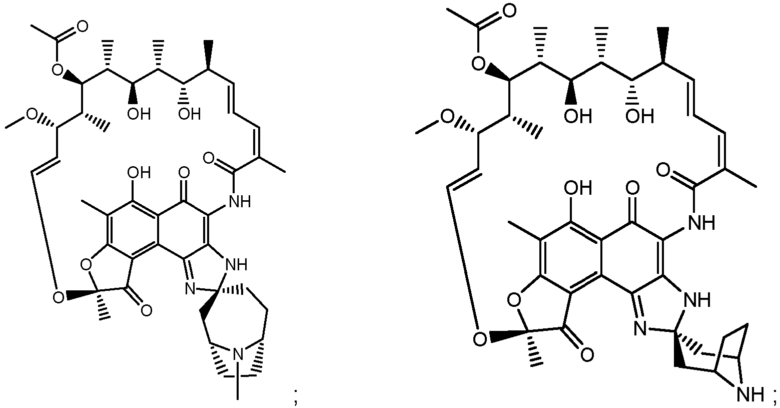 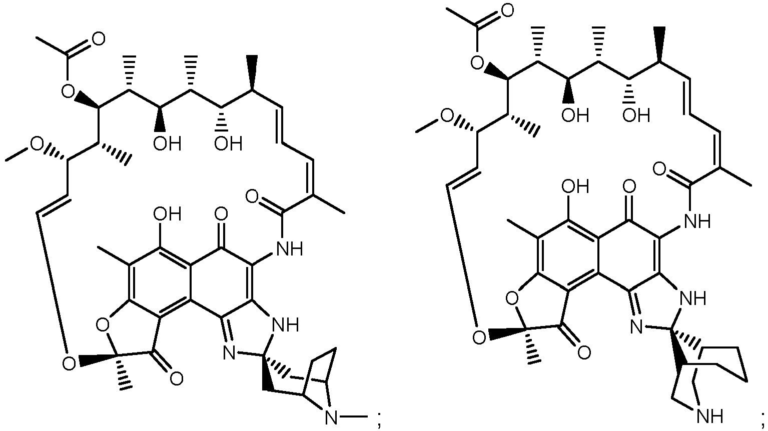 et 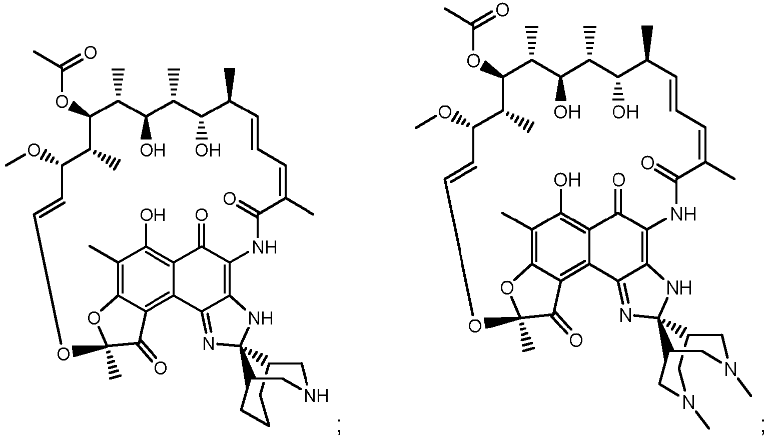 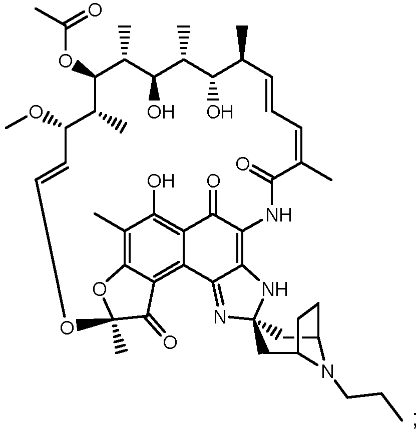 dans lequel, de préférence, ledit composé est choisi dans le groupe constitué de : 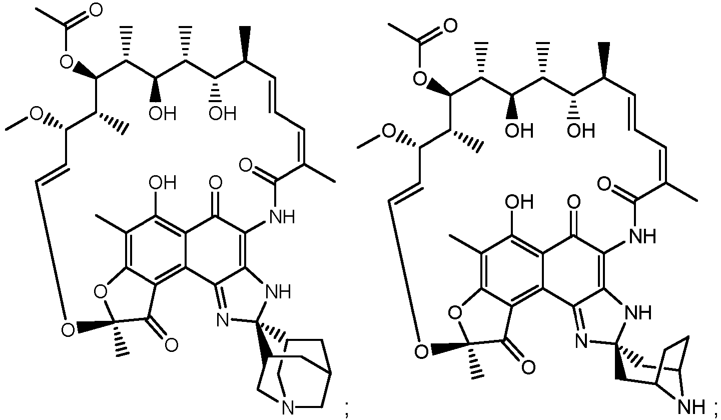 et

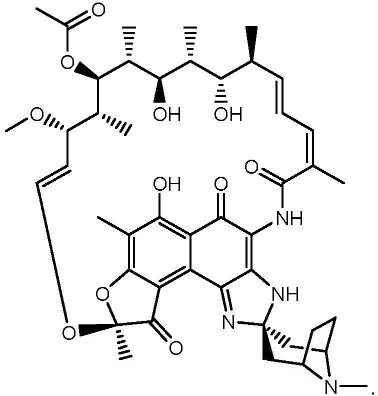

13. Composition pharmaceutique comprenant au moins un composé selon l'une quelconque des revendications précédentes, ou un sel, tautomère, solvate ou hydrate pharmaceutiquement acceptable de celui-ci, et un excipient pharmaceutiquement acceptable.

14. Composé selon l'une quelconque des revendications 1 à 12, ou composition pharmaceutique selon la revendication 13, destiné à être utilisé comme médicament, de préférence destiné à être utilisé dans un procédé de prévention ou de traitement d'une maladie chez un sujet, dans lequel ladite maladie est une infection, dans lequel de préférence une infection bactérienne.

15. Composé ou composition pharmaceutique destiné à être utilisé selon la revendication 14, dans lequel ladite maladie est une infection bactérienne, dans lequel ladite infection bactérienne est causée par une bactérie appartenant à *Acinetobacter* spp., *Clostridium* spp., *Enterococcus* spp., *Hemophilus* spp., *Legionella* spp., *Mycobacterium* spp. (Mycobacteriatuberculeuses et non tuberculeuses), Neisseria spp., *Staphylococcus* spp., *Streptococcus* spp., *Listeria monocytogenes, Moraxella catarrhalis, Bacillus* spp., *Bacteroides* spp., *Gardnerella vaginalis, Lactobacillus* spp., *Mobiluncus* spp., *Helicobacter pylori, Campylobacter jejuni*, *Chlamydia trachomatis* et/ou *Toxoplasma gondii* ; de préférence, une infection bactérienne causée par *S. aureus, A. baumannii, E. faecium, E. faecalis, S. epidermidis, S. pneumoniae, S. pyogenes, H. influenzae, M. abscessus, M. tuberculosis,* et/ou *M. smegmatis* ; et dans lequel, de préférence, ladite infection bactérienne est causée par une ou plusieurs bactéries appartenant au genre *Acinetobacter* ; de préférence en outre par *A. baumannii* ; et, encore plus préférablement, par *A. baumannii* qui possède un ou plusieurs mécanismes de résistance aux rifamycines telles que la rifabutine et/ou la rifampicine.
